# EUROPEAN PATENT SPECIFICATION

(11) **EP 2 771 433 B1**
(45) Date of publication and mention of the grant of the patent: **27.04.2016**
(21) Application number: 12843409.9
(22) Date of filing: 29.10.2012
(51) Int. Cl.: C10G 45/00, C10G 3/00, C11B 1/10, C11B 1/02, C11B 1/04, C07C 1/207, C07C 5/02, C11C 3/00, C10G 11/18, C11C 3/12

(54) **PROCESSES FOR UPGRADING ALGAE OILS AND PRODUCTS THEREOF**
VERFAHREN ZUR VEREDELUNG VON ALGENÖLEN UND PRODUKTE DARAUS
PROCÉDÉS DE VALORISATION D'HUILES ALGALES ET PRODUITS ASSOCIÉS

(30) Priority: 28.10.2011 US 201161553128 P
(43) Date of publication of application: 03.09.2014
(73) Proprietor: Sapphire Energy, Inc., San Diego, CA 92121 (US)
(72) Inventor: SAJKOWSKI, Daniel, San Diego, California 92121 (US); ROUSSIS, Stilianos, San Diego, California 92121 (US); CRANFORD, Richard, San Diego, California 92121 (US)
(74) Representative: Hoffmann Eitle
(86) International application number: PCT/US2012/062468
(87) International publication number: WO 2013/063595

(56) References cited:
- WO-A1-2010/124030
- WO-A1-2012/088546
- WO-A2-2008/151149
- US-A1- 2010 038 284
- US-A1- 2011 071 327
- US-A1- 2011 071 327
- US-A1- 2011 107 656
- US-A1- 2011 230 632
- US-A1- 2011 287 503

## Description

### CROSS REFERENCE TO RELATED APPLICATIONS

This application claims the benefit of U.S. Provisional Patent Application Serial No. 61/553,128, filed 28 October 2011, of which is herein incorporated by reference in its entirety for all purposes.

### BACKGROUND

Many renewable oils, or "bio-oils", are composed almost exclusively of triglycerides and/or free fatty acids that are derivatives of triglycerides. Examples are canola and soy bean oils among the plant oils that can be alternatively a food product, and camelina and jatropha among non-food oils. Even tallow is composed almost exclusively of triglycerides. The interest in these oils as fuels is intense, given the desire to lessen the country and even world's dependence on petroleum. Processes, such as UOP's Ecofining™, have been commercially developed. The paradigm has emerged that oils from all plants and algae, including various species and processes for their production, consist predominantly and even exclusively of triglycerides and fatty acids. This has led inventors to extend their work on triglycerides and fatty acids to oils from algae, by listing algae oils with high-triglyceride oils in their patent applications regarding renewable oils, even though they have not actually processed algae derived oils.

In fact, algae oils produced in commercially-scaleable and economic processes do not consist of exclusively triglycerides and fatty acids, as disclosed in currently-pending patent applications by Sapphire Energy, Inc. Recent publications have also found this is to be the case (Valdez, et al. "Liquefaction of Nannachloropsis Sp. and the Influence of Solvents." Energy & Fuels (2011). This is a critical point and the composition of algae oils requires substantially different processing than has previously been discussed for renewable oils. In fact, algae oil has a very unique composition and may be considered an algae biocrude, in that it contains a wide variety of compounds, as opposed to simpler, high-triglyceride vegetable oils. Yet, the algae biocrude is also very different from fossil petroleum, in that many of said variety of compounds are not present in petroleum or in oils from oil sands, coal and shale oil. Therefore, it is not clear or obvious from past work on vegetable oils or fossil petroleum how to upgrade algae oil to feedstock for petroleum refineries or to commercial products.

US 2010/038284 relates to a method for the catalytic hydrotreatment of a load based on a diesel fuel oil and a biological load based on plant oils and/or animal fats in a hydrotreatment unit.

US 2011/287503 relates to methods for producing hydrocarbon oils from algal biomass.

WO 2012/088546 relates to a method for the production of aromatics from renewable sources.

This disclosure recognizes this critical difference and provides specific processing routes unique to algae biocrude. The processing technologies of this disclosure borrow from existing technologies, however, the sequence and processing conditions surprisingly require an understanding of algae oil that is very different from what is the accepted paradigm and basis for previously cited and patented renewable oil technology. Therefore, unique upgrading methods, and unique compositions of matter resulting therefrom, are disclosed herein for algae biocrude.

### SUMMARY

This disclosure relates to upgrading renewable oils that have been extracted from algae biomass. Certain embodiments may comprise upgrading the algae oils by one or more thermal or catalytic processes and/or the resulting compositions of matter. The algae oils are significantly different from high-triglyceride vegetables oils and animal fats that recently have been the focus of renewable oil development, in that the algae oils contain a wide range of compounds instead of mainly triglycerides, and they contain high amounts of both nitrogen and oxygen. In this disclosure, upgrading of algae oils has been accomplished by thermal processing or catalytic hydroprocessing with catalyst and/or operating conditions different from those proposed for high-triglyceride renewable fuels, and yet which are possible in existing petroleum refinery process units. Further, in this disclosure, catalytic cracking of catalytically hydrotreated algae oil has been accomplished with advantageous yield structures. The catalytic hydrotreating and catalytic-cracking results herein indicate that, in spite of the complex nature of algae oils, they are good candidates for feedstocks for conventional petroleum refineries, and that upgraded algae oil products are good candidates for high-quality fuels or fuel blending components. This includes using the algae oils as crude oil substitutes wherein they are introduced as a direct substitute for crude oil and fed to the crude distillation units or as an intermediate where they are fed directly to processing units downstream of the crude distillation unit And, it also includes feeding algae oil to processing units outside of the refinery battery limits that are operated with the intent of producing fuels from seed oils (camelina, palm oil, etc.) and/or tallow.

This disclosure comprises upgrading methods and/or equipment, and/or the compositions of matter, resulting from thermal processing, catalytic-hydroprocessing, and/or catalytic cracking, of renewable oil extracted from algae biomass. Certain embodiments comprise thermal treatment, decarboxylation, and/or moderate-to-high-severity hydrotreatment as methods of preparing algae oil, or fractions thereof, to be effective feedstock for subsequent processing to ultimately produce fuels, petrochemical feedstocks, lube basestock, or other products. Embodiments of particular interest use two or more of thermal treatment, decarboxylation, and medium-to-high severity hydrotreatment as said methods of preparing algae oil or its fractions. Certain embodiments utilize medium-to-high severity catalytic hydrotreatment, optionally with thermal treatment prior to said medium-to-high severity hydrotreatment, followed by fluidized catalytic cracking to obtain gasoline and light cycle oil (distillate) from algae oil.

Certain methods for preparation of algae oils for subsequent upgrading comprise medium-to-high severity hydrotreatment, for example, at pressures of 800 - 2000 psig in hydrogen, 300-425 degrees C (more typically 350 - 400 degrees C), and 0.5 - 2 1/hr (hr-1) LHSV (more typically about 1 1/hr LHSV), over one or more hydrotreating catalysts. Examples of hydrotreating catalysts are NiMo and/or Co/Mo on alumina or silica-alumina supports, for example, with BET surface areas in the range of about 100 - 300 m2/g, and micropores in the average diameter range of 20 -1000 Angstroms, and optionally with macropores in the range of 500 -10,000 Angstroms. Examples of metals loadings include nickel or cobalt ranging from greater than 0 up to about 10 %, and molybdenum ranging from greater than 0 up to about 40%. BET surface area measurements/equations are well-known in the catalyst arts.

In view of the unusual characteristics of algae oil, as described elsewhere in this disclosure, hydrotreating at greater than 1000 psig and even in the range of 1500 - 2000 psig may be needed. Hydrotreating in the range of 800-3000 psig can be conducted according to the methods disclosed herein. Also in view of the unusual characteristics of algae oil, large-pore hydrotreating catalysts including macro-pores may be needed. Examples of such hydrotreating catalysts include catalysts having BET surface areas in the range of 150 - 250 m2/g, micropores in the average diameter range of 50 - 200 Angstroms, and macropores in the range of 1000 - 3000 Angstroms.

Certain methods for preparation of algae oils for subsequent upgrading comprise thermal treatment, (without catalyst and without, or optionally with, the addition of hydrogen. Such thermal treatment, of the entire crude algae oil or a fraction thereof, may be conducted at a range of pressures, including autogenous pressure, and is expected to be beneficial as an early or first step in the processing of algae oils, for example, prior to hydrotreatment. Certain embodiments comprise thermal treatment of a heavy fraction of the crude algae oil, blending the liquid product of the thermal treatment with the lighter fraction of the crude algae oil, and subsequent hydrotreating of the blend

Certain embodiments of hydrotreating, especially moderate-and-high-severity catalytic-hydrotreatment, provide high levels of heteroatom removal, including deoxygenation, denitrogenation, and desulfurization. Saturated hydrocarbons are increased, fatty acids and amides are removed, and nitrogen and oxygen compounds are reduced, leaving only small levels of nitrogen-aromatics and oxygenated compounds in the oil product. Nitriles are not formed in a significant amount. High severity hydrotreating, such as at 1800 - 2000 psig, also removes sterols. Moderate-and-high-severity catalytic-hydrotreatment upgrades the algae oils to an extent that fatty acid content in the product oils is little or none (for example, 99 100 percent removal) compared to algae oil feeds having 15 - 60 area % (by HT GC-MS), for example. Starting with algae oil feeds having saturated hydrocarbon content of less than 5 area %, for example, certain embodiments of moderate- and-high-severity hydrotreating produce upgraded algae oils having a saturated hydrocarbon content of over 60 area %, and in some embodiments, over 70 area % (all by the same HT GC-MS analytical techniques).

An unexpected result of certain thermal and calalytic-hydrotreatment process embodiments is shifting of boiling point distribution away from residue and toward lower-boiling compounds. Specifically, boiling point reductions are exhibited that shift products toward gas oil, distillate, and/or naphtha fractions. The boiling point distribution changes are believed to result from cracking of the algae oil, which is surprising in low- and moderate-severity hydrotreatment, in that the cracking occurs at conditions and with catalysts that would not be expected to cause significant cracking of petroleum feeds. Further, the boiling point distribution changes exhibited in high severity hydrotreatment are within a range that have not been seen to cause deleterious effects in subsequent processing, such as in FCC processing. These boiling point distribution changes may allow algae oil producers and/or conventional refiners to adjust the products obtained from subsequent processes, in order to optimize their process unit operations and/or overall refinery product slate.

Therefore, certain embodiments of thermal and/or catalytic-hydroprocessing of algae oil have been found to remove hetero-atoms, greatly reduce fatty acids, greatly increase saturated hydrocarbons, and crack the algae feed to desirable liquid products that are good feedstocks for subsequent processes. These benefits, and various, but not all embodiments of the invention, will be further described below in the Detailed Description, with reference to the attached Figures and Tables. It should be understood that the Detailed Description, Figures, Tables, and Abstract supply examples and clarifying information but do not necessarily limit the invention to the details, specifics, methods and means therein.

Provided herein as part of the disclosure is an oleaginous composition comprising oil extracted from biomass comprising a microorganism wherein the composition is hydrotreated and the hydrotreated composition comprises: a) from about 30 weight percent to about 90 weight percent carbon containing compounds selected from the group consisting of C8, C9, C10, C11, C12, C13, C14, C15, C16, C17, and C18 containing compounds; b) from about 30 weight percent to about 70 weight percent carbon containing compounds selected from the group consisting of C8, C9, C10, C11, C12, C13, C14, C15, C16, C17, and C18 containing compounds; or c) from about 10 weight percent to about 80 weight percent carbon containing compounds selected from the group consisting of C9, C10, C11, C12, C13, C14, C15, C16, C17, C18, C19, C20, C21, C22, C23, C24, C25, C26, C27, C28, C29, C30, C31, and C32 containing compounds; wherein weight percent is of the total amount of compounds detectable by mass spectrometry or gas chromatography analysis. In some embodiments, the hydrotreated composition comprises: from about 40 to about 85 weight percent carbon containing compounds selected from the group consisting of C8, C9, C10, C11, C12, C13, C14, C15, C16, C17, and C18 containing compounds; from about 65 to about 85 weight percent carbon containing compounds selected from the group consisting of C8, C9, C10, C11, C12, C13, C14, C15, C16, C17, and C18 containing compounds; from about 70 to about 80 weight percent carbon containing compounds selected from the group consisting of C8, C9, C10, C11, C12, C13, C14, C15, C16, C17, and C18 containing compounds; from about 77 to about 84 weight percent carbon containing compounds selected from the group consisting of C8, C9, C10, C11, C12, C13, C14, C15, C16, C17, and C18 containing compounds; from 77.4 to 83.8 weight percent carbon containing compounds selected from the group consisting of C8, C9, C10, C11, C12, C13, C14, C15, C16, C17, and C18 containing compounds; from 77.3 to 85.5 weight percent carbon containing compounds selected from the group consisting of C8, C9, C10, C11, C12, C13, C14, C15, C16, C17, and C18 containing compounds; or from 80.8 to 86.6 weight percent carbon containing compounds selected from the group consisting of C8, C9, C10, C11, C12, C13, C14, C15, C16, C17, and C18 containing compounds. In other embodiments, the hydrotreated composition comprises: from about 43 to about 66 weight percent carbon containing compounds selected from the group consisting of C8, C9, C10, C11, C12, C13, C14, C15, C16, C17, and C18 containing compounds; or from about 42.7 to about 65,7 weight percent carbon containing compounds selected from the group consisting of C8, C9, C10, C11, C12, C13, C14, C15, C16, C17, and C18 containing compounds. In yet other embodiments, the hydrotreated composition comprises: from about 20 to about 70 weight percent carbon containing compounds selected from the group consisting of C9, C10, C11, C12, C13, C14, C15, C16, C17, C18, C19, C20, C21, C22, C23, C24, C25, C26, C27, C28, C29, C30, C31, and C32 containing compounds; from about 30 to about 60 weight percent carbon containing compounds selected from the group consisting of C9, C10, C11, C12, C13, C14, C15, C16, C17, C18, C19, C20, C21, C22, C23, C24, C25, C26, C27, C28, C29, C30, C31, and C32 containing compounds; or from about 23 to about 46 weight percent carbon containing compounds selected from the group consisting of C9, C10, C11, C12, C13, C14, C15, C16, C17, C18, C19, C20, C21, C22, C23, C24, C25, C26, C27, C28, C29, C30, C31, and C32 containing compounds. In some embodiments, prior to hydrotreatment, the oleaginous composition is hydrothermally extracted. In other embodiments, the hydrotreatment is in a semi-batch, batch, or continuous flow reactor. In yet other embodiments, the microorganism is an alga, the alga is a microalga or a macroalga, the microalga is a cyanobacterium, the microorganism is a *Desmodesmus* species or a *Spirulina* species, the microorganism is *a Nannochloropsis* species, or the *Nannochloropsis* species is *Nannochloropsis salina.* In other embodiments, the solvent used for extraction is a heptane, hexane, methyl isobutyl ketone (MIBK), acetonitrile, ethanol, methyl-t-butyl ether (MTBE), methyl ethyl ketone (MEK), propanol, iso propyl alcohol (IPA), methanol, cyclohexane, toluene (methylbenzene), chloroform (trichloromethane), methylene chloride (dichlorotnethane), a polar solvent, a non-polar solvent, or a combination of any two or more thereof. In other embodiments, the solvent used for extraction is hexane, heptane, or methyl isobutyl ketone (MIBK). In some embodiments, the mass spectrometry analysis is high temperature gas chromatography-mass spectrometry (HT GC-MS), gas chromatography-mass spectrometry (GC-MS), or liquid chromatography mass spectrometry, or the gas chromatography analysis is gas chromatography flame ionization detection (GC FID).

Also provided herein as part of the disclosure is an oleaginous composition comprising oil extracted from biomass comprising a microorganism wherein the composition is hydrotreated and the hydrotreated composition: a) has a reduction of nitrogen of at least 70% as compared to the unhydrotreated composition; b) the hydrotreated composition comprises less than 100 ppm of nitrogen; or c) the unhydrotreated composition has up to 8 weight percent nitrogen, and the hydrotreated composition has less than 1 weight percent nitrogen. In some embodiments, the reduction of nitrogen is at least 75%, the reduction of nitrogen is at least 80%, the reduction of nitrogen is at least 85%, the reduction of nitrogen is at least 90%, the reduction of nitrogen is at least 95%, or the reduction of nitrogen is at least 99%. In other embodiments, the hydrotreated composition comprises less than 90 ppm of nitrogen, the hydrotreated composition comprises less than 80 ppm of nitrogen, the hydrotreated composition comprises less than 70 ppm of nitrogen, the hydrotreated composition comprises less than 60 ppm of nitrogen, the hydrotreated composition comprises less than 50 ppm of nitrogen, the hydrotreated composition comprises less than 40 ppm of nitrogen, the bydrotreated composition comprises less than 30 ppm of nitrogen, the hydrotreated composition comprises less than 20 ppm of nitrogen, the hydrotreated composition comprises less than 10 ppm of nitrogen, or the hydrotreated composition comprises about 15 ppm of nitrogen, about 29 ppm of nitrogen, or about 11 ppm of nitrogen. In yet another embodiment, the unhydrotreated composition has up to 7 weight percent nitrogen, and the hydrotreated composition has less than 0.5 weight percent nitrogen. In other embodiments, the unhydrotreated composition has up to 7 weight percent, up to 6 weight percent, up to 5 weight percent, up to 4 weight percent, up to 3 weight percent, tip to 2 weight percent, or up to I weight percent nitrogen, and the hydrotreated composition has less than 0.9 weight percent, less than 0.8 weight percent, less than 0.7 weight percent, less than 0.6 weight percent, less than 0.5 weight percent, less than 0.4 weight percent, less than 0.3 weight percent, less than 0.2 weight percent, or less than 0.1 weight percent nitrogen. In some embodiments, the nitrogen levels are determined by ASTM standard D4629 or elemental analysis. In another embodiment, prior to hydrotreatment, the oleaginous composition is hydrothermally extracted. In other embodiments, the hydrotreatment is in a semi-batch, batch, or continuous flow reactor. In yet other embodiments, the microorganism is an alga, the alga is a microalga or a macroalga, the microalga is a cyanobacterium, the microorganism is a *Desmodesmus* species or a *Spirulina* species, the microorganism is a *Nannochloropsis* species, or the *Nannochloropsis* species is *Nannochloropsis salina.* In other embodiments, the solvent used for extraction is a heptane, hexane, methyl isobutyl ketone (MIBK), acetonitrile, ethanol, methyl-t-butyl ether (MTBE), methyl ethyl ketone (MEK), propanol, iso propyl alcohol (IPA), methanol, cyclohexane, toluene (methylbenzene), chloroform (trichloromethane), methylene chloride (dichloromethane), a polar solvent, a non-polar solvent, or a combination of any two or more thereof. In other embodiments, the solvent used for extraction is hexane, heptane, or methyl isobutyl ketone (MIBK). In some embodiments, the mass spectrometry analysis is high temperature gas chromatography-mass spectrometry (HT GC-MS), gas chromatography-mass spectrometry (GC-MS), or liquid chromatography mass spectrometry, or the gas chromatography analysis is gas chromatography flame ionization detection (GC FID).

Also provided herein as part of the disclosure is an oleaginous composition comprising oil extracted from biomass comprising a microorganism wherein the composition is hydrotreated and the hydrotreated composition comprises a percent mass fraction with a boiling point of from 260 degrees F to 1020 degrees F of between about 40% and about 95% as determined by ASTM protocol D7169. In some embodiments, the hydrotreated composition comprises a percent mass fraction with a boiling point of from 260 degrees F to 1020 degrees F of between about 60% and about 90%; the hydrotreated composition comprises a percent mass fraction with a boiling point of from 260 degrees F to 1020 degrees F of between about 74.4% and about 87.4%; the hydrotreated composition comprises a percent mass fraction with a boiling point of from 260 degrees F to 630 degrees F of between about 30% and about 55%; or the hydrotreated composition comprises a percent mass fraction with a boiling point of from 260 degrees F to 630 degrees F of between about 35.2% and about 51%. In another embodiment, prior to hydrotreatment, the oleaginous composition is hydrothermally extracted. In other embodiments, the hydrotreatment is in a semi-batch, batch, or continuous flow reactor. In yet other embodiments, the microorganism is an alga, the alga is a microalga or a macroalga, the microalga is a cyanobacterium, the microorganism is a *Desmodesmus* species or a *Spirulina* species, the microorganism is a *Nannochloropsis* species, or the *Nannochloropsis* species is *Nannochloropsis salina.* In other embodiments, the solvent used for extraction is a heptane, hexane, methyl isobutyl ketone (MBK), acetonitrile, ethanol, methyl-t-butyl ether (MTBE), methyl ethyl ketone (MEK), propanol, iso propyl alcohol (IPA), methanol, cyclohexane, toluene (methylbenzene), chloroform (trichloromethane), methylene chloride (dichloromethane), a polar solvent, a non-polar solvent, or a combination of any two or more thereof. In other embodiments, the solvent used for extraction is hexane, heptane, or methyl isobutyl ketone (MIBK). In some embodiments, the mass spectrometry analysis is high temperature gas chromatography-mass spectrometry (HT GC-MS), gas chromatography-mass spectrometry (GC-MS), or liquid chromatography mass spectrometry, or the gas chromatography analysis is gas chromatography flame ionization detection (GC- FID).

Further provided herein as part of the disclosure is an oleaginous composition comprising oil extracted from biomass comprising a microorganism wherein the oleaginous composition has: a) at least 60% of its components boiling below about 320 degrees Celsius (about 608 degrees Fahrenheit); or b) at least 90% of its components boiling above about 450 degrees Fahrenheit (about 232.22 degrees Celsius) as determined by ASTM D7169; an oleaginous composition comprising oil extracted from biomass comprising a microorganism, wherein the oleaginous composition has at least 65% of its components boiling below about 320 degrees Celsius; an oleaginous composition comprising oil extracted from biomass comprising a microorganism, wherein the oleaginous composition has at least 70% of its components boiling below about 320 degrees Celsius; an oleaginous composition comprising oil extracted from biomass comprising a microorganism, wherein the oleaginous composition has at least 75% of its components boiling below about 320 degrees Celsius; an oleaginous composition comprising oil extracted from biomass comprising a microorganism, wherein the oleaginous composition has at least 80% of its components boiling below about 320 degrees Celsius; an oleaginous composition comprising oil extracted from biomass comprising a microorganism, wherein the oleaginous composition has at least 85% of its components boiling below about 320 degrees Celsius; an oleaginous composition comprising oil extracted from biomass comprising a microorganism, wherein the oleaginous composition has at least 90% of its components boiling below about 320 degrees Celsius; an oleaginous composition comprising oil extracted from biomass comprising a microorganism, wherein the oleaginous composition has at least 95% of its components boiling below about 320 degrees Celsius; an oleaginous composition comprising oil extracted from biomass comprising a microorganism, wherein the oleaginous composition has at least 99% of its components boiling below about 320 degrees Celsius; an oleaginous composition comprising oil extracted from biomass comprising a microorganism, wherein the oleaginous composition has at least 85% of its components boiling above about 475 degrees Fahrenheit; an oleaginous composition comprising oil extracted from biomass comprising a microorganism, wherein the oleaginous composition has at least 80% of its components boiling above about 500 degrees Fahrenheit; or an oleaginous composition comprising oil extracted from biomass comprising a microorganism, wherein the oleaginous composition has at least 75% of its components boiling above about 550 degrees Fahrenheit. In one embodiment, the oleaginous composition has been hydrotreated. In other embodiments, prior to hydrotreatment, the oleaginous composition is hydrothermally extracted. In one embodiment, the percentage of components is determined by ASTM protocol D7169. In other embodiments, the hydrotreatment is in a semi-batch, batch, or continuous flow reactor. In yet other embodiments, the microorganism is an alga, the alga is a microalga or a microalga, the microalga is a cyanobacterium, the microorganism is a *Desmodesmus* species or a *Spirulirra* species, the microorganism is a *Nannochloropsis* species, or the *Nannochloropsis* species is *Nannochloropsis salina.* In other embodiments, the solvent used for extraction is a heptane, hexane, methyl isobutyl ketone (MIBK), acetonitrile, ethanol, methyl-t-butyl ether (MTBE), methyl ethyl ketone (MEK), propanol, iso propyl alcohol (IPA), methanol, cyclohexane, toluene (methylbenzene), chloroform (trichloromethane), methylene chloride (dichloromethane), a polar solvent, a non-polar solvent, or a combination of any two or more thereof. In other embodiments, the solvent used for extraction is hexane, heptane, or methyl isobutyl ketone (MIBK). In some embodiments, the mass spectrometry analysis is high temperature gas chromatography-mass spectrometry (HT GC-MS), gas chromatography-mass spectrometry (GC-MS), or liquid chromatography mass spectrometry, or the gas chromatography analysis is gas chromatography flame ionization detection (GC FID).

Provided herein as part of the disclosure is an oleaginous composition comprising oil extracted from biomass comprising a microorganism wherein the composition is hydrotreated and the hydrotreated composition comprises: from about 70.8 to about 86.6 weight percent Carbon; from about 9.5 to about 14.5 weight percent Hydrogen; or from about 0.03 to about 3.6 weight percent Nitrogen. In some embodiments, the oleaginous composition further comprises less than or equal to about 0.76 weight percent Sulfur. In other embodiments, the oleaginous composition further comprises less than or equal to about 2.6 weight percent Oxygen by difference. In one embodiment, prior to hydrotreatment, the oleaginous composition is hydrothermally extracted. In other embodiments, the hydrotreatment is in a semi-batch, batch or continuous flow reactor. In yet other embodiments, the microorganism is an alga, the alga is a microalga or a macroalga, the microalga is a cyanobacterium, the microorganism is a *Desmodesmus* species or a *Spirulina* species, the microorganism is a *Nannochloropsis* species, or the *Nannochloropsis* species is *Nannochloropsis salina.* In other embodiments, the solvent used for extraction is a Heptane, hexane, methyl isobutyl ketone (MIBK), acetonitrile, ethanol, methyl-t-butyl ether (MTBE), methyl ethyl ketone (MEK), propanol, iso propyl alcohol (IPA), methanol, cyclohexane, toluene (methylbenzene), chloroform (trichloromethane), methylene chloride (dichloromethane). a polar solvent, a non-polar solvent, or a combination of any two or more thereof. In other embodiments, the solvent used for extraction is hexane, heptane, or methyl isobutyl ketone (MIBK). In some embodiments, the mass spectrometry analysis is high temperature gas chromatography-mass spectrometry (HT GC-MS), gas chromatography-mass spectrometry, (GC-MS), or liquid chromatography mass spectrometry, or the gas chromatography analysis is gas chromatography flame ionization detection (GC FID)

Also provided herein as part of the disclosure is an oleaginous composition comprising oil extracted from biomass comprising a microorganism wherein the composition is hydrotreated and the hydrotreated composition comprises: a) an area percent of saturated hydrocarbons from about 36.3 to about 75.7; an area percent of unsaturated hydrocarbons from about 0.3 to about 5.5; an area percent of N-aromatics from about 0.1 to about 1.2; and an area percent of oxygen compounds from about 0.7 to about 5.6; or b) an area percent of saturated hydrocarbons from about 43.0 to about 74.0; an area percent of unsaturated hydrocarbons of less than or equal to 0.7; an area percent of aromatics from about 0.7 to about 2.3; and an area percent of oxygen compounds from about 1.4 to about 2.7. In one embodiment, prior to hydrotreatment, the oleaginous composition is hydrothermally extracted. In other embodiments, the hydrotreatment is in a semi-batch, batch, or continuous flow reactor. In yet other embodiments, the microorganism is an alga, the alga is a microalga or a macroalga, the microalga is a cyanobacterium, the microorganism is a *Desmodesmus* species or a *Spirulina* species, the microorganism is a *Nannochloropsis* species, or the *Nannochloropsis* species is *Nannochloropsis salina.* In other embodiments, the solvent used for extraction is a heptane, hexane, methyl isobutyl ketone (MIBK), acetonitrile, ethanol, methyl-t-butyl ether (MTBE), methyl ethyl ketone (MEK), propanol, iso propyl alcohol (IPA), methanol, cyclohexane, toluene (methylbenzene), chloroform (trichloromethane), methylene chloride (dichloromethane), a polar solvent, a non-polar solvent, or a combination of any two or more thereof. In other embodiments, the solvent used for extraction is hexane, heptane, or methyl isobutyl ketone (MIBK). In some embodiments, the mass spectrometry analysis is high temperature gas chromatography-mass spectrometry (HT GC-MS), gas chromatography-mass spectrometry (GC-MS), or liquid chromatography mass spectrometry, or the gas chromatography analysis is gas chromatography flame ionization detection (GC FID).

Also provided herein is a method of upgrading renewable oil obtained from biomass, the method comprising: a) providing the renewable oil; b) dissolving at least a portion of the renewable oil in a solvent; and c) upgrading the renewable oil in the solvent by a method comprising: hydrotreating the renewable oil in the solvent in the presence of a catalyst, at a temperature of from about 300 degrees C to about 500 degrees C; a total pressure and/or hydrogen partial pressure of from about 800 psi to about 3000 psi; a space velocity from about 0.1 volume of oil per volume of catalyst per hour to about 10 volume of oil per volume of catalyst per hour; and a hydrogen feed rate of from about 10 m³ H₂/m³ dissolved oil to about 1700m³ H_{2/}m³ dissolved oil, to obtain a hydrotreating effluent. In one embodiment, prior to step a), step b), and step c) the renewable oil was not refined-bleached-deodorized (RBD). In other embodiments, the method further comprises, separating a hydrotreated oil from the hydrotreating effluent; and further upgrading the hydrotreated oil by sending the hydrotreated oil or a fraction thereof to one or more of an FCC unit, a hydrocracking unit, a hydro isomerization unit, a dew axing unit, a naphtha reformer, or a unit utilizing Ni/Mo, Co/Mo, Ni/W, a precious metal, a noble metal, a group VIII catalyst, or a zeolite catalyst In other embodiments, the solvent is naphtha, diesel, kerosene, light gasoil, heavy gasoil, resid, heavy crude, dodecane, a cyclic solvent, an aromatic solvent, a hydrocarbon solvent, crude oil, any product obtained after distillation of crude oil and/or the further refining of crude oil fractions, or any combination thereof. In yet other embodiments, the space velocity is from about 0.1 volume of oil per volume of catalyst per hour to about 6 volume of oil per volume of catalyst per hour; from about 0.2 volume of oil per volume of catalyst per hour to about 5 volume of oil per volume of catalyst per hour; from about 0.6 volume of oil per volume of catalyst per hour to about 3 volume of oil per volume of catalyst per hour; or about 1.0 volume of oil per volume of catalyst per hour. In some embodiments, the hydrogen feed rate is from about 100 m³ H₂/m³ dissolved oil to about 1400 m³ H₂/m³ dissolved oil; from about 100 m³ H₂/m³ dissolved oil to about 1000 m³ H₂/m³ dissolved oil; from about 100 m³ H₂/m³ dissolved oil to about 800 m³ H₂/m³ dissolved oil; from about 200 m³ H₂/m³ dissolved oil to about 500m³ H₂/m³ dissolved oil; or about 600 m³ H₂/m³ dissolved oil. In yet other embodiments, the total pressure and/or hydrogen partial pressure is from about 1000 psi to about 2000 psi, about 1500 psi to about 2000 psi; or selected from the group consisting of: 1000 psi to 1100 psi, 1100 psi to 1200 psi, 1200 psi to 1300 psi, 1300 psi to 1400 psi, 1400 psi to 1500 psi, 1500 psi to 1600 psi, 1600 psi to 1700 psi, 1700 psi to 1800 psi, 1800 psi to 1900 psi, 1900 psi to 2000 psi, 2000 psi to 2100 psi, 2100 psi to 2200 psi, 2200 psi to 2300 psi, 2300 psi to 2400 psi, 2400 psi to 2500 psi, 2500 psi to 2600 psi, 2600 psi to 2700 psi, 2700 psi to 2800 psi, 2800 psi to 2900 psi, and 2900 psi to 3000 psi. In some embodiments, the temperature is in a range selected from a group consisting of: 300 to 310, 310 to 320,320 to 330, 330 to 340, 340 to 350, 350 to 360,360 to 370, 370 to 380, 380 to 390, 390 to 400, 400 to 410,410 to 420, 420 to 430, 430 to 440, 440 to 450, 450 to 460,460 to 470,470 to 480,480 to 490, and 490 to 500 degrees C. In still other embodiments, the catalyst is a large-pore catalyst selected from the group consisting of petroleum residuum/bitumen hydrotreating catalysts; the catalyst comprises Ni/Mo and/or Co/Mo on an alumina or a silica-alumina support; or the catalyst is characterized by having a pore structure comprising macro-pores and characterized by BET surface areas in the range of about 10 m²/g to about 350 m²/g or about 150 m²/g to about 250 m²/g; micropores in the average diameter range of about 50 Angstroms to about 200 Angstroms; or macropores in the range of about 1000 Angstroms to about 3000 Angstroms. In other embodiments, the method further comprises, either prior to step b) or after step b), thermally treating the renewable oil prior to hydrotreating, by raising the renewable oil to a temperature in the range of about 300 to about 600 degrees C, and holding at about that temperature for a hold time in the range of 0 minutes to about 8 hours, about 0.25 to about 8 hours, or about 0.5 to about 2 hours. In another embodiment, the thermal treatment is conducted at less than 1000 psi. In other embodiments, the thermal treatment is conducted in a range of about atmospheric to about 300 psi. In one embodiments, no hydrogen is added to the thermal treatment process. In one embodiment, the method further comprising fluid-catalytic-cracking (FCC) the hydrotreated oil. In other embodiments, the biomass is algal biomass; the algal biomass comprises microalga and/or macroalga; the microalga is a cyanobacterium; the microalga is a *Desmodesmus, Spirulina,* or *Nannochloropsis* species; or the *Nannochloropsis* species is *Nannochloropsis salina.* Also provided herein is a hydrotreating effluent made by any one of the method embodiments described above.

Also provided herein is a hydrotreated effluent made by the process of: a) providing a renewable oil obtained from a biomass; b) dissolving at least a portion of the renewable oil in a solvent; and b) upgrading the renewable oil in the solvent by a method comprising: hydrotreating the renewable oil in the solvent at a temperature of from about 300 degrees C to about 500 degrees C; a total pressure and/or hydrogen partial pressure of from about 800 psi to about 3000 psi; a space velocity from about 0.1 volume of oil per volume of catalyst per hour to about 10 volume of oil per volume of catalyst per hour; and a hydrogen feed rate of from about 10 m³ H₂/m³ dissolved oil to about 800m³ H₂/m³ dissolved oil, to obtain the hydrotreating effluent In one embodiment, prior to step a), step b), and step c) the renewable oil was not refined-bleached-deodorized (RBD). In other embodiments, the method further comprises, separating a hydrotreated oil from the hydrotreating effluent; and further upgrading the hydrotreated oil by sending the hydrotreated oil or a fraction thereof to one or more of an FCC unit, a hydrocracking unit, a hydroisomerization unit, a dewaxing unit, a naphtha reformer, or a unit utilizing Ni/Mo, Ca/Mo, Ni/W, a precious metal, a noble metal, a group VIII catalyst, or a zeolitic catalyst. In other embodiments, the solvent is naphtha; diesel, kerosene, light gasoil, heavy gasoil, resid, heavy crude, dodecane, a cyclic solvent, an aromatic solvent, a hydrocarbon solvent, crude oil, any product obtained after distillation of crude oil and/or the further refining of crude oil fractions, or any combination thereof. In yet other embodiments, the space velocity is from about 0.1 volume of oil per volume of catalyst per hour to about 6 volume of oil per volume of catalyst per hour, from about 0.2 volume of oil per volume of catalyst per hour to about 5 volume of oil per volume of catalyst per hour; from about 0.6 volume of oil per volume of catalyst per hour to about 3 volume of oil per volume of catalyst per hour; or about 1.0 volume of oil per volume of catalyst per hour. In some embodiments, the hydrogen feed rate is from about 100 m³ H₂/m³ dissolved oil to about 1400 m³ H₂/m³ dissolved oil; from about 100 m³ H₂/m³ dissolved oil to about 1000 m³ H₂/m³ dissolved oil; from about 100 m³ H₂/m³ dissolved oil to about 800 m³ H₂/m³ dissolved oil; from about 200 m³ H₂/m³ dissolved oil to about 500m³ H₂/m³ dissolved oil; or about 600 m³ H₂/m³ dissolved oil. In yet other embodiments, the total pressure and/or hydrogen partial pressure is from about 1000 psi to about 2000 psi, about 1500 psi to about 2000 psi; or selected from the group consisting of: 1000 psi to 1100 psi, 1100 psi to 1200 psi, 1200 psi to 1300 psi, 1300 psi to 1400 psi, 1400 psi to 1500 psi, 1500 psi to 1600 psi, 1600 psi to 1700 psi, 1700 psi to 1800 psi, 1800 psi to 1900 psi, 1900 psi to 2000 psi, 2000 psi to 2100 psi, 2100 psi to 2200 psi, 2200 psi to 2300 psi, 2300 psi to 2400 psi, 2400 psi to 2500 psi, 2500 psi to 2600 psi, 2600 psi to 2700 psi, 2700 psi to 2800 psi, 2800 psi to 2900 psi, and 2900 psi to 3000 psi. In some embodiments, the temperature is in a range selected from a group consisting of: 300 to 310, 310 to 320, 320 to 330, 330 to 340, 340 to 350, 350 to 360, 360 to 370, 370 to 380, 380 to 390, 390 to 400, 400 to 410, 410 to 420,420 to 430,430 to 440, 440 to 450, 450 to 460, 460 to 470,470 to 480, 480 to 490, and 490 to 500 degrees C. In still other embodiments, the catalyst is a large-pore catalyst selected from the group consisting of petroleum residuum/bitumen hydrotreating catalysts; the catalyst comprises Ni/Mo and/or Co/Mo on an alumina or a silica-alumina support; or the catalyst is characterized by having a pore structure comprising macro-pares and characterized by BET surface areas in the range of about 10 m²/g to about 350 m²/g or about 150 m²/g to about 250 m²/g; micropores in the average diameter range of about 50 Angstroms to about 200 Angstroms; or macropores in the range of about 1000 Angstroms to about 3000 Angstroms. In other embodiments, the method further comprises, either prior to step b) or after step b), thermally treating the renewable oil prior to hydrotreating, by raising the renewable oil to a temperature in the range of about 300 to about 600 degrees C, and holding at about that temperature for a hold time in the range of 0 minutes to about 8 hours, about 0.25 to about 8 hours, or about 0.5 to about 2 hours. In another embodiment, the thermal treatment is conducted at less than 1000 psi. In other embodiments, the thermal treatment is conducted in a range of about atmospheric to about 300 psi. In one embodiment, no hydrogen is added to the thermal treatment process. In one embodiment, the method further comprising fluid-catalytic-cracking (FCC) the hydrotreated oil. In other embodiments, the biomass is algal biomass; the algal biomass comprises microalga and/or macroalga; the microalga is a cyanobacterium; the microalga is a *Desmodesmus, Spirulina, or Nannochloropsis* species; or the *Nannochloropsis* species is *Nannochlompsis salina*

Also disclosed herein is a method of upgrading a renewable oil extracted from aquatic biomass, the method comprising: a) providing a renewable oil feed extracted from aquatic biomass, the renewable oil feed comprising less than 10 area % saturated hydrocarbons, 15 to 60 area % fatty acids, over 3 wt% nitrogen, and over 5 wt-% oxygen, wherein area % is measured and calculated from HT-GC-MS analysis; b) hydroprocessing the renewable oil feed by a method comprising hydrotreating the renewable oil feed over hydrotreating catalyst at one or more pressures in the range of 1000 to 2000 psig in the presence of added hydrogen; and c) separating a liquid oil product from the hydrotreating effluent, wherein the liquid oil product has a lower boiling point distribution than the renewable oil feed and contains lower heteroatoms, lower fatty acids, and lower amides than the renewable oil feed. In some embodiments, the pressure is in a range selected from the group consisting of: 1000 to 1.050, 1.050 to 1100, 1100 to 1150, 1150 to 1200, 1200 to 1250,1250 to 1300, 1300 to 1350, 1350 to 1400, 1400 to 1450, 1450 to 1500, 1500 to 1550, 1550 to 1600, 1600 to 1650, 1650 to 1700, 1700 to 1750,1750 to 1800, 1800 to 1850, 1850 to 1900, 1900 to 1950, and 1950 to 2000 psig. In other embodiments, the hydroprocessing is performed at a temperature in the range of 300 to 500 degrees C. In yet other embodiments, the temperature is in a range selected from a group consisting of: 300 to 310, 310 to 320, 320 to 330, 330 to 340, 340 to 350, 350 to 360, 360 to 370, 370 to 380, 380 to 390, 390 to 400, 400 to 410, 410 to 420, 420 to 430, 430 to 440, 440 to 450, 450 to 460, 460 to 470, 470 to 480, 480 to 490, and 490 to 500 degrees C. In one embodiment, the hydroprocessing increases saturated hydrocarbons from less than 10 area % in the renewable oil feed to over 70 area % in the liquid oil product, wherein area % is determined by HT GC-MS. In another embodiment, the hydroprocessing reduces nitrogen from over 3 wit-% in the renewable oil feed to less than 2 wt-% in the liquid oil product, reduces oxygen from over 5 wt% in the renewable oil feed to less than or equal to 0.5 wt-% in the liquid oil product, reduces free fatty acids from 15 to 60 area -% in the renewable oil feed to less than 0.05 wt-% in the liquid oil product and wherein the hydroprocessing increases saturated hydrocarbons from less than 10 area % in the renewable oil feed to over 60 area % in the liquid oil product, wherein area % is determined by HT GC-MS. In yet another embodiment, the hydroprocessing step further reduces acid amides from over 8 wt-% in the renewable oil feed to less than 0.05 wt-% in the liquid oil product In one embodiment, the renewable oil feed contains greater than 80 mass % 630 degrees F + TBP boiling material and the liquid oil product has been cracked in the hydroprocessing step and contains less than 70 mass % 630 degrees F + TBP boiling material. In another embodiment, the renewable oil feed contains less than 10 mass % 400 - 630 degrees F TBP boiling material and the liquid oil product has been cracked in the hydroprocessing step and contains greater than 30 mass % 400 - 630 degrees F TBP boiling material. In yet another embodiment, the renewable oil feed contains less than 10 mass % 400 - 630 degrees F TBP boiling material and the liquid oil product has been cracked in the hydroprocessing step and contains greater than 40 mass % 400 - 630 degrees F TBP boiling material In one embodiment, the liquid oil product has been cracked in the hydroprocessing step and contains 3-6 mass-% boiling at 260 to 400 degrees F, 6 to 10 mass-% boiling at 400 to 490 degrees F, 20 to 40 mass-% boiling at 490 to 630 degrees F, and 35 to 40 mass-% boiling at 630 to 1020 degrees, and 10 to 27 mass-% boiling at 1020 + degrees F. In yet another embodiment, the hydroprocessing effluent is distilled into fractions and at least one fraction is recycled for additional hydroprocessing to further crack the at least one fraction. In another embodiment, the renewable oil feed is an algae oil extracted from algae biomass.

### BRIEF DESCRIPTION OF THE DRAWINGS

These and other features, aspects, and advantages of the present invention will become better understood with regard to the following description, appended claims and accompanying figures, where:
**Figure 1** is a photo of oil upgraded product samples from the seven experiments ("runs" 1SEBR, 2SEBR, 3SEBR, 4SEBR, 5SEBR, 6SEBR, and 7SEBR, left to right) of EXAMPLE I of this disclosure, wherein the processes may be some but not the only embodiments of the invention.
**Figure 2** is a distillation curve overlay, of three of the experiments of **Figure 1****,** that is, 1 SEBR- 3 SEBR, compared to the algae oil feed.
**Figure 3** **is** a distillation curve overlay, of three other of the experiments of **Figure 1**, that is, 4 SEBR- 6 SEBR, compared to the algae oil feed.
**Figure 4** is a distillation curve overlay, of the one thermal experiment (without catalyst) of **Figure 1****,** that is 7 SEBR compared to the algae oil feed.
**Figure 5** is **a** bar-graph showing % mass fraction of 6 fractions commonly used in petroleum refining, for the oil products of 1 SEBR - 3 SEBR of **Figure 1** and **Figure 2** compared to algae oil feed.
**Figure 6** is a graph showing % mass fraction for the 1 SEBR - 3 SEBR oil products and algae oil feed of **Figure 5****.**
**Figure 7** **is** a har-graph showing % mass fraction of the 6 fractions commonly used in petroleum refining, for the oil products of 4 SEBR - 6 SEBR of **Figure 1** and **Figure 3** compared to algae oil feed.
**Figure 8** is a graph showing % mass fraction for the 4 SEBR - 6 SEBR oil products and algae oil feed of **Figure 7****.**
**Figure 9** is a bar-graph showing % mass fraction of 6 fractions, for the oil product of 7 SEBR of **Figure 1** and **Figure 4** compared to algae oil feed.
**Figures 10** is a graph showing % mass fraction for the 7 SEBR oil products and algae oil feed of Figure 9.
**Figure 11** is a HT GC-MS chromatogram of the algae oil feed of EXAMPLE I and EXAMPLE II.
**Figure 12** is a HT GC-MS chromatogram of the oil product of 1 SEBR.
**Figure 13** is a HT GC-MS chromatogram of the oil product of 3 SEBR.
**Figure 14** is a HT GC-MS chromatogram of the oil product of 5 SEBR.
**Figure 15** is a HT GC-MS chromatogram of the oil product of 7 SEBR.
**Figure 16** is a bar-graph of peak area % of compound groups, measured by HT GC-MS, in oil products from 1 SEBR - 3 SEBR, compared to the algae oil feed
**Figure 17** is a bar-graph of peak area % of compound groups, measured by HT GC-MS, in oil products from 4 SEBR - 6 SEBR, compared to the algae oil feed
**Figure 18** is a bar-graph of peak area % of compound groups, measured by HT GC-MS, in oil product from 7 SEBR, compared to the algae oil feed.
**Figure 19** is a comparison of HT GC-MS chromatograms of the oil products from 1 SEBR (EXAMPLE I) and 8 SEBR (EXAMPLE II), illustrating the effect of a temperature change from 350 degrees C (1 SEBR) to 375 degrees C (8 SEBR).
**Figure 20** is a comparison of HT GC-MS chromatograms of the oil products from 4 SEBR (EXAMPLE I) and 9 SEBR (EXAMPLE II), illustrating the effect of a residence time change from 1 hour (4 SEBR) to 2 hours (9 SEBR).
**Figure 21** is a comparison of HT GC-MS chromatograms of the oil products from 4 SEBR (EXAMPLES I) and 10 SEBR (EXAMPLES II), illustrating the effect of a pressure change from 1000 psig (4 SEBR) to 1950 psig (10 SEBR).
**Figure 22** is a bar-graph of peak area % of compound groups, measured by HT GC-MS, in oil products from 1 SEBR) (EXAMPLE I) and 8 SEBR (EXAMPLE II), compared to the algae oil feed.
**Figure 23** is a bar-graph of peak area % of compound groups, measured by HT GC-MS, in oil products from 4 SEBR (EXAMPLE I), 9 SEBR (EXAMPLE II) and 10 SEBR (EXAMPLE II), compared to the algae oil feed.
**Figure 24** is a graph comparing chromatograms ("fingerprints"), compound groups, and elemental analysis of an algae oil feed according to one embodiment of the invention and an example HVGO. Figure 24 shows that algae oil feed is in the HVGO boiling point range.
**Figure 25** is a Boduszynski Plot (C number versus AEBP), modified to include an indication (arrow) of the location of an algae oil feed according to many embodiments of the invention.
**Figure 26** is a graph comparing chromatograms ("fingerprints"), compound groups, and elemental analysis of the oil product from Run 6 SEBR (EXAMPLE I) and an example jet-A fuel.
**Figure 27** is a graph comparing chromatograms ("fingerprints") of the oil product from Run 8 SEBR (EXAMPLE II) and an example jet-A fuel.
**Figure 28** is a plot of weight percent conversion vs. catalyst/oil ratio for the MAT testing of EXAMPLE III, which is predictive of performance in a fluidized catalytic cracking (FCC) unit, for a *Nannochloropsis-derived* crude algae oil and a reference vacuum gas oil (VGO).
**Figure 29** **is** a plot of coke yield (wt%) vs. conversion (wt%) from the MAT testing of EXAMPLE III for the two feedstocks of **Figure 28****.**
**Figure 30** is a plot of weight percent conversion vs. catalyst/oil ratio for the MAT testing of EXAMPLE III, wherein MAT test results using the three hydrotreated algae oils of EXAMPLE I are added to **Figure 28****.**
**Figure 31** is a plot of coke yield (wt%) vs. conversion (wt%), wherein test results for the three hydrotreated algae oils of EXAMPLE I are added to the plot of **Figure 29****.**
**Figure 32** **-** **Figure 37** are plots of the yields from the MAT testing of EXAMPLE III, specifically gasoline yield **(****Figure 32****),** LCO yield **(****Figure 33****),** DCO yield **(****Figure 34****),** and TC2, TC3 AND TC4 in **Figure 35****,** **Figure 36****,** and **Figure 37****,** respectively.
**Figure 38A** and **B** show oil products after upgrading.
**Figures 39A****, B, C** and **D,** show boiling point distribution plots of upgraded oil products from Run 1 described in EXAMPLE VIII. The y-axis represents boiling point in degrees C and the x-axis represents mass percentage recovered.
**Figures 40A****, B, C** and **D,** show boiling point distribution plots of upgraded oil products from Run 2 described in EXAMPLE VIII. The y-aris represents boiling point in degrees C and the x-axis represents mass percentage recovered.
**Figures 41A****, B, C** and **D,** show boiling point distribution plots of upgraded oil products from Run 3 described in EXAMPLE VIII. The y-axis represents boiling point in degrees C and the x-axis represents mass percentage recovered.
**Figure 42** shows a HT GC-MS analysis of Run 1 and Run 2 of EXAMPLE VIII. The first bar of each set of two bars is from Run 2 and is shown as the normalized area percent *Desmodesmus* (hexane extracted), the second bar of each set of two bars is from Run and is shown as the normalized area percent *Spirulina* (hexane extracted). The Y-axis is normalized area percent and the x-axis lists various compound types. The number of carbons increases from left to nght.
**Figure 43** shows a HT GC-MS analysis of Run 1 and Run 3 of EXAMPLE VIII. The first bar of each set of two bars is from Run 1, the second bar of each set of two bars is from Run 3. The Y-axis is normalized area percent and the x-axis lists various compound types. The number of carbons increases from left to right.
**Figure 44A** shows the boiling point distribution plot of the light fraction of Run 4. The y-axis represents percentage yield and the x-axis represents temperature in degrees C.
**Figure 44B** shows the boiling point distribution plot of the heavy fraction of Run 4. The y-axis represents percentage **yield** and the x-axis represents temperature in degrees C.
**Figure 45A** shows the boiling point distribution plot of the light fraction of Run 5. The y-axis represents percentage yield and the x-axis represents temperature in degrees C.
**Figure 45B** shows the boiling point distribution plot of the heavy fraction of Run 5. The y-axis represents percentage yield and the x-axis represents temperature in degrees C.
**Figure 46A** shows a HT GC-MS chromatogram of the oil product from the light fraction of Run 4. The y-axis represents abundance and the x-axis represents time in minutes.
**Figure 46B** shows a HT GC-MS chromatogram of the oil product from the heavy fraction of Run 4. The y-axis represents abundance and the x-axis represents time in minutes.
**Figure 47A** shows a HT GC-MS chromatogram of the oil product from the light fraction of Run 5. The y-axis represents abundance and the x-axis represents time in minutes.
**Figure 47B** shows a HT GC-MS chromatogram of the oil product from the heavy fraction of Run 5. The y-axis represents abundance and the x-axis represents time in minutes.
**Figure 48** shows a HT GC-MS chromatogram of the hydrotreatcd oil product from 7SEBR. The y-axis represents abundance and the x-axis represents time in minutes.
**Figure 49** shows a HT GC-MS chromatogram of the hydrotreated oil product from 10SEBR). The y-axis represents abundance and the x-axis represents time in minutes.

### DETAILED DESCRIPTION

Referring to the following Examples, including the Tables and Figures, there are described several but not all of the methods and compositions of matter according to embodiments of the invention. Certain methods comprise thermal processing end/or catalytic hydroprocessing of algae oil feeds or fractions thereof. The methods may further comprise subsequent processing of the liquid oil products from the thermal processing and/or catalytic hydroprocessing, for example, by fluid catalytic cracking (FCC). Certain compositions of matter may comprise the products from these methods.

The algae oils fed to these various processes and combinations of processes are unusual in that they are biocrudes, containing a wide range of compounds (very different from bigh-triglyceride vegetables oils) and containing compounds rare or non-existent in fossil petroleum. The algae oil feedstocks, may be obtained from algae biomass by various methods and be of various compositions, for example, including but not necessarily limited to the methods of treatment/extraction and the compositions described in U.S. Provisional Patent Applications Serial No. 61/367,763, filed July 26,2010; Serial No. 61/432,006, filed January 12, 2011; Serial No. 61/521,687, filed August 9, 2011; and Serial No. 61/547,391, filed October 14, 2011.

The algae oil feedstocks and/or products resulting from the upgrading. processes of this disclosure may be analyzed and optimised, for example, by methods comprising the analytical methods disclosed in Application Serial 61/547391 (incorporated herein). Thermal treatment processes that may be used in embodiments of this disclosure include, for example, those disclosed in Provisional Applications Serial No. 61/504,134, filed July 1, 2011 and Serial No. 61/552,628, filed October 28, 2011.

Certain processes of this disclosure may be batch or semi-batch, for example. In large-scale processing such as in a refinery, however, most of the processes will be continuous, wherein feed and a hydrogen stream flow continuously through one or more reactors and the hydrogen is separated from the effluent and recycled to the reactor(s). The oil separated from the reactor effluent (that is, separated from gasses/light ends in the reactor effluent), will be typically fed to downstream processing, but alternatively, fractions of the oil separated from the reactor effluent may be fed to different downstream processes.

### Algae Strains and Growing Conditions for Biomass

The renewable crude oils of this disclosure may be extracted by various means from biomass that has been alive within the last 50 years. The renewable crude oil may be extracted by various means from of naturally-occurring non-vascular photosynthetic organisms and/or from genetically-modified non-vascular photosynthetic organisms. Genetically modified non-vascular photosynthetic organisms can be, for example, where the chloroplast and/or nuclear genome of an algae is transformed with a gene(s) of interest. As used herein, the term non-vascular photosynthetic organism includes, but is not limited to, algae, which may be macroalgae and/or microalgae. The term microalgae includes, for example, microalgae (such as Nannochloropsis sp.), cyanobacteria (blue-green algae), diatoms, and dinoflaggellates. Crude algae oil may be obtained from said naturally-occurring or genetically-modified algae wherein growing conditions (for example, nutrient levels, light, or the salinity of the media) are controlled or altered to obtain a desired phenotype, or to obtain a certain lipid composition or lipid panel.

In certain embodiments of this disclosure, the biomass is substantially algae, for example, over 80 wt% algae, or over 90 wt% algae, or 95 -100 wt% algae (dry weight). In the Examples of this disclosure, the algae oil feedstock is obtained from biomass that is photosynthetic algae grown in light Other embodiments, however, may comprise obtaining algae biomass or other "host organisms" that are grown in the absence of light. For example, in some instances, the host organisms may be photosynthetic organisms grown in the dark or organisms that are genetically modified in such a way that the organisms' photosynthetic capability is diminished or destroyed. In such growth conditions, where a host organism is not capable of photosynthesis (e.g., because of the absence of light and/or genetic modification), typically, the organism will be provided with the necessary nutrients to support growth in the absence of photosynthesis. For example, a culture medium in (or on) which an organism is grown, may be supplemented with any required nutrient, including an organic carbon source, nitrogen source, phosphorous source, vitamins, metals, lipids, nucleic acids, micronutrients, and/or an organism-specific requirement. Organic carbon sources include any source of carbon which the host organism is able to metabolize including, but not limited to, acetate, simple carbohydrates (e.g., glucose, sucrose, and lactose), complex carbohydrates (e.g., starch and glycogen), proteins, and lipids. Not all organisms will be able to sufficiently metabolize a particular nutrient and that nutrient mixtures may need to be modified from one organism to another in order to provide the appropriate nutrient mix. One of skill in the art would know how to determine the appropriate nutrient mix.

Several, but not the only, examples of algae from which suitable oil may be extracted are *Chlamydomonas* sp., *Dunaliella* sp., *Scenedesmus* sp., *Desmodesmus* sp., *Chlorella* sp., *Desmid* sp., and *Nannochloropsis* sp. Examples of cyanobacteria from which suitable crude oil may be obtained include *Synechococcus* sp., *Spirulina* sp., *Synechocystis* sp. *Athrospira* sp., *Prochlorococcus* sp., *Chroococcus* sp., *Gleaecapsa* sp., *Aphanocapsa* sp., *Aphanothece* sp., *Merismopadia* sp., *Microcystis* sp., *Coelosphaerium* sp., *Prochlarothrix* sp., *Oscillatoria* sp., *Trichodesmium* sp., *Microcoleus* sp., *Chmococcidiopisis* sp., *Anabaena* sp., *Aphanizomenon* sp., *Cylindrospermopsis* sp., *Cylindrospermum* sp., *Tolypothrix* sp., *Leptolyngbya* sp., *Lyngbya* sp., or *Scytonema* sp..

### Hydrothermal-Treatment of Biomass, with Acidification, for Production of Crude Algae Oil

While the renewable crude oils of this disclosure may be extracted by various means from naturally occurring non-vascular photosynthetic organisms and/or from genetically-modified non-vascular photosynthetic organisms, the algae oils of particular interest have been extracted from hydrothermally treated algae biomass. Various solvents may be used, for example, heptanes, hexanes, and/or methyl isobutyl ketone (MIBK). Certain embodiments of the hydrothermal treatment comprise an acidification step. Certain embodiments of the hydrothermal treatment comprise heating (for clarity, here, also called "heating to a first temperature"), cooling, and acidifying the biomass, followed by re-heating and solvent addition, separation of an organic phase and an aqueous phase, and removal of solvent from the organic phase to obtain an oleaginous composition. A pretreatment step optionally may be added prior to the step of heating to the first temperature, wherein the pretreatment step may comprise heating the biomass (typically the biomass and water composition of step (a) below) to a pretreatment temperature (or pretreatment temperature range) that is lower than said first temperature, and holding at about the pretreatment temperature range for a period of time. The first temperature will typically be in a range of between about 250 degrees C and about 360 degrees, as illustrated by step (b) listed below, and the pretreatment temperature will typically be in the range of between about 80 degrees C and about 220 degrees C. In certain embodiments the holding time at the pretreatment temperature range may be between about 5 minutes and about 60 minutes. In certain embodiments, acid may be added during the pretreatment step, for example, to reach a biomass-water composition pH in the range of about 3 to about 6.

The hydrothermal extraction methods used for the algae oil feed embodiments detailed in the Examples of this document were extracted from algae biomass by the processes described in U.S. Patent Application Serial No. 61/367,763, filed July 26,2010 and Serial No. 61/432,006, filed January 12,2011 (both incorporated herein). It should be noted that the extraction methods may be conducted as a batch, continuous, or combined process. Specifically, unless otherwise specified herein, the extraction procedures for the crude algae oils of the Examples were:
a) Obtaining an aqueous composition comprising said biomass and water;
b) Heating the aqueous composition in a closed reaction vessel to a first temperature between about 250 degrees C and about 360 degrees C and holding at said first temperature for a time between 0 and 60 minutes;
c) Cooling the aqueous composition of (b) to a temperature between ambient temperature and about 150 degrees C;
d) Acidifying the cooled aqueous composition of (c) to a pH from about 3.0 to less than 6.0 to produce an acidified composition;
e) Heating the acidified composition of (d) to a second temperature of between about 50 degrees C and about 150 degrees C and holding the acidified composition at said second temperature for between about 0 and about 30 minutes;
f) Adding to the acidified composition of (e) a volume of a solvent approximately equal in volume to the water in said acidified composition to produce a solvent extraction composition, wherein said solvent is sparingly soluble in water, but oleaginous compounds are at least substantially soluble in said solvent;
g) Heating the solvent extraction composition in closed reaction vessel to a third temperature of between about 60 degrees C and about 150 degrees C and holding at said third temperature for a period of between about 15 minutes and about 45 minutes;
h) Separating the solvent extraction composition into at least an organic phase and an aqueous phase;
i) Removing the organic phase from said aqueous phase; and
j) Removing the solvent from the organic phase to obtain an oleaginous composition.

Multiple solvent extractions can be performed, for example, steps f) to j) can be repeated several times. The solvent extraction composition of step g) can be heated to ambient (about 25 degrees C) to about 200 degrees C, or about 80 degrees C to about 120 degrees C.

### Characteristics of Crude Algae Oil from Hydrothermally-Treated (HTT) Biomass

As stated in the Background section, algae oils produced in commercially-scaleable and economic processes do not consist of exclusively triglycerides and fatty acids, but rather comprise a wide variety of compounds, many of which are not present in fossil petroleum or oils from oil sands, coal and shale oil. Certain of these complex crude algae oils are those obtained from the HTT methods of this disclosure. These extracted crude algae oils may be described as full boiling range algae oils, which, in this disclosure, means the oleaginous material obtained from the extraction without subsequent distillation/fractionation. If distillation/fractionation is done after extraction, various fractions of the extracted algae oil may be obtained as desired, wherein the volume of a particular fraction will be dependent upon the boiling point distribution of the full-boiling-range algae oil. Worked Examples I and II of this document utilized full-boiling-range (not fractionated) crude algae oil feeds from *Nannochloropsis salina.* It should be understood, however, that fractions of the crude algae oils may be feedstocks for certain processes of this disclosure, and/or that fractions of upgraded oil product from certain processes of this disclosure may be subsequently processed by additional processes of this disclosure or by additional physical or chemical processes.

EA and HT GC-MS methods of this disclosure (including in the Provisional Patent Applications incorporated herein), show that these complex crude algae oils comprise a wide range of compounds, including a significant amount of 1020 degrees F+ material and a significant amount of currently-unidentifiable material. EA has shown greater than 5 area % oxygen (and more typically 6-10 area % oxygen), greater than 3 area % nitrogen (and more typically 3.5 - 6 area % nitrogen), less than 2 area % sulfur (and more typically less than 1.5 area % sulfur), and hydrogen tocarbon molar ratios of greater than 1.5 (and more typically 1.6 - 2.1). Of the total peak area of HT GC-MS chromatograms, less than 10 area % has been identified as saturated hydrocarbons, less than 10 area % has been identified as aromatics, and greater than 15 area % has been identified as fatty acids. Also, of the total peak area, 1 - 2 area % has been identified as nitrides, 1 - 15 area % has been identified as amides; over 5 area % has been identified as sterols plus steroids, and 1 - 10 area % and 1-15 area % has been identified as nitrogen compounds and oxygen compounds (other than fatty acids), respectively. The complex crude algae oils of this disclosure may also contain fatty acid esters, sterols, carotenoids, tocopherols, fatty alcohols, terpenes, and other compounds, but typically only a small amount of triglycerides, for example, < 1 area %, < 0.1 area %, or < 0.01 triglycerides.

In addition, metals contained in algae oils may be of particular interest, as they will affect upgrading selections because of possible metals-caused catalyst deactivation/poisoning. Certain HTT methods disclosed in Provisional Patent Application Serial No. 61/367,763 and Serial Number No. 61/432,006 (both incorporated herein) comprise an acidification step that may reduce metals content in the crude algae oil. Examples of the metals contained in crude algae oil by various HTT methods are shown in Provisional Patent Application Serial No. 61/367,763 and 61/432,006, and metals reduction absolute values and/or percentages may be calculated therefrom.

The wide range of compound types in crude algae oils, including many compounds other than fatty acids, is unexpected in view of the relatively simple, triglyceride oils from vegetables and plants oils, and is unexpected even in view of the fatty acid moieties that might be obtained from hydrolysis of triglyceride oils. Further, this wide range of compound types may be disconcerting to petroleum refiners, whose refineries are typically designed for fossil petroleum comprising mainly saturated hydrocarbons and aromatics. The high acid, nitrogen and oxygen content, metals, heavy materials, and currently-unidentifiable materials of the crude algae oil may cause concern for fossil petroleum refiners, who avoid feedstock changes that might cause operating upsets, shortened catalyst life, and/or corrosion of equipment.

The oxygen content of these complex crude algae oils may be explained by the many carbonyl groups, mainly of fatty acids in the algae oil. A wide range of oxygen content may be seen, for example,1 - 35 wt%, but more typically oxygen content is typically 5-35 wt% and more typically 5-15 wt%. The fatty acid moieties may range, for example, from about 4 to about 30 carbon atoms, but typically 10 to 25 carbon atoms, and even more typically, 14 to 20 carbon atoms. The fatty acid moieties most commonly are saturated or contain 0, 1, 2, 3, or more double bonds (but typically fewer than six).

In the Examples, the algae oil feeds have not been subjected to any RBD processing (the refining, bleaching, and deodorizing process conventionally known and used for high-triglyceride bio-oils), nor subjected to any of the individual steps of refining, bleaching or deodorizing, after being extracted and before the upgrading processes of the Examples. Certain embodiments of this disclosure illustrate that RBD is not necessarily needed for crude algae oil upgrading to refinery feedstocks and/or upgraded products.

### Analytical Methods

The analytical methods used for the algae oil feeds and the upgraded products discussed herein are those described in detail in Provisional Patent Application Serial No. 61/547391 (incorporated herein), and with reference to data shown in Application Serial No. 61/521,687 (incorporated herein). Boiling points and boiling distribution curves were obtained by Simulated Distillation (ASTM D7169), wherein data is presented in mass percent boiling at a given temperature. Compositional analysis (compound groups and types) were obtained by gas chromatography-flame ionization detector (GC-FID) or HT GC-MS, including advanced and/or specially-modified methods and apparatus, wherein the data is reported in area percent. Compositional analysis (compound groups and types) can be obtained, for example, using various types of mass spectrometry, such as gas chromatography-mass spectrometry (GC-MS), high-temperature GC-MS (HT GC-MS), or liquid chromatography mass spectrometry. One skilled in the art would be able to choose the correct type of mass spectrometry analysis to use. Elemental analysis (EA) was obtained by using a Perkin Elmer 240 Elemental Analyzer for CHNS/O, in current state-of-the art methods related to ASTM D5291 (for C, H, N) and ASTM D1552 and D4239 (for S), as are understood by those of skill in the art. Nitrogen levels can also be determined by ASTM standard D4629.

Many of the crude algae oils of this disclosure may be described as having a broad boiling point range, for example, approximately 300 - 1350 degrees F true boiling point. It may be noted that the heavy fraction in the boiling point distribution is usually reported as 1020 degrees F+, as this is a conventional refinery vacuum distillation tower cut-point between "distillable" material and "non-distillable" material. The SIMDIST boiling point curves of this disclosure, including the Provisional Patent Applications incorporated herein, allow description of the 1020 degrees F+ material in more detail, for example, by estimating the 1020 -1200 degrees F fraction, the 1200 - FBP fraction, and the small portion above the FBP that is "non-detectable" or "non-distillable" even by SIMDIST. From the Application Serial Number 61/521,687 SIMDIST boiling curves, one may see that certain crude algae oils contain a 1020 -1200 degrees F fraction in the range of about 10-18 mass %, a 1200 - FBP fraction in the range of about 8-15 mass%, and a portion that is non-detectable/non-distillable by SIMDIST in the range of about 2 - 5 mass %. Thus, the SIMDIST data in this disclosure, including those in Application Serial Number 61/521687, may be described as including compounds up to about C-100 and having boiling points up to about 1350 degrees F, or, in other words, providing a boiling point curve of percent off (mass fraction) vs. temperature up to about 1350 degrees F. This translates to the SIMDIST equipment and methods used by Applicant providing data representing over about 95 percent of the material in the crude algae oil, but not representing the last few percent of the material, for example, about 2 - 5 mass percent of the material.

The HT GC-MS procedures and equipment used to obtain the data in this disclosure provide spectral/chromatogram data representing a large portion, but again not all, of the crude algae oil. Said HT GC-MS spectral/chromatogram data represents the crude algae oil portion boiling in a range of IBP to about 1200 degrees F, or, in other words, the entire crude algae oil except for approximately the 1200 - FBP fraction and the SIMDIST-non-detectable material over the final boiling point By again referring to the 1200 degrees F cutpoint of the SIMDIST curves in Application Serial Number 61/521687, one may describe the portion of the crude algae oil represented by the HT GC-MS spectral/chromatogram as about 80 - 90 mass percent of the crude algae oil.

Of the total peak area of the crude algae oil HT GC-MS chromatograms in this disclosure, including those in Application Serial Number 61/521687, about 50 - 75 percent of the peak area may be specifically identified and named. This means that the chromatogram is the "fingerprint" of about 80 - 90 mass percent of the crude algae oil, and about 50 - 75 percent of the fingerprint total peak area may be specifically named and categorized by compound type/class.

By this same approach, one may see from distillation curves and HT GC-MS data for upgraded algae oil products of this disclosure and the Provisional Patent Applications incorporate herein, that the upgraded algae oil products typically are lighter in boiling point than the crude algae oil, containing less 1020 degrees F+ material and less 1200 degrees F + material. Therefore, the SIMDIST curves represent about 98 -100 mass percent of the upgraded algae oil products, and the HT GC-MS chromatogram total peak area represents higher percentage (compared to that of the crude algae oil) of the upgraded algae oil products, for example, about 90-100 mass percent. About 70 - 95 area percent of the total peak area of the upgraded oil product chromatograms is identifiable.

### EXAMPLES

Now referring specifically to the following Examples, including the Tables and Figures, there are described and shown some but not the only embodiments of the invented upgrading processes and process combinations. Also shown are some but not the only embodiments of analyses and other description of the upgraded algae oil compositions of matter.

Extracted algae oils, which comprise a unique mixture of molecular species as disclosed herein, including in the Provisional Applications incorporated herein, can be upgraded by thermal or catalytic-hydroprocessing, for example, for subsequent processing in additional processes. Said additional processes may be, for example, fluid catalytic cracking units (FCC), isomerization units, reforming units, hydrocrackers, BTX units (for petrochemicals from aromatics), lube oil basestock facilities, or other refinery processes. The thermal and/or catalytic-hydroprocessing methods of this disclosure, including the optional FCC-cracking step, are unique approaches to upgrading of renewable oil, in view of the upgrading schemes currently proposed for bio-renewable oils and in view of the differences between algae `biocrude" and fossil petroleum crude oils.

### EXAMPLE I

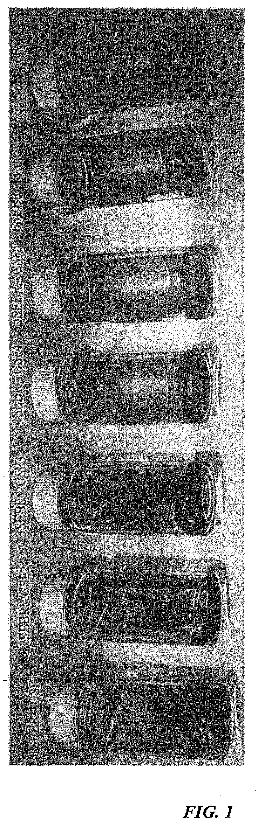

Algae oil feed was produced by hydrothermal treatment and heptane extraction of *Nannochloropsis salina,* according to the methods listed above in the section entitled "Hydrothermal-Treatment of Biomass, with Acidification, for Production of Crude Algae Oil". The hydrothermal treatment step (step b in the method listed above) was conducted at 260 C for 0.5 hour. The resulting algae oil feed was subjected to various hydroprocessing experiments, including decarboxylation, (1SEBR), hydrogenation (2SEBR), hydrogenation followed by decarboxylation (3SEBR), and three variations of catalytic-hydrotreatment (4SEBR), 5SEBR and 6SEBR). Said algae oil feed was also subjected to a thermal run in hydrogen (without catalyst, 7SEBR). The conditions and catalysts of these experiments are summarized in **Table 1,** and the oil products of these experiments are shown in **Figure 1****.**

**Table 1: Catalyst/Process Conditions**

| **1SEBR - CSF1** | **2SE6R-CSF2** | **3SEBR**-**CSF3** | **4SEBR-CSF4** | **5SEBR-CSF5** | **6SEBR-CSf6** | **7SEBR**-**CSF7** |
|---|---|---|---|---|---|---|
| decarboxylated oil | hydrogenated oil | decarboxylated product from 2SEBR | hydrotreated oil | hydrotreated oil | hydrotreated oi | hydrotreated oil |
| Ni/C, 350°C, 300 psi H_{2,}semi-batch reactor | Pd/C, 200°C, 300 psi H_{2,}semi-batch reactor | Ni/C, 350°C, 300 psi H_{2,} semi-batch reactor | Ni/Mo, 370°C, 1000 psi H₂,batch reactor | Ni/Mo, 370°C,1500 psi H₂, batch reactor | Ni/Mo, 370°C,1800 psi H₂, batch reactor | no catalyst, 370 °C, 1800 psi H₂,batch reactor |
| liquid | semi-liquid | semi-liquid | solid | solid | solid | semi-liquid |

Experimental runs 1 - 7SEBR were conducted in a semi-batch reactor (continuous flow of H2 while the oil, and catalyst when used (in all runs except 7SEBR), remained in a well stirred reactor at pressure and temperature). At the end of each 1 hour residence time run, the oil was removed using chloroform and then conducting a rotary evaporation (60C under vacuum) to recover the hydrotreated algal oil. The hydrotreated oil was then subjected to further analyses and is referred to below as the "oil product". The recovered oil and residual material adhering to the catalyst exceeded 72% in all cases with the remainder of the mass lost to the hydrogen flowing through the reactor or material adhering to the reactor. For example, the recovered oil and residual material exceeded 90% in the case of 2SEBR, 4SEBR and 5SEBR. Recovered oil as a percent of oil plus residual material adhering to the catalyst ranged from a low of 61% for 3SEBR to a high of 100% for 1 SEBR and 2SEBR and values of 75% or higher for 4-7SEBR.

Three variations of catalytic hydrotreating were conducted at the same temperature (370 degrees C) with the same catalyst, but at three pressures ranging from 1000 psi to 1800 psi. Specifically, 4SEBR, 5SEBR, and 6SEBR were conducted at 1000 psig, 1500 psig, and 1800 psig pressure, respectively. The hydrotreatment catalyst was a commercially-available NiMo/Al₂O₃ that had been pre-sulfided and handled prior to the semi-batch reaction such that re-oxidation did not occur. The NiMo/ Al₂O₃ catalyst used for these hydrotreating experiments was a sample of catalyst used for processing Canadian oil sands, believed to have a pore structure with BET surface area in the range of 150-250 m2/g, micropores in the average diameter range of 50 - 200 Angstroms, and macropores in the range of 1000 - 3000 Angstroms.

### Boiling Range and Fractions

Simulated Distillation (ASTM D7169) was used to characterize algae oil feed from *Nannochloropsis salina* and the upgraded oil products, as detailed in the Analytical Methods section above. In order to facilitate interpretation of the results, the oil products from the seven runs were divided into 3 groups, based on the upgrading conditions, and compared to the algal oil feed. The three groups were: Group 1) Decarboxylated/Hydrogenated Samples vs. Feed; Group 2) Hydrotreated Samples vs. Feed; and Group 3) No Catalyst Samples ("blank thermal run") vs. Feed.

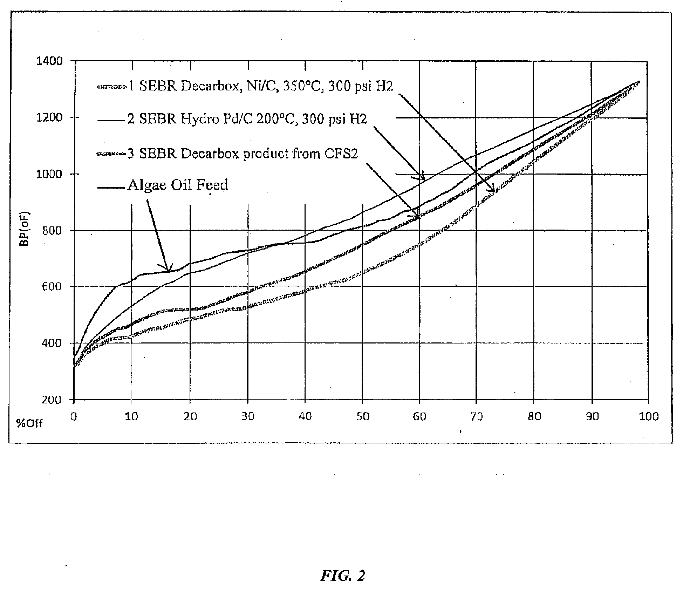

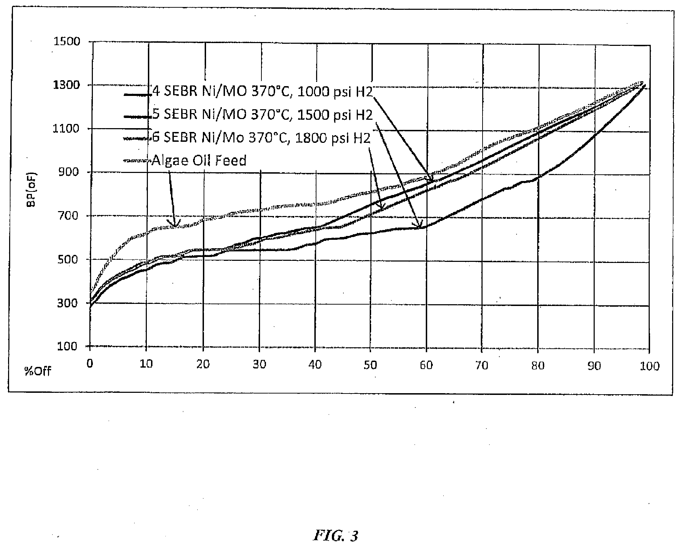

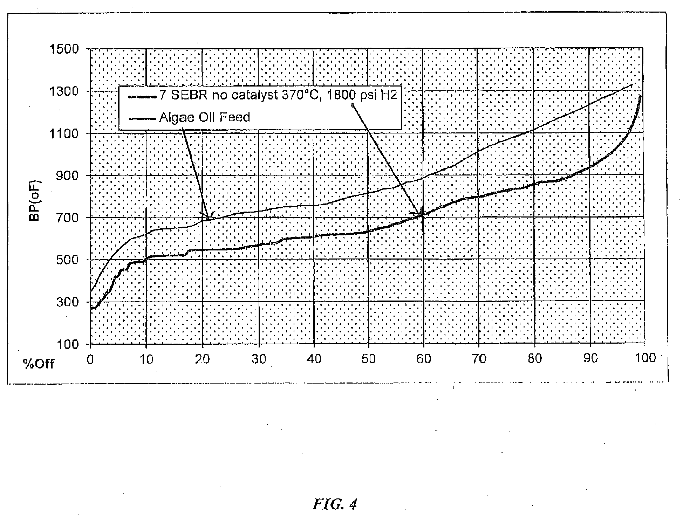

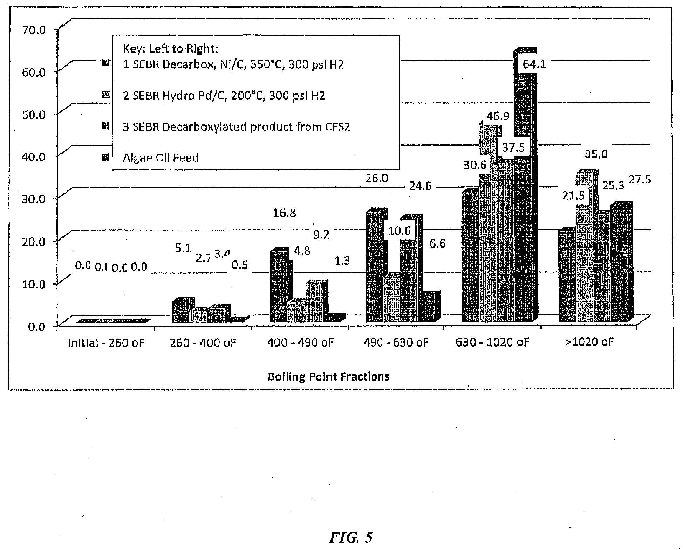

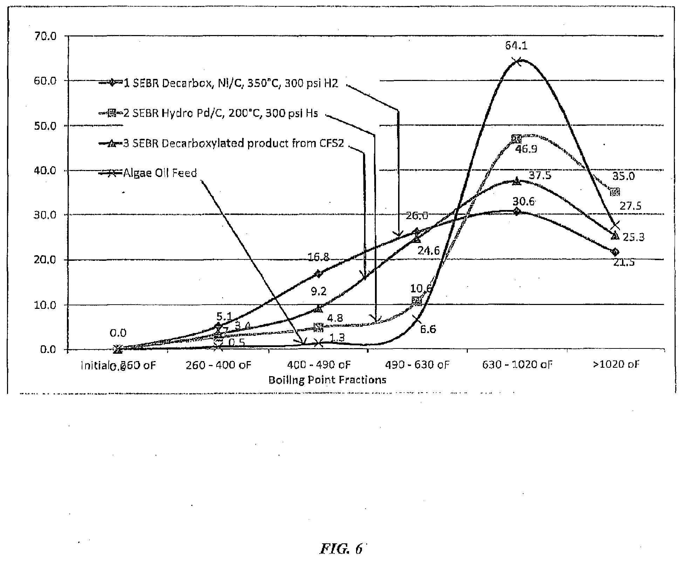

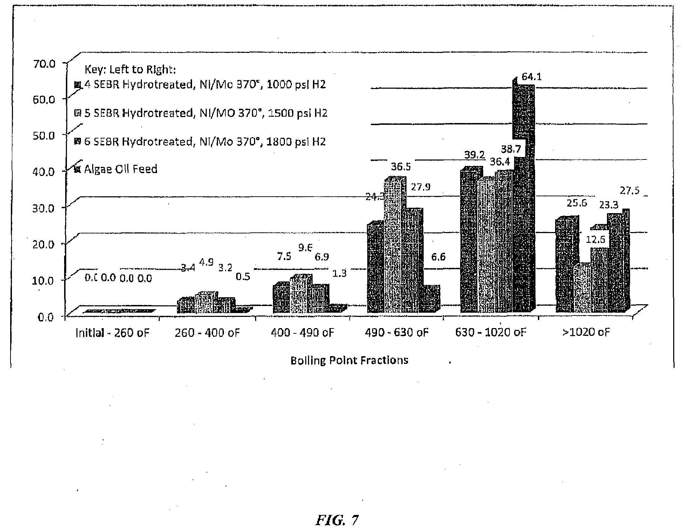

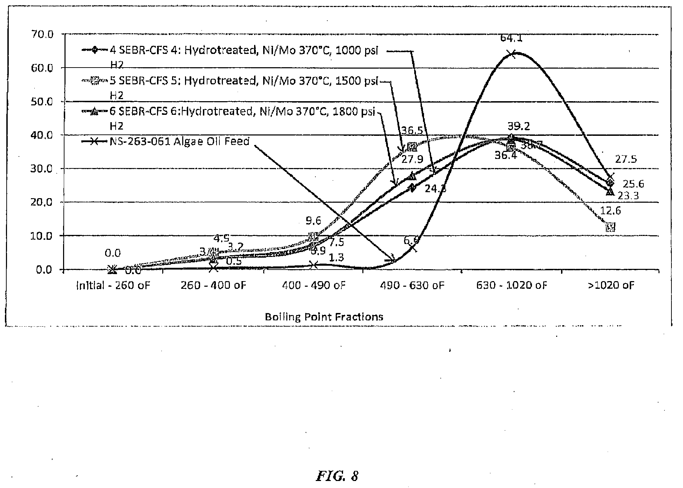

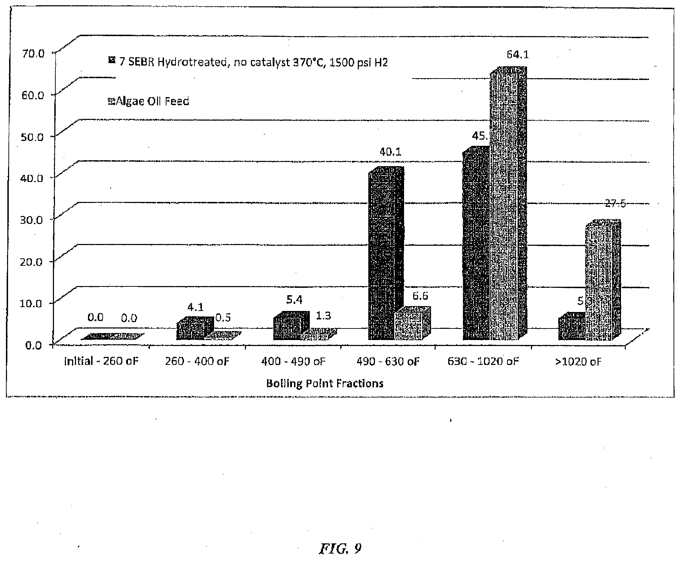

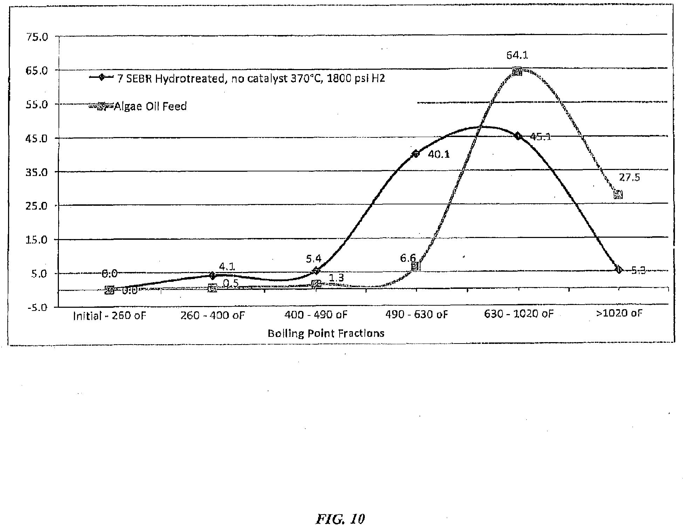

Boiling point curves for the oil products of the upgrading processes are overlayed and compared to the algae oil feed in **Figure 2** **-** **Figure 4****.** Fraction mass % values for the products are tabulated and compared to algae oil feed in **Tables 2-4.** The fraction mass % values are also graphed in **Figure 5** **-** **Figure 10****,** wherein **Figure 5** and **Figure 6** show the Group No. 1 products compared to the algae oil feed, **Figure 7** and Figure 8 show the Group No. 2 products compared to the algae oil feed, and **Figure 9** and **Figure 10** show the Group No. 3 product compared to the algae oil feed.

**Table 2: %Mass Fraction - Decarboxylatod/Hydrogenated Samples (1SEBR-3SEBR) Versus Feed**

| decarboxylated/hydrogenated samples vs. feed | FRACTION MASS % | | | | | |
|---|---|---|---|---|---|---|
| SAMPLE ID | initial-260 °F | 260 °F-400 °F | 400 °F-490 °F | 490 °F-630 °F | 640 °F-1020 °F | >1020 °F |
| 1 SEBR-CFS 1:decarbox. Ni/C, 350°C, 300 psi H₂ | 0.0 | 5.1 | 16.8 | 26.0 | 30.6 | 21.5 |
| 2 SEBR-CFS 2:hydro Pd/C, 200°C, 300 psi H₂ | 0.0 | 2.7 | 4.8 | 10.6 | 46.9 | 35.0 |
| 3 SEBR-CFS 3:decarboxylated product from CFS2 | 0.0 | 3.4 | 9.2 | 24.6 | 37.5 | 25.3 |
| NS-263-061 algae oil feed | 0.0 | 0.5 | 1.3 | 6.6 | 64.1 | 27.5 |

**Table 3: % Mass Fraction - Decarboxylated/Hydrogenated Samples (4SEBR-6SEBR) Versus Feed**

| hydrotreated samples vs. feed | FRACTION MASS % | | | | | |
|---|---|---|---|---|---|---|
| SAMPLE ID | initial-260 °F | 260°F-400 °F | 400 °F-490 °F | 490 °F-630 °F | 640 °F-1020 °F | >1020 °F |
| 4 SEBR-CFS 4: Hydrotreated, Ni/Mo 370°C, 1000 psi H₂ | 0.0 | 3.4 | 7.5 | 24.3 | 39.2 | 25.6 |
| 5 SEBR-CFS 5: Hydrotreated, Ni/Mo 370°C, 1500 psi H₂ | 0.0 | 4.9 | 9.6 | 36.5 | 36.4 | 12.6 |
| 6 SEBR-CFS 6:Hydrotreated, Ni/Mo 370°C, 1800 psi H₂ | 0.0 | 3.2 | 6.9 | 27.9 | 38.7 | 23.3 |
| NS-263-061 algae oil feed | 0.0 | 0.5 | 1.3 | 6.6 | 64.1 | 27.5 |

**Table 4: % Mass Fraction - No Catalyst Sample (7SEBR) Versus Feed**

| no catalyst sample vs. feed | FRACTION MASS % | | | | | |
|---|---|---|---|---|---|---|
| SAMPLE ID | initial-260 °F | 260°F-400 °F | 400 °F-490 °F | 490 °F-630 °F | 640 °F-1020 °F | >1020 °F |
| 7 SEBR-CFS 7: Hydrotrcated, no catalyst 370°C, 1800 psi H₂ | 0.0 | 4.1 | 5.4 | 40.1 | 45.1 | 5.3 |
| NS-263-061 algae oil feed | 0.0 | 0.5 | 1.3 | 6.6 | 64.1 | 27.5 |

For all catalyst runs (which used NiMo/ Al₂O₃, Ni/C or Pd/C, the latter two associated with decarboxylation or hydrogenation, respectively), the mass fractions of the oil product, compared to the algae oil feed, were shifted to lower boiling point ranges. Compared to the feed, the 490 - 630 degrees F and 400 - 490 degrees F fractions showed the most significant increase. The vacuum residue in the feed was 27.5 mass %, and that fraction was slightly reduced by decarboxylation (1SEBR - 20%) and by hydrogenation followed with decarboxylation (3SEBR -10%). Hydrotreatment over a Ni/MO catalyst at 1500 psi H₂ reduced the vacuum residue by more than 50% while hydrotreatment at 1000 and 1800 psi H₂ caused reduction of only 8 to 15% (4 to 6 SEBR):

The blank thermal run without catalyst (7SEBR) was done at "maximum conditions" (to match the conditions of 6SEBR, 370 degrees C, 1800 psi H₂, except without catalyst) and showed the largest reduction of vacuum residue to lower boiling point products. It may be noted, however, that 85% of the product from this run remained in the boiling point range between 490 and 1020 degrees F.

A comparison of the boiling distribution graphs and fraction mass % values shows a desirable amount of distillate in the jet fuel and diesel boiling point range (400 - 630 degrees F) for the Ni/C decarboxylated product as well as for the 1500 psi H₂, Ni/Mo, hydrotreated product.

### Compound Groups by HT GC-MS

High Temperature GC-MS (HT GC-MS) was used to identify compounds in the algae oil feed and the seven upgraded oil products, to measure effects of the different upgrading methods and process conditions on oil quality and composition. HT GC-MS provided information at the molecular level for study of the upgrading processes in terms of deoxygenation, denitrogenation, and desulfurization, as well as olefin saturation.

The modified HT GC-MS equipment and methods used for the Examples are detailed earlier in this document and in Provisional Patent Application 61/547,391 (incorporated herein), with a summary being: Column-Zebron ZB-1HT Inferno™, Dimensions - 15 meter x 0.25 mm x 0.1 µm. Injection- PTV, GERSTEL CIS4 @ 10 °C,12°/sec to 380 °C, 0.1 %L, Split 1:10; Carrier Gas - Helium @ 1.5 mL/min (constant flow); Oven Program: 40 °C for 1 min to 380 °C @ 20 °C/min, 10 min hold; Detector - MSD, Interface @ 300 °C, Source @ 230 °C, Quad @ 150 °C; Sample: 2% in CS2. Approximately 200 peaks per sample were detected. Roughly 50% of the peaks accounting for 75% to 90% of the total peak area were identified, with a minimum match quality requirement: ≥80%. The HT GC-MS chromatograms were integrated and peak spectra (TIC) compared against the NIST08 and Wiley 9 library. Identified peaks were sorted according to the following compound classes: Hydrocarbons-Saturated, Hydrocarbons - Unsaturated, Naphthenes and Aromatics, Aromatics containing Nitrogen, Acid Amides, Nitriles, Fatty Acids, Oxygen Compounds (non Fatty Acids), Sterols/Tocopherols, and Sulfur Compounds.

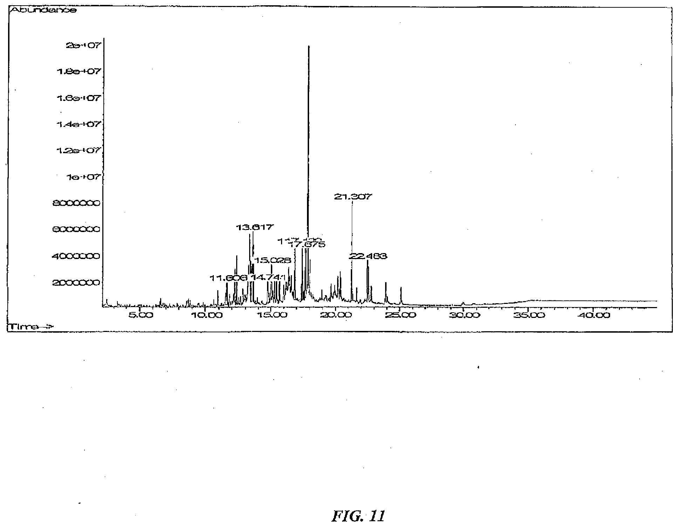

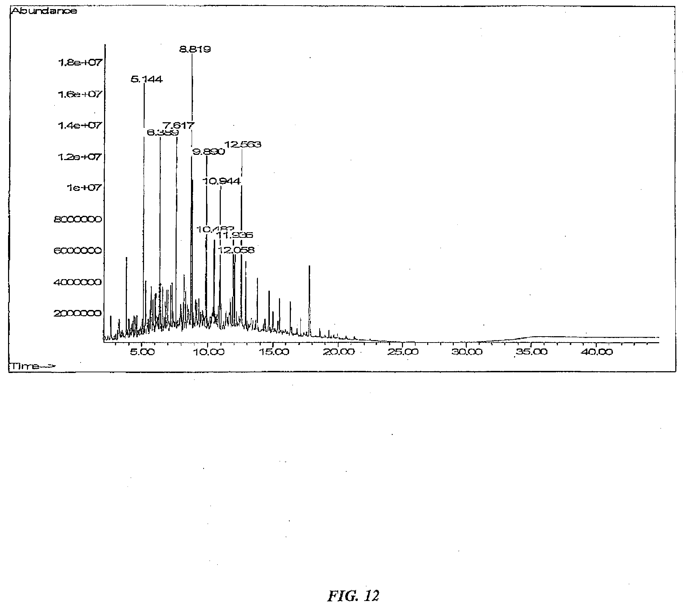

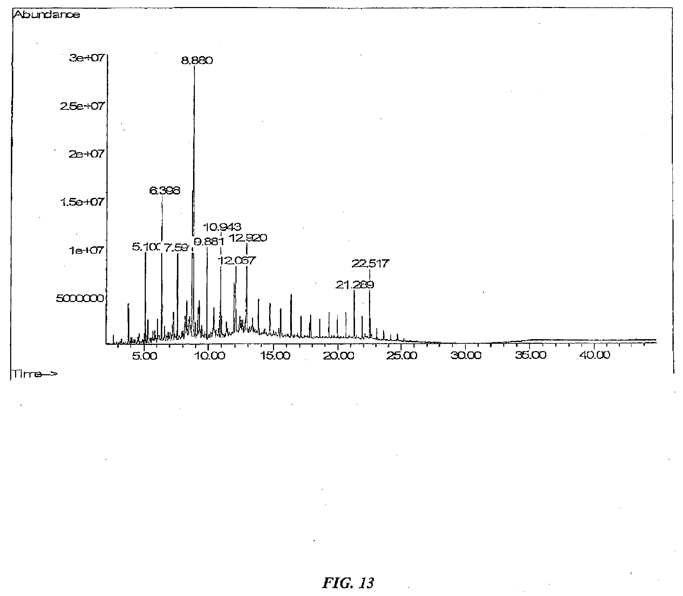

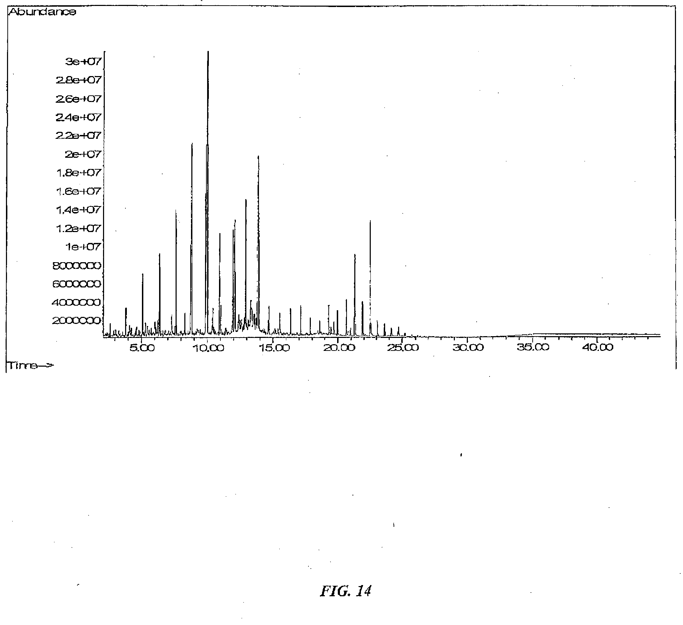

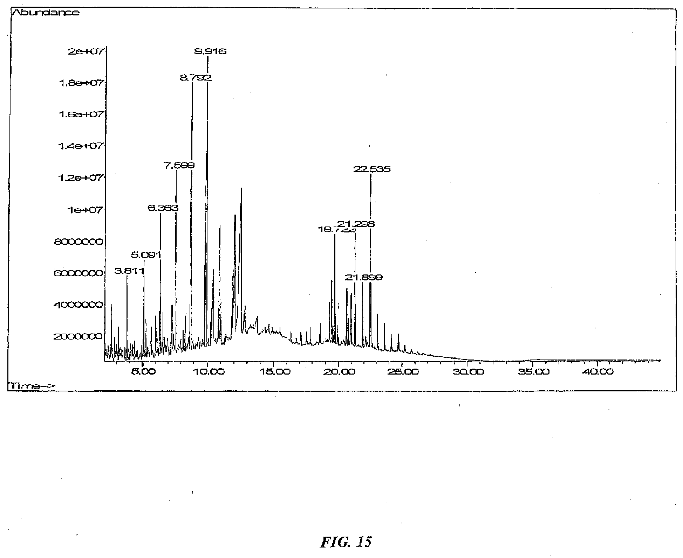

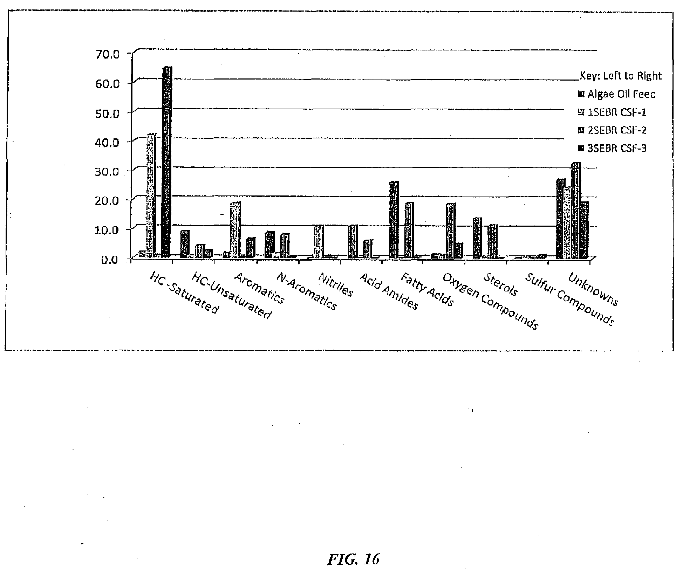

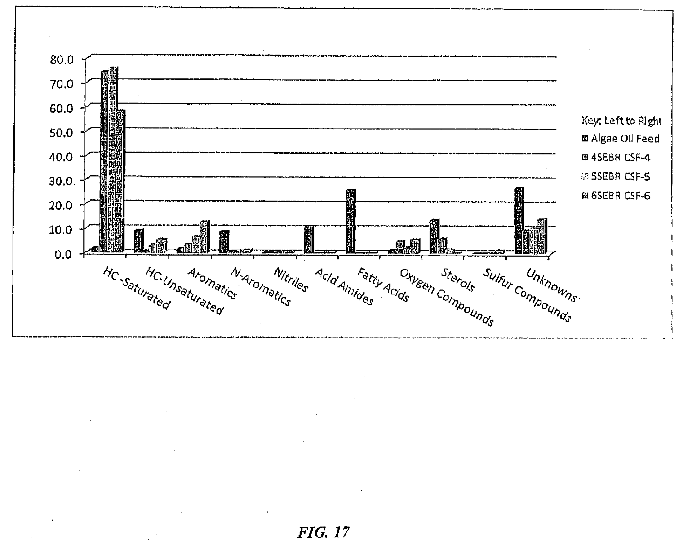

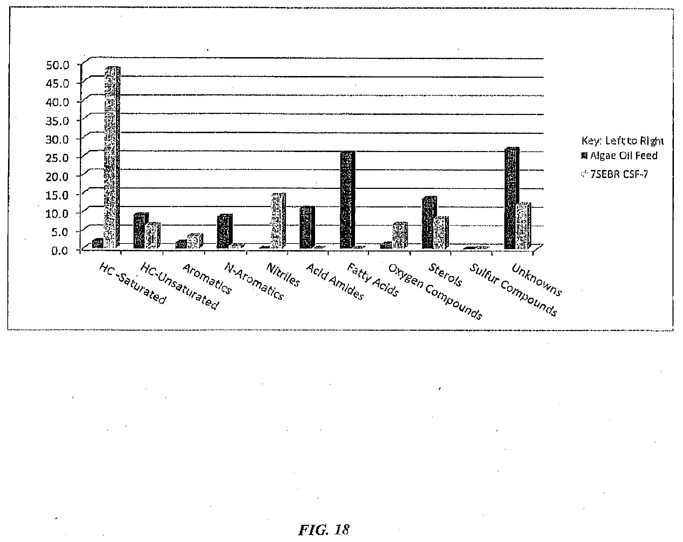

For easier comparison of the results, the seven product samples were divided into the same three groups as in the Boiling Range and Fractions section above. **Figures 11 - 15** portray, respectively, the HT GC-MS chromatograms of the algae oil feed, and the oil products from runs 1SEBR, 3SEBR), 5SEBR, and 7SEBR). **Table 5** reports compound types in the feed and products, and **Table 6** reports the most abundant compounds for the feed and products. **Figures 16 -18****,** respectively, show the products of group 1 (decarboxylated/bydrogenated products); group 2 (hydrotreated products); and group 3 (no catalyst, thermal run product), compared to the algae oil feed.

**Table 5: Compound Classes - Summary for 1SEBR-7SEBR**

| | **algae oil feed** | **1SEBR-CSF-1** | **2SEBR-CSF-2** | **3SEBR-CSF-3** | **4SEBR CSF-4** | **5SEBR CSF-5** | **6SEBR CSF-6** | **7SEBR CSF-7** |
|---|---|---|---|---|---|---|---|---|
| HC-saturated | 2.0 | 42.0 | 0.9 | 64.7 | 74.2 | 75.7 | 58.1 | 48.2 |
| HC-unsaturated | 9.1 | 1.0 | 4.3 | 2.7 | 0.9 | 3.2 | 5.5 | 6.4 |
| napthenes and aromatics | 1.7 | 18.6 | 0.3 | 6.5 | 3.5 | 6.5 | 12.6 | 3.4 |
| N-aromatics | 8.6 | 1.6 | 8.0 | 0.6 | 0.7 | 0.2 | 1.2 | 0.7 |
| nitrile | 0.0 | 10.8 | 0.0 | 0.0 | 0.0 | 0.0 | 0.0 | 14.3 |
| acid amides | 10.9 | 0.0 | 5.9 | 0.0 | 0.0 | 0.0 | 0.0 | 0.0 |
| fatty acids | 25.9 | 0.0 | 18.6 | 0.0 | 0.0 | 0.0 | 0.0 | 0.0 |
| oxygen compounds | 1.3 | 1.0 | 18.4 | 5.0 | 4.8 | 2.1 | 5.6 | 6.6 |
| sterols | 13.6 | 0.4 | 11.2 | 0.1 | 6.0 | 1.5 | 0.1 | 8.2 |
| sulfur compounds | 0.0 | 0.4 | 0.0 | 1.0 | 0.0 | 0.0 | 1.2 | 0.0 |
| unknowns | 26.9 | 24.1 | 32.6 | 19.1 | 9.5 | 10.4 | 14.0 | 12.0 |

**Table 6: Most Abundant Compounds - Summary,1SEBR-7SEBR**

| **algae oil feed** | **1SEBR-CSF1** | **2SEBR-CSF2** |
|---|---|---|
| 11.21 cis-9-hexadecenoic acid | 6.98 pentadecane | 9.64 isoprapyl linoleate |
| 8.71 pentadecane nitrile | 5.11 dodecane | 6.4 n-hexadecanoic acid |
| 8.3 n-hexadecanoic acid | 4.07 pentadecane nitrile | 4.5 4-nitro-1H pyrazolo-3-carboxylic a |
| 6.5 hexadecane | 3.63 tridecane | 3.84 cholesterol |
| 5.31 cholesterol | 3.53 tetradecane | 3.83 4,5-diamino-1,3-pyrimidin-2,6-dio |

| **3SEBR-SCF3** | **4SEBR-CSF4** | **5SEBR-CSF5** |
|---|---|---|
| 16.79 pentadecane | 16.54 hexadecane | 16.29 hexadecane |
| 4.1 tridecane | 8.08 eicosane | 8.42 eicosane |
| 3.03 nonadecane | 8.06 pentadecane | 7.53 pentadecane |
| 2.67 heptadecane | 5.25 nonadecane | 4.81 nonadecane |
| 2.51 hexadecane 2,6,10,14-tetramethyl- | 4.24 tetracosane | 3.94 heptadecane |

| **6SEBR-SCF6** | **7SEBR-CSF7** | |
|---|---|---|
| 10.56 hexadecane | 8.71 pentadecane nitrile | |
| 5.59 eicosane | 6.5 hexadecane | |
| 3.62 pentadecane | 4.32 pentadecnae | |
| 3.57 nonadecane | 2.98 hexadecane, 2,6,10,14-tetramethyl- | |
| 3.21 hexadecane, 2,6,10,14-tetramethyl- | 2.7 octadecane, 1-iodo | |

In all runs with the exception of 2SEBR (Pd/C - 200 °C, 300 psi H₂, the fatty acids of the algae oil feed were practically completely removed. This fatty-acid removal occurred even in the thermal run (7SEBR).

Run 1SEBR (Ni/C, 350 oC, 300 psi H₂) and Run 7SEBR (thermal) showed some removal of oxygen and complete removal of fatty acids but with some levels of oxygenated compounds remaining, that is, primarily esters, alcohols, and aldehydes. The oil products of 1 SEBR and 7SEBR also showed the conversion of nitrogen compounds, that is, primarily amides into nitriles. That conversion into nitriles was not observed in any other run/samples.

Run 2SEBR (Pd/C catalyst, 200 degrees C, 300 psig) did not appear to remove any hetero atoms. Out of all the samples tested the product from 2SEBR appeared to be the closest to the original feed. The 2SEBR product still contained 18.6 area % fatty acids (25.9 area % in the feed) and about 18.4 area % other oxygenated compounds (ester, aldehyde, alcohol). The 2SEBR product also showed hardly any removal of nitrogen/oxygen, again, still remaining the closest to the feed out of all the product samples.

Run 3SEBR (decarboxylated 2SEBR) showed complete removal of fatty acids but had some oxygenated compounds remaining (5 area %). Amides were removed completely and no formation of nitriles was observed. Saturated hydrocarbons increased significantly to 64.7 area %, unsaturated HC reduced to 2.7 area %. Aromatics increased to a total of 6.5 area %, compared to over 1.7 area % in the feed.

The catalytic-hydrotreating runs, 4SEBR, 5SEBR, and 6SEBR, used NiMo/Al₂O₃ catalyst as described above, at 370 degrees C, and at 1000, 1500 and 1800 psi H₂, respectively. In general, all three samples showed complete removal of fatty acids and amides, no formation of nitriles, small levels of nitrogen aromatics remaining, as well as small levels of oxygenated compounds remaining. In other words, partial nitrogen removal appeared to take place in samples 4SEBR - 6 SEBR, where the amides were removed (without formation of nitriles) but nitrogen containing aromatics stayed behind (indoles, pyrrolidide). Nitrogen removal from aromatic compounds is more difficult than from aliphatic compounds. Removal of sterols increased with hydrogen pressure. Aliphatic hydrocarbons as large as Tetratetracontane (C44H90) were observed in these products, possibly via dimerization reactions.

Overall, the catalytic-hydrotreating runs 4SEBR - SEBR 6 showed the highest level of hetero-atom removal, particularly deoxygenation and denitrogenation, along with the highest level of saturation observed in any sample. Sample 5SEBR showed the lowest level of hetero-atoms and highest level of saturated hydrocarbons at 75.7 area %. Saturated aliphatic hydrocarbons, like hexadecane (C16H34), eicosane (C20H42), and pentadecane (C15H32), are the most abundant compounds in the catalytically-hydrotreated samples.

Run 6SEBR surprisingly reversed the trend with naphthenes/aromatics going up and saturation going down somewhat (58 area % vs. 75.7 area % for 5SEBR). This result was somewhat unexpected and may be due to the sample not being prepared homogenously, as a liquefied portion and a solid portion of the sample were observed when looking at it closely.

It should also be noted that, in the catalytically-hydrotreated samples 4SEBR to 6SEBR, the fraction of unknowns in the total HT GC-MS chromatogram peak area (quality of the library match < 80%) was reduced to the lowest values (at ∼ 10 area %).

The thermal run (7SEBR), which was in the presence of hydrogen but not of any catalyst, showed removal of the fatty acids, significant removal of oxygen (but leaving a relatively high level of oxygenated compounds, 6.6 area %), and only some nitrogen/nitriles removal. The thermal run (like sample 1SEBR) showed conversion of amides and nitrogen containing aromatics into nitriles (14.3 area %). Sterols remained at a relatively high level of 8.2 area %. Saturated hydrocarbons, similarly to 1SEBR, increased significantly compared to the blank but were not as high as in the catalytically-hydrotreated oils 4SEBR to 6SEBR.

For most samples, amides were removed completely, while the aromatic nitrogen remained at almost the same level. This observation may be expected as it is more difficult to remove aromatic nitrogen than nitrogen bound in aliphatic compounds.

It may be noted that only very few sulfur-containing compounds were detected by HT-GC/MS. Trace elemental analysis would be required to determine the total amount of sulfur compounds in the algae oil feeds and products.

### Elemental Analysis

Elemental Analysis, as described in the Analytical Methods section above, was performed on the algae oil feed and the seven product oils. The elemental analysis results are shown in **Table 7.** Note that, based on the hydrogen and carbon values in **Table 7,** the H/C mole ratios for the algae oil feed and the seven oil products are as follows: 1.65 for the algae oil feed; 1.69 for 1SEBR; 1.74 for 2SEBR; 1.73 for 3SEBR; 1.89 for 4SEBR; 1.95 for 5SFBR; 2.05 for 6SEBR; and 2.05 for 7 SEBR. Thus, it may be seen that processes of EXAMPLE I provided oil products with H/C mole ratios higher than that of the algae oil feed, for examples, with increases in the mole ratio in the ranges of 0.1- 0.2, 0.2 - 0.3, and about 0.4. It is expected that, with adjustment of conditions and/or catalyst, the increases may be higher, for example, 0.4 - 0.5.

**Table 7**

| | **algae oil feed** | **1-SEBR)** | **2-SEBR** | **3-SEBR** | **4-SEBR** | **5-SEBR** | **6-SEBR** | **7-SEBR** |
|---|---|---|---|---|---|---|---|---|
| C | 77.9 | 80.9 | 76.7 | 77.1 | 82.9 | 82.3 | 85.0 | 82.4 |
| H | 10.7 | 11.4 | 11.1 | 11.1 | 13.1 | 13.4 | 14.5 | 12.4 |
| N | 3.9 | 4.1 | 4.6 | 4.9 | 1.5 | 1.5 | 0.7 | 3.5 |
| O | 6.8 | 1.9 | 7.8 | 7.8 | 0.5 | <0.5 | <0.5 | 1.5 |
| S | 0.37 | 0.49 | 0.43 | 0.72 | 0.70 | 0.76 | 0.45 | 0.73 |

The highest level of heteroatom removal appeared to occur in the hydrotreated oils 4SEBR - 6SEBR. Those samples appeared to have undergone the highest level of deoxygenation, denitrogenation, and desulfurization. The hydrotreated samples also exhibited the largest increase in "C" and "H", indicating the formation of saturated hydrocarbons.

It should also be noted that the decarboxylated oil (1SEBR, Ni/C, 350 degrees C, 300 psi H₂, semi-batch reactor) showed significant removal of oxygen but nitrogen remained high; nitriles were formed (based on GC/MS findings).

Samples 2SEBR and 3SEBR stayed closest to the original feed, with no significant amount of deoxygenation, denitrogenation or desulfurization observed.

Surprisingly, the thermal run 7SEBR showed significant removal of "O", down to 1.5 wt%. However, nitrogen (formation of nitriles) and sulfur in the thermal run product remained at the level of the feed.

From the EXAMPLE I EA data, it may be expected that medium-to-high severity hydrotreating conditions (increased H₂ pressure and/or increased temperature) may allow for additional heteroatom removal like nitrogen removal from aromatics. For example, hydrotreating pressures of greater than 1500 psig, and even as high as about 2000 psig may be needed. It is of particular interest that, in hydrotreating of algae oil, pressures may be needed (1500 - 2000 psig) that are comparable to hydrotreaters designed to handle difficult petroleum feeds such as coker-derived gas oils, rather than the low pressures (less than 1000 psig and typically about 500 - 800) used for virgin gas oil and virgin distillate hydrotreaters. It is also of particular interest that pressures may be needed (1500 - 2000 psig) that are much higher than those proposed (for example, about 300 - 500 psig) for high-triglyceride renewable oils.

### Carbonaceous Solids

**Table 12** shows the total solids recovered from the reactor after each of the catatytic-hydroprocessing runs 1 - 6SEBR, the catalyst charge, and (by difference) the residue on catalyst. **Table 12** also shows the total TGA weight loss, and the TGA weight loss excluding that below 150 degrees C. One may note from the data for the three hydrotreating, runs (4,5, and 6SEBR), that total TGA weight loss, and TGA weight loss excluding that below 150 degrees C, both decreased from 1000 psig to 1500 psig, and again from 1500 psig to 1800 psig. From this data, it appears that higher pressure helped prevent build-up of carbonaceous solids (for example, coke and metals) on the catalyst during hydrotreating of the algae oil feed of EXAMPLE I.

**Table 12: Comparison of Measured Amount of Residue on Catalyst with Amount Calculated from TGA Results**

| **experiment** | **solids recovered (g)** | **catalyst charge** | **residue on catalyst (by difference)(g)** | **TGA:total weight loss (g)** | **TGA:weight loss excluding that below 150°C** |
|---|---|---|---|---|---|
| 1SEBR | 0.2 | 9.2 | 0.0 | 0.4 | 0.2 |
| 3SEBR | 13.5 | 9.0 | 4.5 | 4.7 | 3.2 |
| 4SEBR | 10.3 | 7.6 | 2.7 | 2.7 | 1.7 |
| 5SEBR | 10.1 | 7.7 | 2.4 | 2.6 | 1.5 |
| 6SEBR | 9.6 | 7.5 | 2.1 | 2.0 | 1.4 |
| 7SEBR | N/A | N/A | N/A | N/A | N/A |

### EXAMPLE II

Additional experimentation was conducted using the same algae oil feed as in EXAMPLE I, from Nannochloropsis, but upgrading the feed at different process conditions. Specifically, the effects of increased reaction temperature, residence time and hydrogen pressure on the upgrading of algae oil were studied. The generated algae oil products and samples were analyzed by Elemental Analysis (EA) and High Temperature GC-MS, according to the procedures detailed earlier in this document.

**Table 8** contains the list of runs and the corresponding experimental conditions. The runs in this Example, therefore, include a decarboxylation experiment at higher temperature (375 °C) than the decarboxylation run 1 SEBR (350,°C. Thus, results from 1SEBR and 8 SEBR may be compared, as shown in **Table 8.** Run 9SEBR was a catalytic-hydrotreatment run at 1000 psig but at longer residence time (2 hours) than the catalytic-hydrotreating runs (1 hour) of EXAMPLE I; thus, results from 4SEBR and 9SEBR may be compared. Run 10SEBR was a catalytic-hydrotreatment run at 1950 psig and 1 hour residence time, and may be compared to any of the lower-pressure catalytic-hydrotreatment runs of EXAMPLES I. Runs 4SEBR, 9SEBR, and 10SEBR correspond to hydrotreatment at 1000 psi H₂ for 1Hr;1000 psi H₂ for 2Hrs; and 1950 psi H₂ for 1Hr, respectively, as shown in **Table 8.**

**Table 8: Example II-Catalyst/Process Conditions (Compared to Selected Runs from Example 1)**

| **Sample ID** | **Description** | **Variant in Experimental Condition** |
|---|---|---|
| 1SEBR | Ni/C, 350°C, 300 psi H₂, semi-batch reactor | reference |
| 8SEBR | Ni/C, 375°C, 300 psi H₂, semi-batch reactor | increased temperature |
| 4SEBR | Ni/Mo, 370°C, 1000 psi H₂, 1 hour, batch reactor | reference |
| 9SEBR | Ni/Mo, 370°C, 1000 psi H₂, 2 hours, batch reactor | increased reaction time |
| 10SEBR | Ni/Mo, 370°C, 1950 psi H₂, 1 hour, batch reactor. | increased pressure |

**Tables 9** and **10** contain the elemental analysis (EA) results from two different labs. No major change was observed in the heteroatom content of the product oils due to the increased conditions. The % C content decreased compared to the reference conditions, but the reason for that is not clear at this stage. Note that, based on **Table 9,** the H/C mole ratios for 8SEBR, 9 SEBR, and 10SEBR are 1.58,1.93, and 1.85, respectively.

**Table 9: Elemental Analysis (EA) of Example If Runs (Compared to Selected Runs from Example I) - Results from Laboratory A**

| | | **EA: Internal Results** | | | | |
|---|---|---|---|---|---|---|
| | | **1-SEBR** | **8-SEBR** | **4-SEBR** | **9-SEBR** | **10**-**SEBR** |
| **%C** | ***ave:*** | **83.19** | | 86.57 | 72.28 | 79.88 |
| **%*H*** | ***ave:*** | 11.65 | 10.06 | 13.94 | 10.76 | 12.29 |
| ***%N*** | ***ave:*** | 3.07 | 3.16 | 0.19 | 0.12 | 0.03 |
| ***%S*** | ***ave:*** | 0.37 | 0.40 | 0.72 | 0.50 | 0.52 |
| ***%O**** | ***ave:*** | 1.87 | 2.54 | 0.51 | 0.65 | 0.70 |

**Table 10: Elemental Analysis (EA) of Example II Runs (Compared to Selected Runs from Example I) -Results from Laboratory B**

| | | **EA: External Results** | | | | |
|---|---|---|---|---|---|---|
| | | **1 SEBR** | **8 SEBR** | **4 SEBR** | **9 SEBR** | **10 SEBR** |
| ***%C*** | ***ave:*** | 80.90 | 70.82 | 82.86 | 77.04 | 76.82 |
| ***%H*** | ***ave:*** | 11.40 | 9.50 | 13.10 | 12.10 | 12.96 |
| ***%N*** | ***ave:*** | 4.07 | 3.54 | 1.46 | < 0.5 | < 0.5 |
| ***%S*** | ***ave:*** | <1.544 | 0.28 | **<0**.**2035** | 0.19 | 0.26 |
| ***%O*** | ***ave:*** | 1.87 | 2.54 | 0.51 | 0.65 | 0.70 |

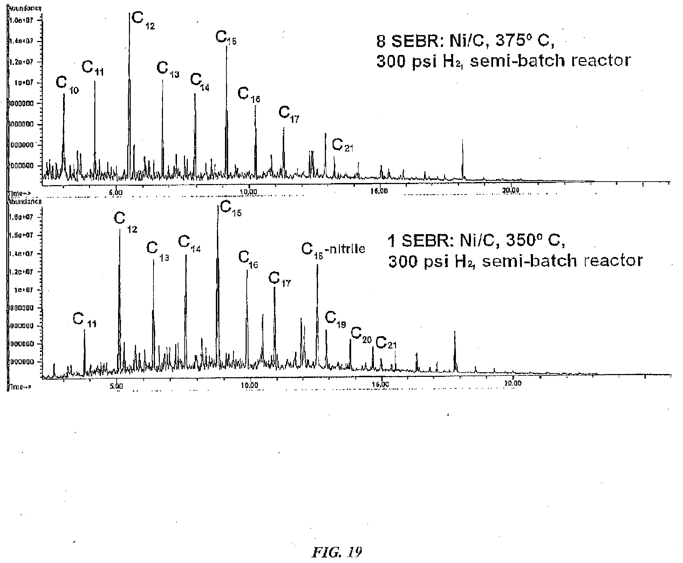

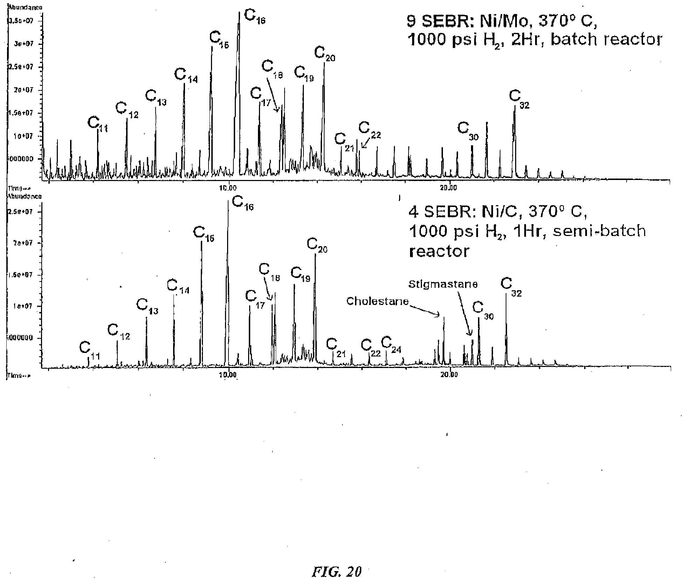

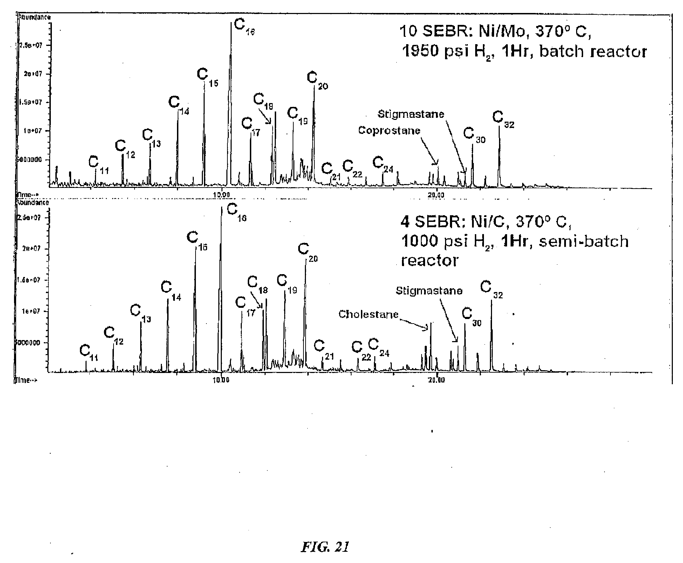

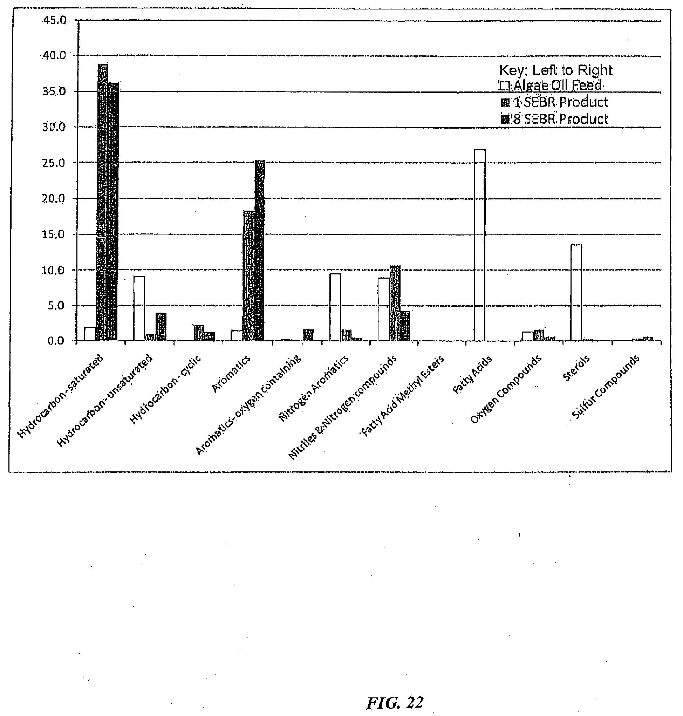

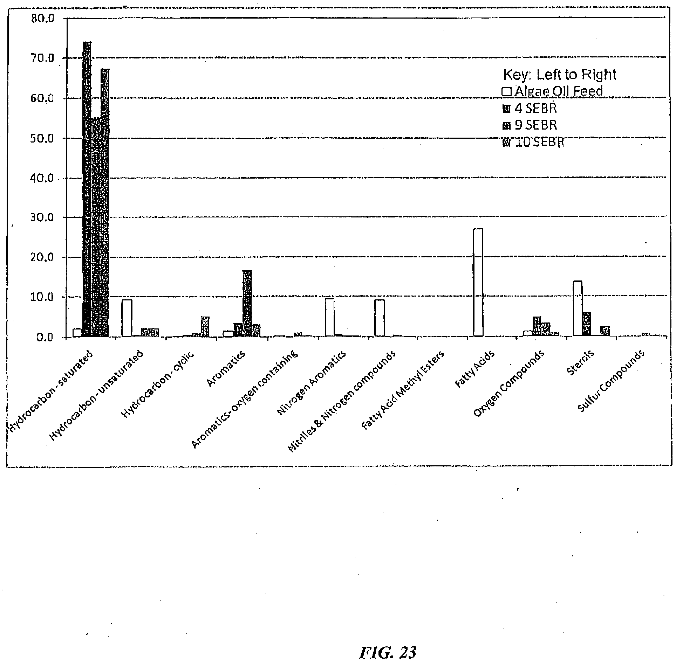

**Figures 19 - 21** show the HT- GCMS chromatograms of 8SEBR compared to 1SEBR, 9SEBR compared to 4SBBR, and 10SEBR compared to 4SEBR, respectively. **Figures 22** and **23** show distributions of the detected compound types. **Table 11** contains the breakdown of compound distributions. From these Figures and **Table 11,** it may be seen that the higher decarboxylation temperature leads to the decomposition of nitriles and the production of increased amounts of aromatics and olefins. The increased catalyst-hydrotreating reaction time leads to the increases of aromatics and the decrease of saturated hydrocarbons. The increased H₂ pressure leads to the decrease of the remaining sterols and oxygen compounds and the increase of the cyclic saturated compounds (naphthenes). These results are consistent with the expectations of the increased severity of the experimental conditions.

**Table 11: Breakdown of Compound Types in Algal Oil Feed and Products -Example II Runs Compared to Selected Example I Runs**

| | 300°C, heptane | decarboxylation | | hydrotreatment | | |
|---|---|---|---|---|---|---|
| | | (Ni/C, 300 psi H₂, semi-batch reactor) | | (Ni/Mo, 370°C, semi-batch reactor) | | |
| | algae oil feed | 350°C | 375°C | 1000 psi H₂ 1 hour | 1000 psi H₂ 2 hours | 1950 psi H₂ 1hour |
| | | **1SEBR** | **8SEBR** | **4SEBR** | **9SEBR** | **10SEBR** |
| hydrocarbon-saturated | 1.9 | 38.9 | 36.3 | 74.2 | 55.3 | 67.5 |
| hydrocarbon-unsaturated | 9.1 | 1.0 | 4.0 | 0.3 | 2.2 | 2.2 |
| hydrocarbon-cyclic | 0.0 | 2.3 | 1.4 | 0.6 | 0.9 | 5.2 |
| aromatics | 1.5 | 18.4 | 25.4 | 3.5 | 16.5 | 3.1 |
| aromatics-oxygen containing | 0.2 | 0.0 | 1.8 | 0.0 | 1.2 | 0.4 |
| nitrogen aromatics | 9.5 | 1.6 | 0.6 | 0.7 | 0.1 | 0.1 |
| nitriles and nitrogen compounds | 8.9 | 10.8 | 4.4 | 0.0 | 0.5 | 0.2 |
| fatty acid methyl esters | 0.0 | 0.0 | 0.0 | 0.0 | 0.0 | 0.0 |
| fatty acids | 26.9 | 0.0 | 0.0 | 0.0 | 0.0 | 0.0 |
| oxygen compounds | 1.3 | 1.6 | 0.7 | 4.9 | 3.6 | 1.0 |
| sterols | 13.6 | 0.4 | 0.0 | 6.0 | 0.2 | 2.6 |
| sulfur compounds | 0.0 | 0.4 | 0.7 | 0.0 | 0.8 | 0.4 |
| unknowns | 27.0 | 23.6 | 24.7 | 9.3 | 18.6 | 17.3 |
| **TOTAL=** | 99.9 | 98.9 | 99.9 | 99.5 | 99.8 | 100.0 |

### EXAMPLE III

Additional experimentation was conducted using the same algae oil feed as in EXAMPLE I, from *Nannochloropsis,* by catalytically cracking the algae oil feed in a Micro Catalytic Cracking (MAT) system. MAT equipment and tests are well known in petroleum refining R & D, and have been designed and evolved over the years to be highly correlated with large-scale fluidized catalytic cracking (FCC) units. The predictive ability of MAT tests is rather remarkable considering they require only grams of feed, whereas commercial FCC units can process over 100 million barrels per day (MBPD) of feed. The MAT tests, like commercial FCC units, operate at cracking temperatures of about 1000 degrees F and with very short catalyst-feed contact times (1 - 5 seconds), and use zeolite-based catalysts at atmospheric pressure.

In this Example, MAT testing is used to compare FCC processing of algae oil feed (crude algae oil) and FCC processing of a reference petroleum feedstock from a European refinery, specifically, a petroleum-derived vacuum gas oil (VGO) containing roughly 10 mass % resid, having an API of 22, and a sulfur level of 0.61 wt%. **Table 13** shows the yield structure in MAT testing of the standard VGO (first column of data) and the algae oil feed (second column of data), with the difference calculated and shown in the third data column. Several comments are also supplied in the third data column.

**Table 13**

| | **Yields (wt%)** | | | **Comment** |
|---|---|---|---|---|
| | standard VGO feed | extracted algae oil feed | difference algae oil - VGO | |
| C/O ratio | 1.981 | 2.008 | 0.026 | |
| conversion | 50.514 | 49.885 | -0.629 | about the same conversion extracted algae oil makes significantly less gas significantly more coke significantly more distillate range material |
| gasoline (C5-421°F) | 40.623 | 29.286 | -11.337 | |
| Coke yield | 2.296 | 10.047. | 7.751 | |
| LCO yield | 18.236 | 35.631 | 17.395 | |
| LPG yield | 6.001 | 5.536 | -0.465 | |
| H2+C1+C2+H2S | 1.512 | 3.225 | 1.713 | |
| H2+C1+C2 | 1.512 | 3.225 | 1.713 | |
| T.C3 | 3.228 | 2.313 | -0.916 | |
| T.C4 | 2.772 | 3.223 | 0.450 | |
| C4-=/tot. C4's | 0.0705 | 0.759 | 0.055 | |
| C3=/tot. C3's | 0.867 | 0.528 | -0.340 | |
| H2 | 0.148 | 0.063 | -0.085 | |
| H2S | 0.000 | 0.000 | 0.000 | |
| CH4 | 0.544 | 1.030 | 0.486 | |
| C2+ | 0.412 | 1.193 | 0.782 | |
| C2= | 0.408 | 0.938 | 0.530 | |
| C3+ | 0.428 | 1.092 | 0.664 | |
| C3= | 2.800 | 1.221 | -1.580 | |
| iC4+ | 0.674 | 0.269 | -0.405 | |
| nC4+ | 0.145 | 0.506 | 0.361 | |
| iC4= | 0.803 | 1.111 | 0.308 | |
| nC4= | 1.150 | 1.337 | 0.187 | |
| C4= | 1.953 | 2.447 | 0.494 | |
| C4= | 0.082 | 0.000 | -0.082 | |
| DCO | 31.250 | 14.484 | -16.766 | significantly less DCO |
| wt% recovery | 96.276 | 98.929 | | |

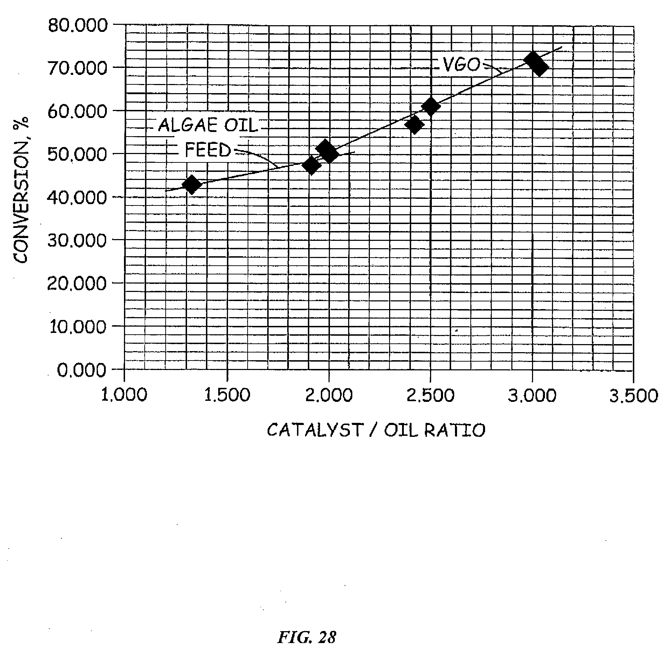

**Figure 28** compares the conversion (percent of the feed converted to distillate and to lighter components such as gasoline, plus coke) at a range of catalyst-to-oil ratios (C/O) for the algae oil feed and the reference petroleum VGO feed. In this test, the algae oil has approximately the same reactivity as the reference VGO; this may be inferred by noting that the algae oil feed has a comparable conversion of about 50% to the VGO at the same C/O ratio.

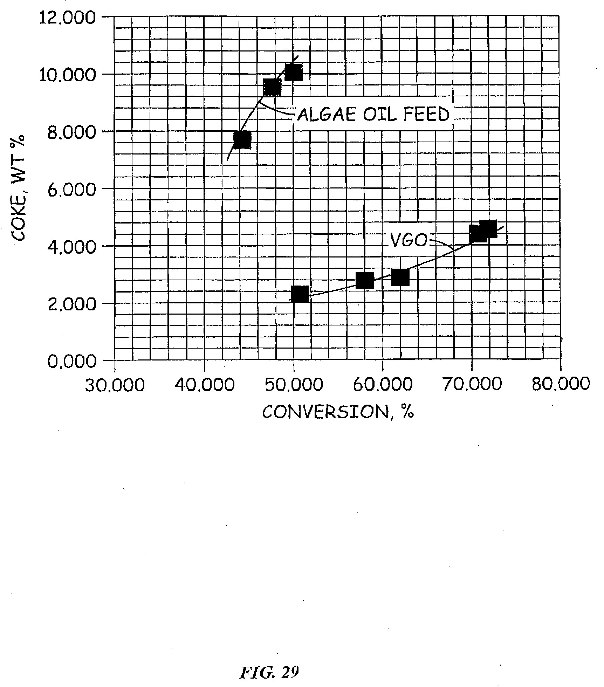

**Figure 29** shows that the coke yield for the algae oil feed is significantly higher than for the VGO. This is important because commercial-scale FCC units operate in such a way that the heat balance drives the conversion of feeds to lower levels when they have high coke yields. Consequently, the algae oil feed is expected to exhibit much lower conversion than VGO in commercial units due to its high coke yield.

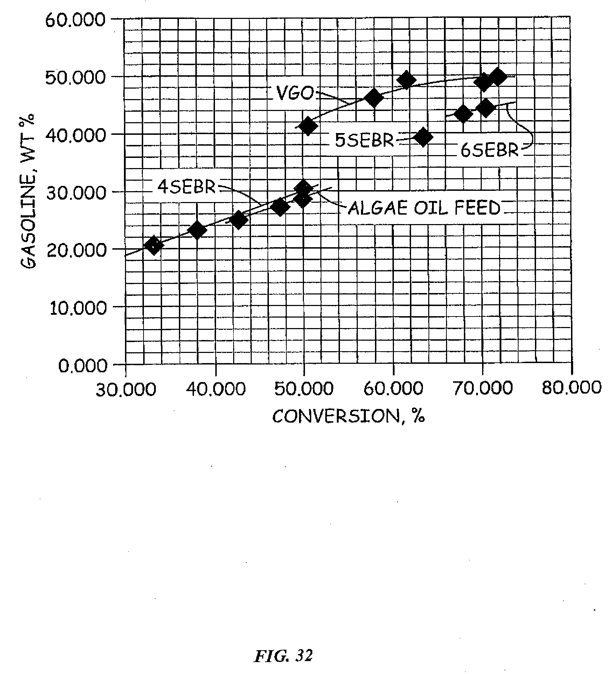

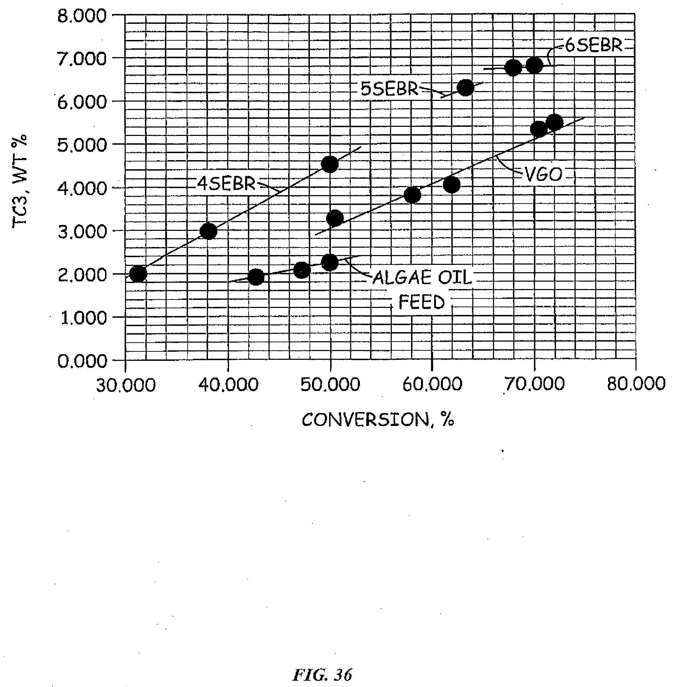

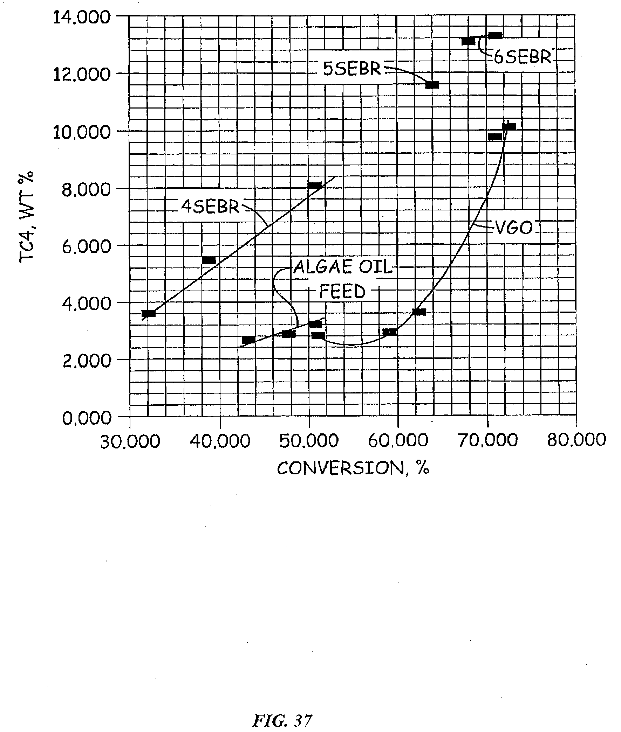

The yields of gasoline, LCO (distillate range material), DCO, TC2, TC3, and TC4 from the algae oil and VGO are shown in **Figures 32 - 37****.** The yields from hydrotreated algae oils, in EXAMPLE IV, are also shown in **Figures 32 - 37** for study of the effect of hydrotreating prior to FCC processing.

In an FCC unit, higher coke yields are favored by heavier compounds (especially 1000 degrees F+ material) and basic nitrogen-containing compounds in the feed to the unit. The later react with and poison the acidic catalytic sites in the zeolite used as the cracking catalyst, thus making coke and also reducing conversion. Oxygen-containing compounds may also contribute to increased coke yields, and, separately, to lower conversions.

Therefore, in a catalytic cracking process, the algae oil feed of this EXAMPLE exhibits coke yields that may be problematic for many FCC units. This suggests that inclusion of this unhydrotreated algae oil feed in an existing FCC unit as a significant percent of the total feed would lower the overall convention in the FCC unit due to the impact of the coke on the unit heat balance.

### EXAMPLE IV

The hydrotreated oil products of EXAMPLE I, that is, the oil products of 4SEBR, 5 SEBR, and 6SEBR, were used as feeds for catalytic cracking in the MAT system described above in EXAMPLE III. The procedures were consistent with those used for the algae oil feed vs. VGO comparison of EXAMPLE III, allowing comparison of the data from EXAMPLE III and this Example. The MAT testing, as discussed above, is predictive of commercial FCC performance. Limited oil product sample volume from 5SEBR resulted in limited MAT data for algae oil hydrotreated at 1500 psig.

**Table 14** shows the yield structure in MAT testing of the standard VGO (first column of data, as is also included in **Table 13** of EXAMPLE III) and of the high-severity-hydrotreated oil (6SEBR, second column of data), with the difference calculated and shown in the third data column. Several comments are also supplied in the fourth column.

**Table 14**

| | **Yields (wt%)** | | | **Comment** |
|---|---|---|---|---|
| | standard VGO feed | 6SEBR | difference 6SEBR - VGO | |
| C/O ratio | 3.031 | 2.475 | -0.556 | 6SEBR cracks to the same conversion at a lower C/O ration |
| conversion | 70.565 | 70.268 | -0.298 | |
| gasoline (C5-421°F) | 48.613 | 44.357 | -4.256 | 6SEBR makes slightly less gasoline |
| Coke yield | 4.492 | 4.932 | 0.440 | 6SEBR makes slightly more coke (10%) |
| LCO yield | 15.870 | 27.392 | 11.523 | 6SEBR makes significantly more distillate |
| LPG yield | 15.208 | 20.117 | 4.909 | 6SEBR makes more LPG |
| H2+C1+C2+H2S | 2.076 | 0.862 | -1.214 | |
| H2+C1+C2 | 2.076 | 0.862 | -1.214 | 6SEBR makes less fuel gas |
| T.C3 | 5.314 | 6.821 | 1.507 | 6SEBR makes more propane/propylene |
| T.C4 | 9.894 | 13.296 | 3.402 | 6SEBR makes more butane/butylene |
| C4=/tot. C4's | 0.679 | 0.652 | -0.027 | comparable butylene composition |
| C3=/tot. C3's | 0.856 | 0.874 | 0.018 | comparable propylene composition |
| H2 | 0.211 | 0.097 | -0.114 | |
| H2S | 0.010 | 0.000 | -0.010 | |
| CH4 | 0.718 | 0.230 | -0.488 | |
| C2+ | 0.587 | 0.146 | -0.441 | |
| C2= | 0.559 | 0.388 | -0.170 | |
| C3+ | 0.768 | 0.861 | 0.093 | |
| C3= | 4.546 | 5.961 | 1.414 | |
| iC4+ | 2.526 | 3.608 | 1.082 | |
| nC4+ | 0.655 | 1.019 | 0.364 | |
| iC4= | 2.225 | 2.752 | 0.528 | |
| nC4= | 4.489 | 5.916 | 1.427 | |
| C4= | 6.714 | 8.669 | 1.955 | |
| C4= | 0.177 | 0.000 | -0.177 | |
| DCO | 13.565 | 2.340 | -11.225 | |
| wt% recovery | 97.786 | 104.828 | | 6SEBR makes significantly less DCO |

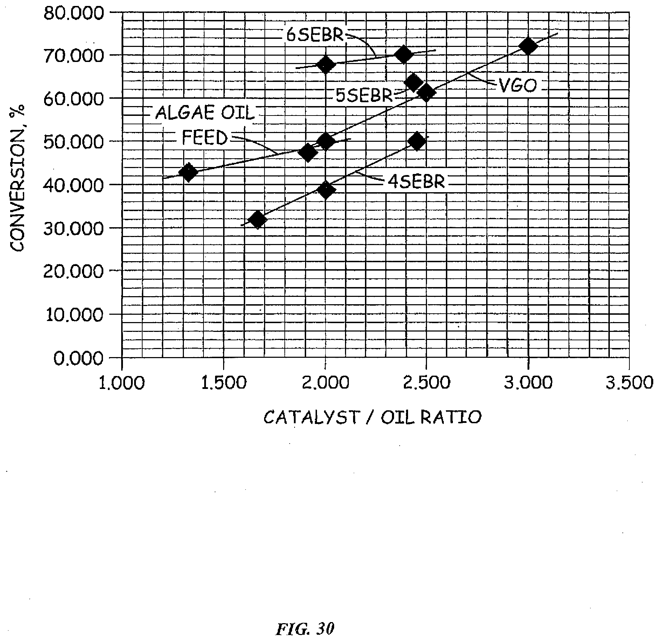

Figure 30 shows the reactivity of the three hydrotreated algae oils, compared to the algae oil feed (crude algae oil) and VGO, in the FCC process. The algae oil that had been hydrotreated at higher severity (6SEBR, 1800 psig) showed superior reactivity compared to the algae oils hydrotreated at lower severity (4 and 5SEBR), with the higher-severity-hydrotreated oil being more reactive than the VGO. That is, conversion of the high-severity-hydrotreated algae oil in the MAT test is higher than that for VGO at the same C/O range of about 2 - 2.5. The moderately-hydrotreatcd oil (5SEBR, 1500 psig) was about as reactive as the VGO, whereas the material produced from bydrotreating at 1000 psi was, very surprising, less reactive than the VGO and the crude algae oil feed.

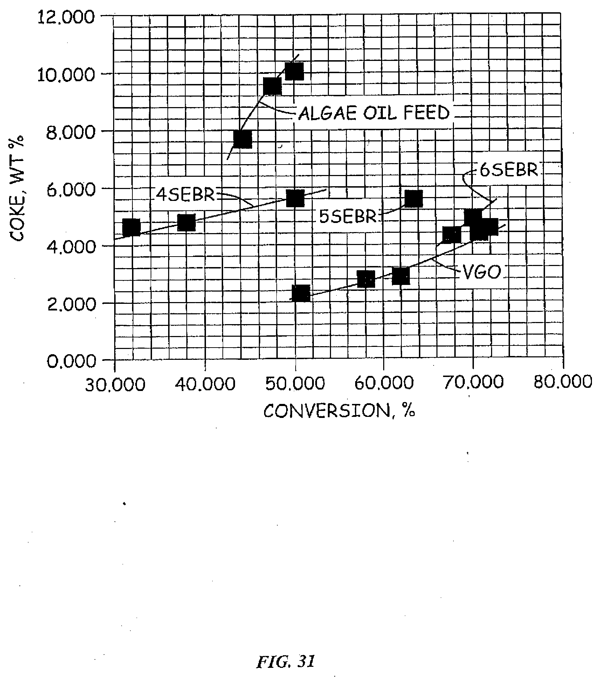

As shown in **Figure 31****,** hydrotreating improved the coke yields relative to those from the crude algae oil. The coke yield from the 1800-psig-hydrotreated algae oil was similar to that of the VGO at the same conversion of about 70 wt%.

**Figure 32 - 37** show the yields from the hydrotreated algae oils in the MAT testing. Product yields are best compared at similar conversions. Therefore, **Figures 32 -37** show weight-% yield key products (y-axis) plotted against conversion (x-axis) as obtained by varying C/O. These key yields are discussed in the following paragraph.

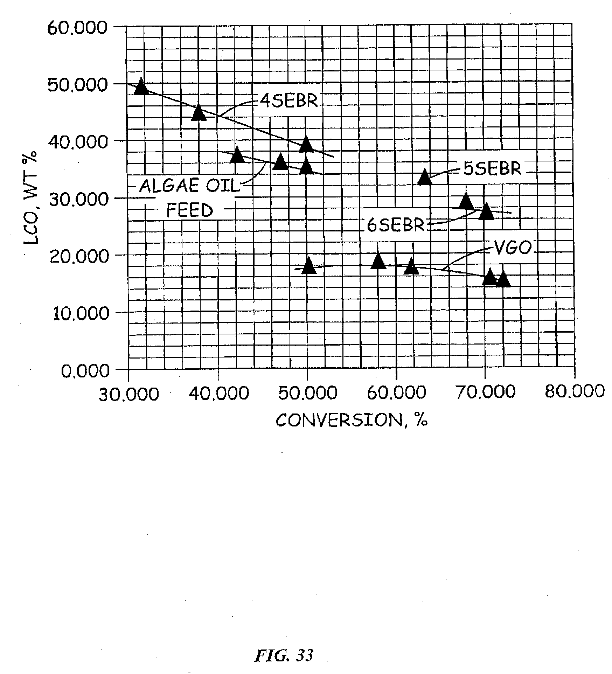

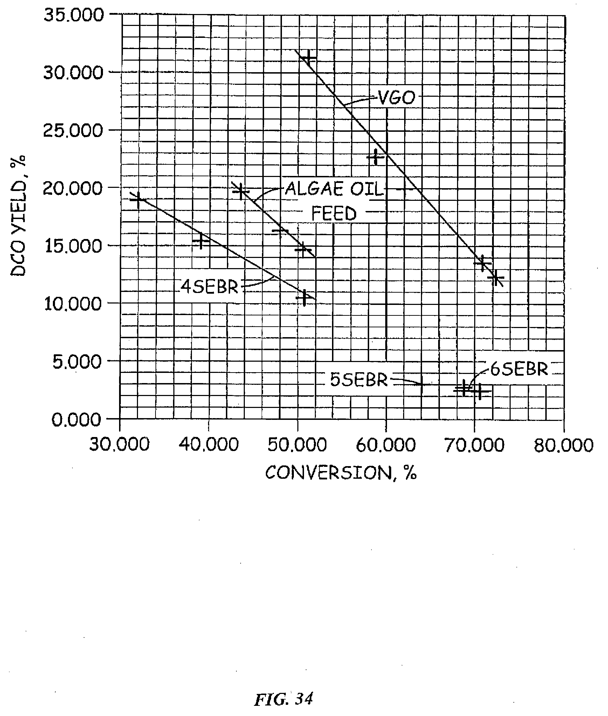

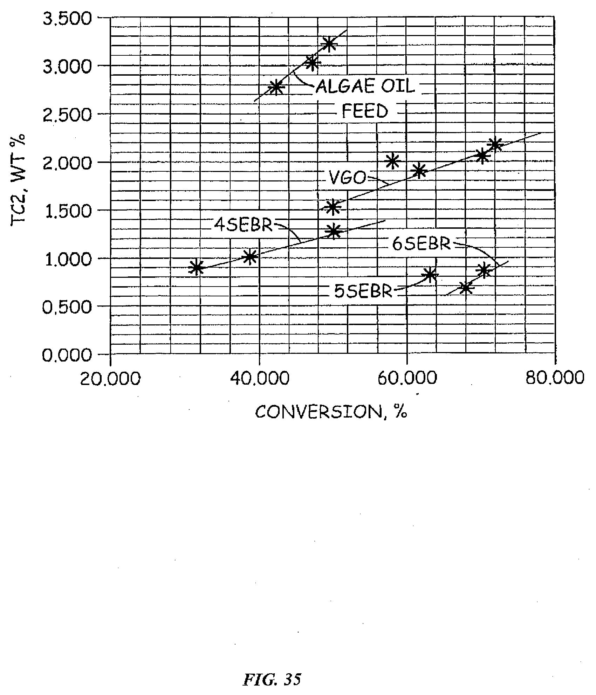

**Figure 32** shows that gasoline yields were lower from algae oil feed (crude algae oil) and its hydrotreated counterparts (the oil products from 4 - 6SEBR), compared to those from VGO at similar conversions. **Figure 33** shows that distillate, yields (LCO or "light cycle oil") were higher from algae oil feed (crude algae oil) and its hydrotreated counterparts, compared to those from VGO at similar conversions. **Figure 34** shows that DCO yields ("decanted oil", the heaviest and least-valued product from catalytic cracking) were markedly lower for from algae oil feed (crude algae oil) and its hydrotreated counterparts, compared to DCO from the VGO at similar conversions. **Figures 35 - 37** show the yields of specific components lighter than gasoline, that is, TC2, TC3, and TC4.

The yield structure obtained by MAT (FCC) testing of the high-severity-hydrotreated algae oil (6SEBR) suggest the high-severity-hydrotreated algae oil may have a higher value than VGO, even when the cost of the highpressure hydrotreating is taken into account. The higher distillate yields and reduction in gasoline yields, along with the significant reduction of low-valued DCO, all increase the value of the hydrotreated algae oil. It should be noted that the lower coke-on-FCC-catalyst of the high-severity-hydrotreated algae oil (6SEBR) helps the heat balance in the FCC, which in turn improves conversion and yields.

### EXAMPLES V

Certain crude algae oils may be hydrotreated in equipment and under conditions that are within the broad range used in one or more petroleum refineries currently or in the past However, due to the complex composition and high molecular weight constituents of said certain crude algae oils, hydrotreating said certain crude algae oils may be in a subset of this broad hydrotreating range previously developed/reserved for hydrotreating petroleum resid, for example, for 1000 or 1020 degree F+ material ("the bottom of the barrel"), oil sands or tar sands, or other mainly bitumen materials. Therefore, catalysts for hydrotreating said certain algae oils are chosen from large pore size (including macro-pore) catalysts, for example, the catalysts used for said petroleum-resid processing or oil-sands upgrading. For example, the catalyst used for hydrotreating in EXAMPLE I above was a catalyst of the type used by upgraders of the bitumen extracted from Canadian oil sands.

Said catalysts with large pore size, including macro-pores, are expected to be used with crude algae oil at high pressures, for example, at 1000 psig or higher or 1500 psig or higher(typically 1500-2000 psig). Thus, it may be noted that, even though certain algae oils contain large amounts of gas oil and distillate boiling-range-material (for example, 55 - 85 wt% or 60 - 80 wt% gas oil plus distillate), the hydrotreating pressures needed for said certain crude algae oils are expected to be significantly higher than pressures used for virgin gas oil or virgin distillate, which tend to in the 500 - 800 psig range.

Therefore, in this Example, a method of upgrading algae oil comprises:
a) obtaining a crude algae oil from algae biomass, the crude algae oil being a full boiling range algae oil comprising material in the boiling range of distillate (about 400 - 630 degrees F) and in the boiling range of gas oil (about 630 - 1020 degrees F) and in the boiling range of vacuum bottoms (about 1020 degrees F+), wherein the total of the distillate plus gas oil boiling range material is at least 55 wt%;
b) hydrotreating the crude algae oil over one or more hydrotreating catalysts adapted for hydro treatment of fossil petroleum resid/bitumen (including oil/bitumen from oil sands or tar sands), and/or over one more hydrotreating catalysts having a pore structure including macro-pores and characterized by BET surface areas in the range of 150 - 250 m2/g, micropores in the average diameter range of 50 - 200 Angstroms, and macropores in the range of 1000 - 3000 Angstroms, wherein said one or more hydrotreating catalysts may comprise Ni/Mo and/or Co/Mo on alumima or silica-alumina supports having said pore structure;
c) wherein the hydrotreating conditions are in the ranges of: 1000 - 2000 psig (or 1500 - 2000 psig, about 0.8 -1.5 1/hr LHSV (or about 1 1/hr LHSV), 300 - 425 degrees C (or 350 - 400 degrees C), with typical gas/oil ratios being at least 2000 sef/b;
d) separating, by conventional separation vessels/methods, the liquid hydrotreated oil from the hydrotreater effluent, typically meaning separating the liquid hydrotreated oil from hydrogen and gasses; and optionally:
e) sending the liquid hydrotreated oil or fractions thereof to at least one of the following: an FCC unit, a hydrocracking unit; a hydroisomerization unit, a dewaxing unit (for example, and then to a lube oil plant to separate a fraction to be used or upgraded to lube basestock), a naphtha reformer, and/or one or more units utilizing catalysts containing Ni/Mo, Co/Mo, and/or Ni/W, and/or catalysts containing metals such as platinum and other precious metals (especially group VIII), including zeolitic catalysts for improved cold flow properties.

Certain alternative embodiments may comprise step (b) instead being: hydrotreating the crude algae oil over one or more hydrotreating catalysts characterized by BET surface areas in the range of about 150 - 250 m2/g, and comprising macropores of at least 1000 Angstroms, wherein said one or more hydrotreating catalysts may comprise Ni/Mo and/or Co/Mo on alumina or silica-alumina supports having said pore structure. Certain alternative embodiments may comprise step (b) instead being: hydrotreating the crude algae oil over one or more hydrotreating catalysts comprising macropores in the range of at least about 1000 Angstroms. Certain alternative embodiments may comprise step (e) instead being selling/trading/transporting the hydrotreated algae oil to another party for subsequent processing. End products from the above processes of this Example may include one or more of gasoline, kerosene, jet fuel, diesel fuel, lube base stock, or BTX plant feedstock, for example. Certain methods of this Example may comprise, consist essentially of, or consist of method steps a-e above. Algae oils/fractions may range from very little to all of the feedstock for the processing unit(s) in steps b and e above, for example, from about 0.1 volume percent up to 100 volume percent of the liquid feedstock being fed to said processing unit(s).

### EXAMPLE VI

Certain crude algae oils may be thermally treated prior to being fed to a catalytic unit. Because of the complex composition and/or the high molecular weight materials of said certain crude algae oils (as discussed earlier in this document and the Provisional Patent Applications incorporated herein), thermal treatment prior to processing in any catalytic unit may be effective in reducing one or more of the following characteristics: oxygen content and/or other heteroatom content, metals content, high molecular weight content, 1000 degree F+ content, 1020 degree F+ content, boiling range/distribution, viscosity, and/or catalyst poisons and/or coke-on-catalyst precursors. In certain embodiments, thermal treatment will reduce most or all of these characteristics.

In certain embodiments, several of these characteristics are expected to be related to catalyst deactivation due to poisoning of catalyst active sites (such as acidic sites being poisoned by basic nitrogen compounds) and/or producing coke-on-catalyst. See, for example, the metals reduction, including reduction of known catalyst deactivators such as Fe, in **Table 3** of Provisional Application Serial No. 61/504,134. See, the solids yields, and decrease in 1020 degrees F+ materials, achieved by thermal treatment in **Tables I and 3,** respectively, of Serial No. 61/504,134. See the decrease in 1020 degrees F+ material in **Table 4** achieved by the thermal run (7SEBR) of EXAMPLE I, above. See also, the residue on catalyst, and corresponding TGA results, from the hydrotreating runs 4 - 6SEBR of EXAMPLE I in **Table 12.** These data suggest that there is significant coke-on-catalyst after the hydrotreating, experiments of EXAMPLE I, and that thermal treatment significantly affects metals and heavy materials content. Therefore, thermal treatment of whole crude algae oil is expected to mitigate, catalyst deactivation and/or coke-on-catalyst production caused by the crude algae oil, thereby extending catalyst life or improving heat balances in continuous catalyst regeneration systems such as FCC units. Also, therefore, the thermal treatment methods of this example may be used in conjunction with hydrotreating over large-pore catalysts (such as described in EXAMPLE III) to improve catalyst lives and/or heat balances in downstream units.

Thermal run 7SEBR in EXAMPLE I illustrates one embodiment of thermal treatment methods, and Provisional Patent Applications Serial No. 61/504,134 and Serial No. 61/552,628 (incorporated herein) include further embodiments of thermal treatment methods. Embodiments of coking and/or visbreaking such as have been designed for fossil petroleum also may be used. In this Example, therefore, a thermal treatment method may be applied to certain crude algae oils, the method comprising:
a) obtaining a crude algae oil from algae biomass, the crude algae oil being a full boiling range algae oil comprising material in the boiling range of distillate (about 400 - 630 degrees F) and in the boiling range of gas oil (about 630 - 1020 degrees F) and in the boiling range of vacuum bottoms (about 1020 degrees F+), wherein the total of the distillate plus gas oil boiling range material is at least 55 wt%;
b) thermally treating the crude algae oil (the whole crude algae oil) by heating the crude algae oil to a temperature in the range of 300 - 450 degrees C, with or without added gas or diluent(s), at a pressure in the range of 0 -1000 psig (and more typically 0 - 300 psig), and holding the algae oil at that temperature for a period of 0 minutes to 8 hours, and more typically 0.25 - 8 hours or 0.5 - 2 hours;
c) separating, by conventional separation vessels/methods, liquid thermally-treated oil from the thermal treatment effluent, typically meaning separating the liquid thermally-treated oil from hydrogen and gasses and from coke/solids; and
d) hydrotreating the liquid thermally-treated oil over one or more hydrotreating catalysts such as those described in EXAMPLE I and/or EXAMPLE III, and under hydrotreating conditions in the ranges described in EXAMPLE I and/or EXAMPLE III**;**
e) separating, by conventional separation vessels/methods, the liquid hydrotreated oil from the hydrotreater effluent, typically meaning separating the liquid hydrotreated oil from hydrogen and gasses; and optionally:
f) sending the liquid hydrotreated oil or fractions thereof to at least one of the following: an FCC unit, a hydrocracking unit; a hydroisomerization unit, a dewaxing unit (for example, and then to a lube oil plant to separate a fraction to be used or upgraded to lube basestock), a naphtha reformer, and/or one or more units utilizing Ni/Mo, Co/Mo, Ni/W, precious metal, noble metal, and/or group VIII catalysts, including zeolitic catalysts for improved cold flow properties.

End products from the above process may include one or more of gasoline, kerosene, jet fuel, diesel fuel, lube base stock, or BTX plant feedstock, for example. Certain methods of this example may comprise, consist essentially of, or consist of method steps a-f above. Algae oils/fractions may range from very little to all of the feedstock for the processing unit(s) in steps b, d, and f above, for example, from about 0.1 volume percent up to 100 volume percent of the liquid feedstock being fed to the processing unit(s).

### EXAMPLE VII

Fractions of certain crude algae oils may be thermally treated prior to being fed to a catalytic unit. Because of the complex composition and/or the high molecular weight materials of said certain crude algae oils (as discussed earlier in this document, the Provisional Patent Applications incorporated herein, and in EXAMPLE VI), thermal treatment of a heavy fraction of certain crude algae oils, prior to processing in any catalytic unit may be effective in reducing one or more of the following characteristics: oxygen content and/or other heteroatom content, metals content, high molecular weight content, 1000 degree F+ content, 1020 degree F+ content, boiling ratige/distribution, viscosity, and/or catalyst poisons and/or coke-on-catalyst precursors. In certain embodiments, the thermal treatment of a heavy portion of crude algae oil will reduce most or all of these characteristics in said heavy portion. In certain embodiments, several of these characteristics of said heavy portion are expected to be related to catalyst deactivation by poisoning of catalyst active sites (such as acidic sites being poisoned by basic nitrogen compounds) and/or producing coke-on-catalyst. The processing of this example is expected to mitigate deactivation of hydrotreating catalyst, generally for the same reasons as cited above in EXAMPLE VI. In this Example, however, the heavy portion of the crude algae oil is chosen for thermal treatment, rather than the whole crude algae oil, because the heavy portion (when not thermally treated) may be the major contributor to hydrotreating catalyst deactivation. Therefore, thermal treatment of said heavy^{.}portion of the crude algae oil may extend catalyst life or improving heat balances in continuous catalyst regeneration systems such as FCC units.

Whole crude algae oil may be separated into a heavy portion and a lighter portion, and thermal treatment methods for said heavy portion may be drawn from thermal run 7SEBR in EXAMPLE I, Provisional Patent Applications Serial No. 61/504,134 and Serial No. 61/552,628 (incorporated herein), or embodiments of coking and/or visbreaking such as have been designed for fossil petroleum. The resulting thermally-treated oil may be sent to a hydrotreater. The lighter portion of the crude algae oil (not thermally treated) may also be hydrotreated, for example, along with the thermally-treated oil from said heavy portion in the same hydrotreating unit. The heavy portion of the crude algae oil, for example, may be, for example, 1000 degrees F +, 1020 degrees F+ material, or another cut intended to contain a majority of the metals prone to deactivate catalyst and/or a majority of the compounds prone to deactivate catalyst by forming coke-on-catalyst that plugs pores and/or otherwise interferes with active catalyst sites. Thus, the thermal treatment methods of this example may be used in conjunction with hydrotreating over large-pore catalysts (such as described in EXAMPLE III) to improve catalyst lives and/or heat balances in downstream units.

For example, the thermal treatment method of this example, may be as follows:
a) obtaining a crude algae oil from algae biomass, and separating the crude algae oil into a heavy portion and a lighter portion;
b) thermally treating said heavy portion by heating the heavy portion to a temperature in the range of 300-450 degrees C, with or without added gas or diluent(s), at a pressure in the range of 0 -1000 psig (and more topically 0 - 300 psig), and holding the heavy portion at that temperature for a period of 0 minutes to 8 hours;
c) separating, by conventional separation vessels/methods, liquid thermally-treated oil from the thermal treatment effluent, typically meaning separating the liquid thermally-treated oil from hydrogen and gasses and from solids; and
d) hydrotreating the liquid thermally-treated oil over one or more hydrotreating catalysts such as those described in EXAMPLE III, and/or under hydrotreating conditions in the ranges described in EXAMPLE III;
e) wherein step (d) may optionally be conducted after blending said lighter portion of the crude algae oil from step (a) with said liquid thermally-treated oil of step (c) and then hydrotreating them together as in step (d);
f) separating, by conventional separation vessels/methods, the liquid hydrotreated oil from the hydrotreater effluent from either step (d) or (e), typically meaning separating the liquid hydrotreated oil from hydrogen and gasses; and optionally:
g) sending the liquid hydrotreated oil from steps (d) or (e), or fractions thereof, to at least one of the following: an FCC unit, a hydrocracking unit; a hydroisomerization unit, a dewaxing unit (for example, and then to a lube oil plant to separate a fraction to be used or upgraded to lube basestock), a naphtha reformer, and/or one or more units utilizing Ni/Mo, Co/Mo, Ni/W, precious metal, noble metal, and/or group VIII catalysts, including zeolitic catalysts for improved cold flow properties.

End products from the above process may include one or more of gasoline, kerosene, jet fuel, diesel fuel, lube base stock, or BTX plant feedstock, for example. Certain methods of this example may comprise, consist essentially of or consist of method steps a-g above. Algae oils/fractions may range from very little to all of the feedstock for the processing unit(s) in steps b, d, e and g above, for example, from about 0.1 volume percent up to 100 volume percent of the liquid feedstock being fed to the processing unit(s).

Integration of algae oils into conventional refinery flowschemes, and algae oil products into commercial fuels, may be benefitted by various embodiments of the upgrading processes demonstrated herein. For example, in the upgrading experiments of the Examples, catalytic-hydrotreatment caused a visible color/phase change of the algae oil products, compared to the algae oil feed. Notable for the hydrotreating series was that the color became successively lighter and the phase went from liquid at room temperature to a wax-like solid at room temperature. In contrast, the algae oil feed and thermally treated materials were black and liquids at room temperature. Notable was a smooth increase in H/C ratio in the series of feed to 4SEBR to 5SEBR to 6SEBR, while the oxygen and nitrogen content decreased. There was substantially less improvement in H/C ratio for the thermally treated product and also less of a reduction in N and O, compared to catalytic-hydrotreatment.

In addition, the upgrading processes describe herein provided unique compositional shifts as detailed by HT GC-MS, as shown in the Tables and Figures. Notable was the reduction in heteroatom (S, N, O)-containing molecules and changes in the types of molecules containing the heteroatoms. Also notable was the increase in the aliphatic character of the mixture.

Benefits in boiling range distribution were also noted. The full-boiling-range algae oil feed comprised a significant amount of material in each of multiple cuts traditionally produced in a crude distillation unit of a petroleum refinery. In the language of petroleum refining, the algae oil feed was a mixture of kerosene/distillate (the 400-630F boiling point range), gas oil (630F-1020F) and residuum (1020F+). Based on the boiling point distribution shifts, saturation, and hetero-atom removal, seen in the upgrading experiments, it may be stated that upgrading by conventional refinery processes may vary the amounts of these cuts (400-490F, 490-630F, 630-1020F and 1020F+) and increase the quality of these cuts, to approach or match desired refinery feedstocks or product specifications. The algae oil feed composition was improved by upgrading from an initial mixture consisting of 1.3 wt % 400-490F, 6.6% 490-630F, 64.1% 630-1020F and 27.5 % 1020F+ material to increased amounts of distillate and gas oil, at the expense of residuum. For example, experiment 5SEBR at 1500 psi hydrogen led to an upgraded product with the composition of 4.9 wt % 260-490F, 9.6% 400-490F(distillate), 36.5% 490-630F(distillate), 36.4% 630-1020F(gas oil) and 12.6 % 1020F+ (residuum). Therefore, it is expected that thermal treatment conditions and/or hydrotreating conditions (temperature, pressure, H₂ flow rate, catalyst type, reactor configuration) may be adjusted to obtain desirable feedstocks for subsequent processing units, and/or desirable product slates and product compositions. In particular, it is expected that suitable changes in process conditions will be able to create product mixtures; up to large amounts of naphtha, distillate, or gas oil. Even thermally treating the algae oil extract in the presence of hydrogen led to increases in distillate and gas oil with corresponding reductions in residuum.

It is believed that the above benefits will move the renewable oil industry closer to the goal of integrating renewable oils into conventional refineries. Given the results shown in the EXAMPLES (1-9), and the benefits described, it is expected that full-boiling-range algae oils may be directly fed to one or more refinery units downstream of the crude distillation units (that is, without being fractionated in the atmospheric or vacuum columns), for upgrading to fuel by thermal processing and/or catalytic hydroprocessing. Or, the full-boiling-range algae oils may be blended with other renewable oils and/or fossil petroleum fractions, to be fed directly to one or more refinery units. Alternatively, it is believed that the algae oils may be fed to a refinery crude unit to obtain distillate, gas oil and residuum fractions, and these corresponding fractions then would be fed to downstream thermal and/or catalytic hydroprocessing.

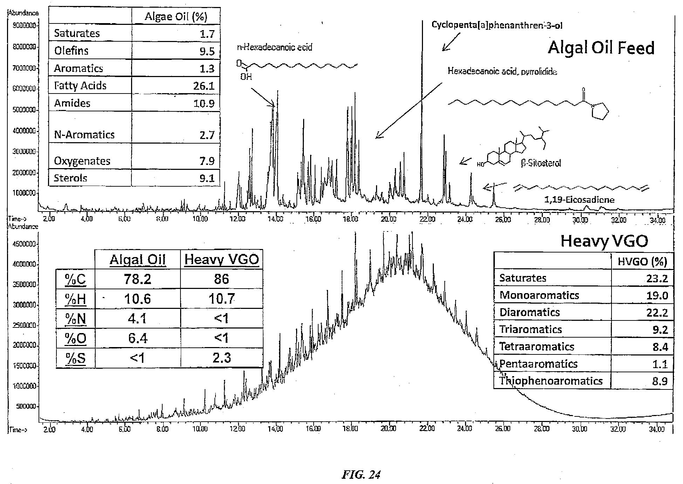

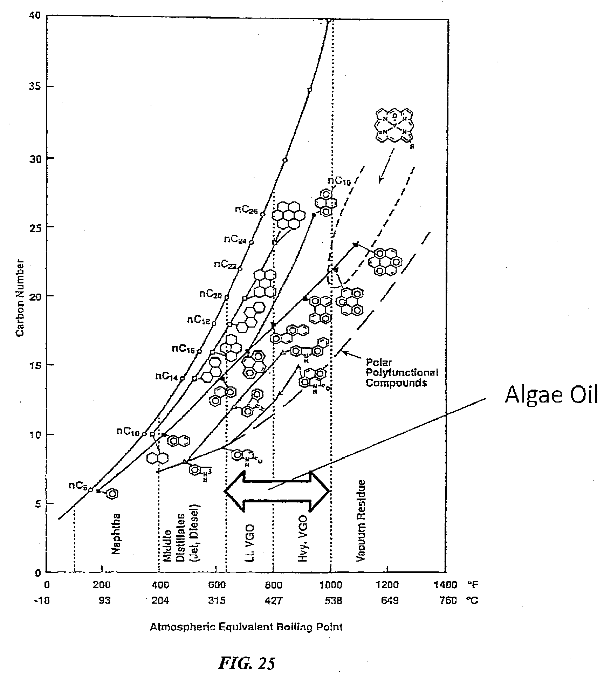

For perspective regarding upgraded algae oils in the refining and fuels context, one may refer to Figures 24 - 27. Algae oil feeds are unusual compared to conventional petroleum refinery feedstocks, as is illustrated by the modified Boduszynski Plot of Figure 25. The algae crude oils comprise a complex mixture of a large number of molecules having varying sizes and therefore varying boiling points, and comprising heteroatoms such as sulfur, nitrogen and oxygen, and also with unique types of molecules. Said unique types of molecules fall generally into the paraffin, olefin and aromatic categories often used to characterize crude oils and oils from other sources, but are significantly different from petroleum crude (including oil from tar sands, oil sands and/or shale oil) and vegetable oils and tallow in terms of the specific compounds and amounts of compound classes. An example illustrating the unusual nature of algae oil feeds is shown by the HT GC-MS fingerprint of a hydrothermally-treated and solvent-extracted algae oil in **Figure 24****.** The algae oil is compared **in** **Figure 24** to the fingerprint and composition of a typical HVGO, which has a boiling range similar to that of the algae oil, but is much different (and simpler) in fingerprint and composition.

Still; it has been shown in the above Examples that algae oil feeds may be upgraded into fuels by conventional refining approaches such as thermal processing and catalytic-hydroprocessing. Because of the unique compositions of the algae oils, the products from upgrading of these algae oils in conventional refinery units are unique. For example, algae oil feeds have been shown in the Examples to behave differently from conventional petroleum feedstocks, in that substantial conversion from one boiling point fraction to another when they are thermally processed or catalytically-hydrotreated. Feeds from fossil petroleum, on the other hand, with the above-mentioned boiling point distribution of kerosene/distillate, gas oil and residuum, would be expected, upon hydrotreatment, to have lower heteroatom content but to yield roughly the same amounts of these products/cuts as were contained in the feed.

This different behavior of the algae oil, in thermal processing and/or hydrotreatment, whether called a boiling distribution shift or cracking, may be important in achieving a flexible and high quality product slate from algae oils, whether or not they are blended with conventional fossil petroleum and/or vegetables oils. This substantial conversion to lower point fractions, when combined with recycle of unconverted fraction(s) if desired, may allow a refiner to obtain up to 80 - 100 % of a fraction selected from the list of naphtha's (butanes to 430F), distillates (430-650F), and gas oils (650-1000F), for example.

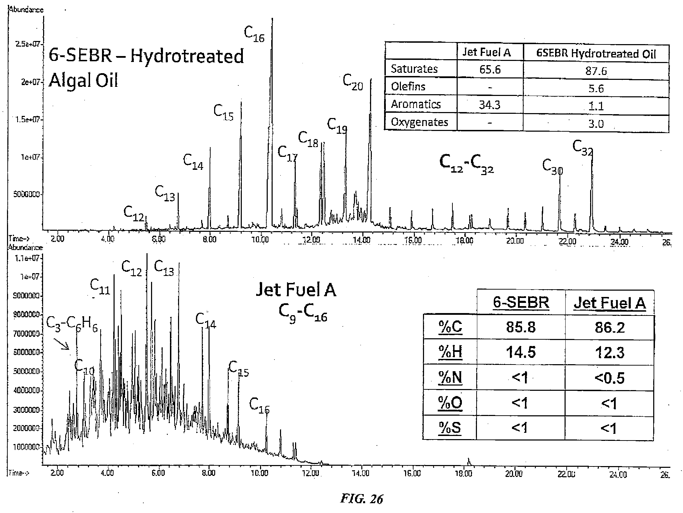

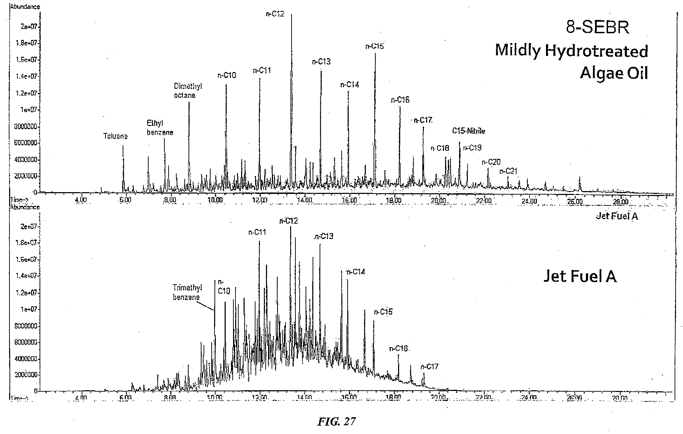

**Figures 26** and **27** illustrate the unusual composition of upgraded algae oil product, compared to a commercial petroleum-derived fuel, jet-A **Figure 26** shows the oil product from processing the algae oil feed in 6SEBR in EXAMPLE I (Ni-Moly catalyst in hydrogen at 1800 psig and 370 degrees C, 1 hour residence time). **Figure 27** shows the oil product from processing of algae oil feed in 8SEBR in EXAMPLE II (Ni/C catalyst in hydrogen at 300 psig at 375 degrees, 1 hour residence time). One may see, in **Figure 26****,** that the upgraded algae oil products, while not at all identical to the jet A in fingerprint and composition, are within a range of composition and characteristics that may allow them, especially with further optimization of operating conditions, to be included or substituted for conventional fuels.

### EXAMPLE VIII

Algae oil feed was obtained from both a *Spirulina* species and a *Desmodesmus* species produced by hydrothermal treatment and hexane or MIBK extraction, according to the methods listed above in the section entitled "Hydrothermal-Treatment of Biomass, with Acidification, for Production of Crude Algae Oil". The hydrothermal treatment step (step b in the method listed above) was conducted at 260 degrees C for 1.0 hour. The acidification step (step d in the method listed above) was conducted at a pH of 5.0. The heating step (step e in the method listed above) was not done. In addition, multiple solvent extractions were conducted (steps f to j were repeated three times).

Algal oil produced as described above was blended with dodecane at a nominal 20% algal oil, 80% dodecane mixture. The algal oil did not completely dissolve in the dodecane solvent. Actual concentrations are as reported in Table **15** as "weight % in dodecane." It should be noted that the solubility of the algal oil in dodecane differed for each algae/solvent combination. Specifically, for algal oil used in Runs 1-3 (described below) the amount of algal oil that dissolved into the dodecane solvent upon preparation of the solution was 90 wt%, 54 wt%, and 26 wt%, respectively.

The solvent can be, but is not limited to naphtha, diesel, kerosene, light gasoil, heavy gasoil, resid, heavy crude, dodecane, a cyclic solvent, an aromatic solvent, a hydrocarbon solvent, crude oil, any product obtained after distillation of crude oil and/or the further refining of crude oil fractions, or any combination thereof.

The resulting algae oil feed was subjected to catalytic-hydrotreatment. The conditions of these experiments are summarized in **Table 15** below. Runs 1-3 were conducted in a continuous-feed reactor. The reactor was operated isothermally and in a downflow/trickle bed mode with oil and hydrogen fed concurrently. The reaction was conducted at temperatures, pressures and space velocities as provided in **Table 15.** The hydrotreatment catalyst was the same catalyst as described in EXAMPLE I. Specifically, it was a commercially-available NiMo/Al₂O₃ that had been pre-sulfided and handled prior to the continuous-feed reaction such that re-oxidation did not occur. The NiMo/Al₂O₃ catalyst was a sample of catalyst used for processing Canadian oil sands, believed to have a pore structure with a BET surface area in the range of 150 - 250 m²/g, micropores in the average diameter range of 50 - 200 Angstroms, and macropores in the range of 1000 - 3000 Angstroms. Products samples were taken after 3, 6, 9 and 12 hours "on oil". Product oil was then analyzed by simulated distillation and HT GC-MS. ASTM standard D4629 was used to analyze nitrogen levels.

**Table 15**

| | **Run No. 1** | **Run No. 2** | **Run No. 3** |
|---|---|---|---|
| **algae type** | *Spirulina* | *Desmodesmus* | *Spirulina* |
| **extraction solvent** | hexane | Hexane | MIBK |
| **s**pace **velocity** | 6 cm³/h, LSHV of 1h⁻¹ | 6 cm³/h, LSHV of 1h⁻¹ | 6 cm³/h,LSHVof 1h⁻¹ |
| **hydrogen feed rate** | 60mL/min | 60mL/min | 60mL/min |
| **Pressure** | 1450 psi | 1450 psi | 1450 psi |
| **Temperature** | 370 degrees C | 370 degrees C | 370 degrees C |
| **weight % in dodecane** | 18.9 | 13.18 | 7.83 |
| **Catalyst** | NiMo/Al₂O₃ pre-sulfides | NiMo/Al₂O₃ pre-sulfided | NiMo/Al₂O₃ pre-sulfided |

Specific details of the catalytic-hydrotreatment protocol are as follows: hydrotreating experiments were performed using 6 cm³ (4.7 g) of the presulfided NiMo/Al₂O₃ catalyst. The liquid feed rate of the algae oil in the dodecane solution was 6 cm³/h. which corresponded to a LHSV of 1 h⁻¹. H₂ feed rate was kept at 60 cm3/min and the pressure of the system was 1,450 psi. Each experiment, all three of which were performed at 370 °C, lasted a total of 12 hours, liquid samples were taken every three hours (at t=3, 6, 9, and 12 h). The catalyst extrudates were used undiluted and their size was approximately 0.85 mm x 1.5 mm x 1.5 mm. The trickle bed tubular (9 mm ID) reactor was down flow for both gas and liquid.

The space velocity can be, but is not limited to, from about 0.1 volume of oil per volume of catalyst per hour to about 10 volume of oil per volume of catalyst per hour; from about 0.1 volume of oil per volume of catalyst per hour to about 6 volume of oil per volume of catalyst per hour; from about 0.2 volume of oil per volume of catalyst per hour to about 5 volume of oil per volume of catalyst per hour; from about 0.6 volume of oil per volume of catalyst per hour to about 3 volume of oil per volume of catalyst per hour; or about 1.6 volume of oil per volume of catalyst per hour. Space velocity and liquid hourly space velocity (LHSV) are used interchangeably throughout the specification.

The hydrogen feed rate can be, but is not limited to, from about 10 m³ H₂/m³ oil to about 1700m³ H₂/m³ oil; from about 100 m³ H₂/m³ dissolved oil to about 1400 m³ H₂/m³ dissolved oil; from about 100 m³ H₂/m³ dissolved oil to about 1000 m³ H₂/m³ dissolved oil; from about 100 m³ H₂/m³ dissolved oil to about 800 m³ H₂/m³ dissolved oil; from about 10 m³ H₂/m³ oil to about 800m³ H₂/m³ oil; from about 100 m³ H₂/m³ oil to about 600m³ H₂/m³ oil; from about 200 m³ H₂/m³ oil to about 500m³ H₂/m³ oil; or about 600 m³ H₂/m³ oil. Hydrogen feed rate can also be measured, for example, as standard cubic feet per barrel.

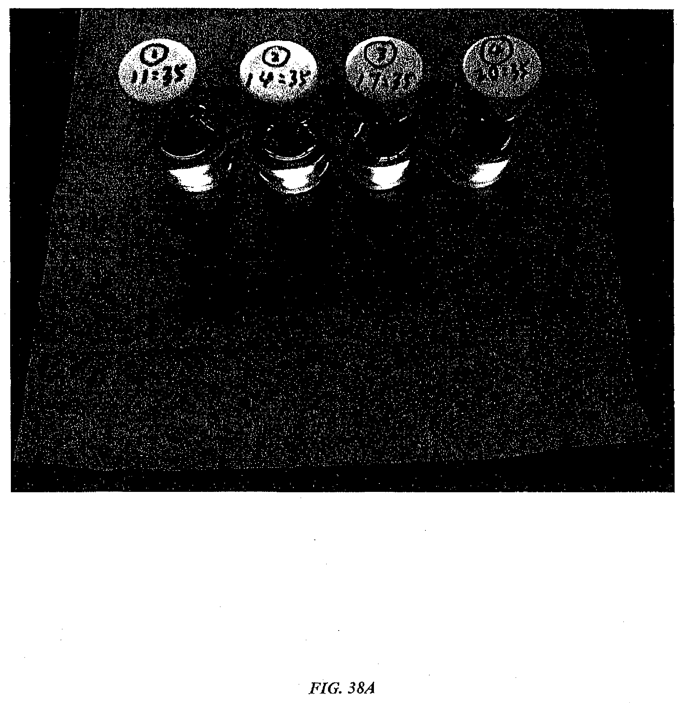

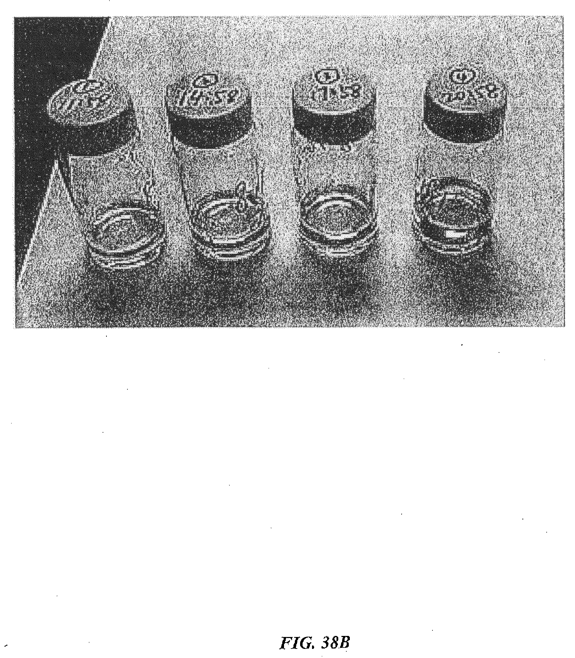

The oil products of these experiments are shown in **Figure 38A** and **Figure 38B****.** **Figure 38A** corresponds to Run No. 1; **Figure 38B** corresponds to Run No. 2. All hydrothermally treated samples (Run 1-3) were dark brown prior to hydrotreating, and clear after hydrotreating, as is shown in **Figure 38A** and Figure **38B**. **Figure 38A** from left to right are samples taken at 3, 6, 9 and 12 hours. **Figure 38B** from left to right are samples taken at 3, 6, 9 and 12 hours. A picture of the Run 3 hydrotreated samples was not taken.

### Boiling Range Distribution

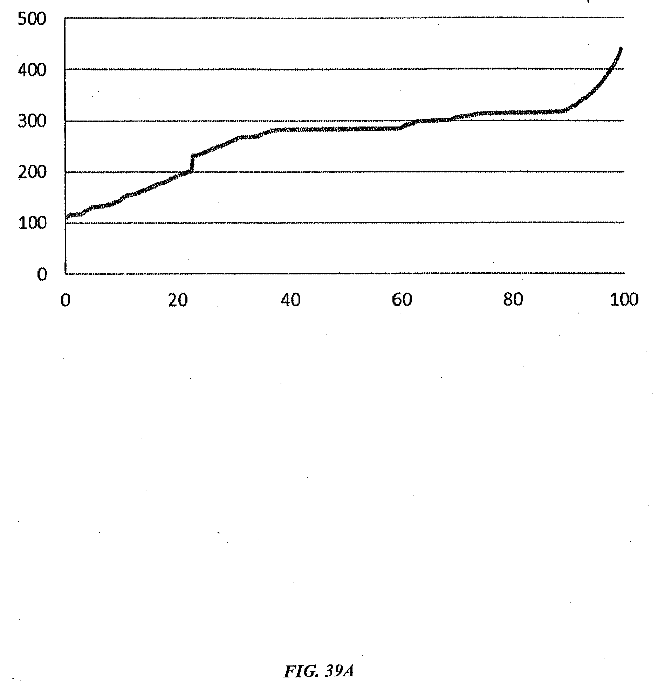

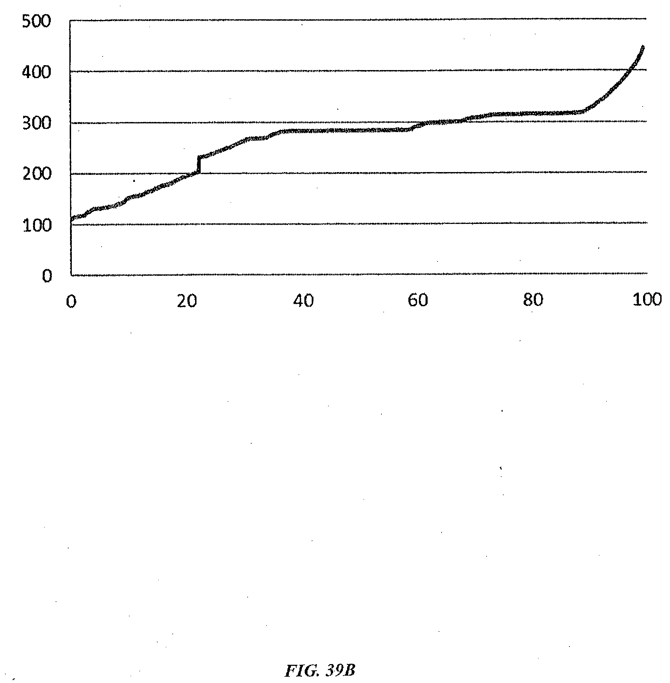

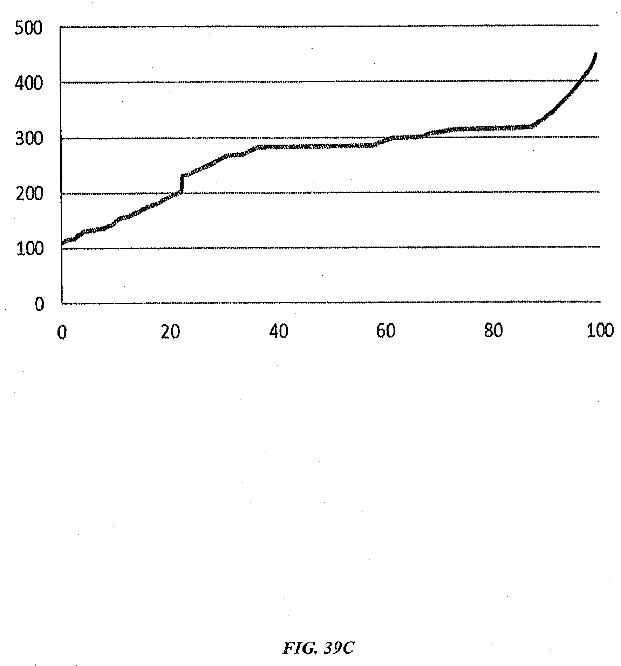

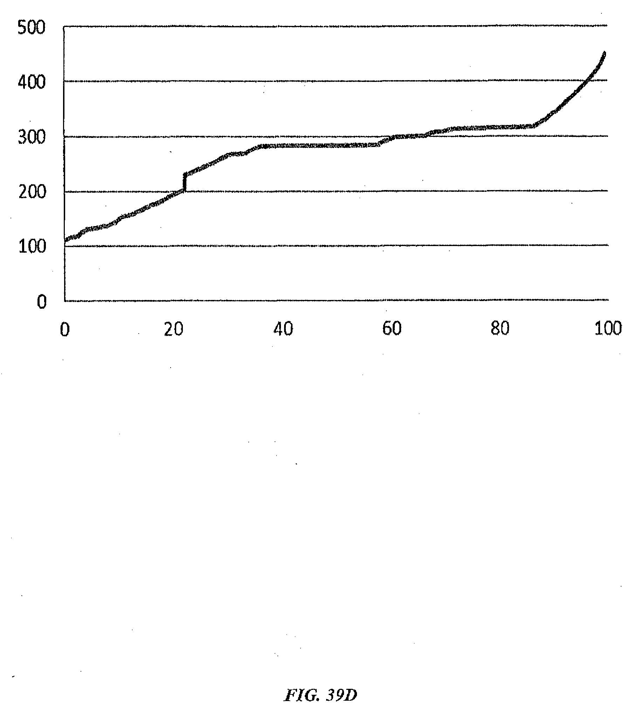

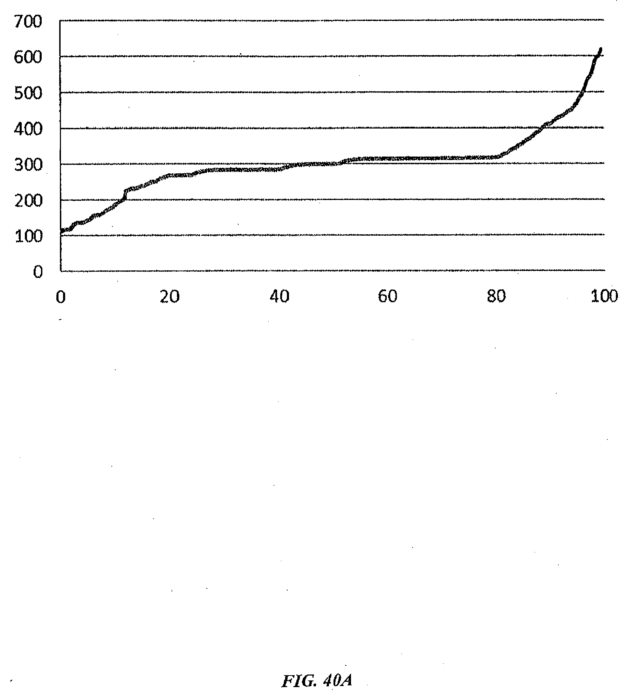

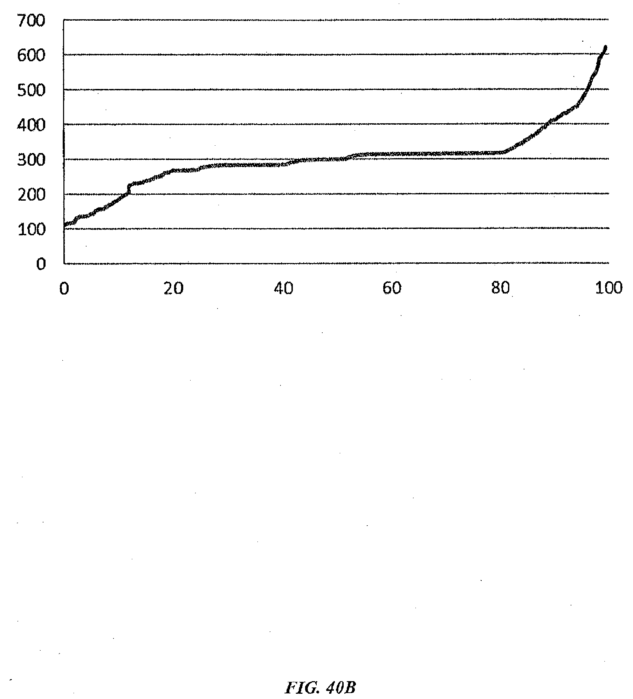

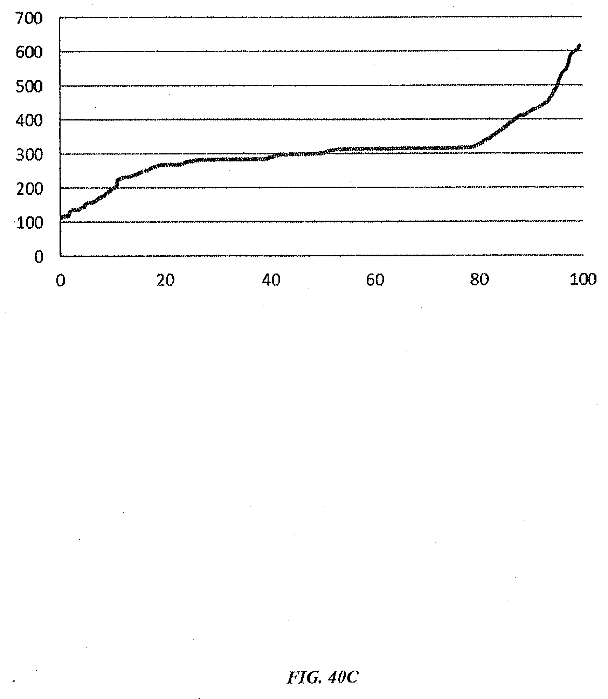

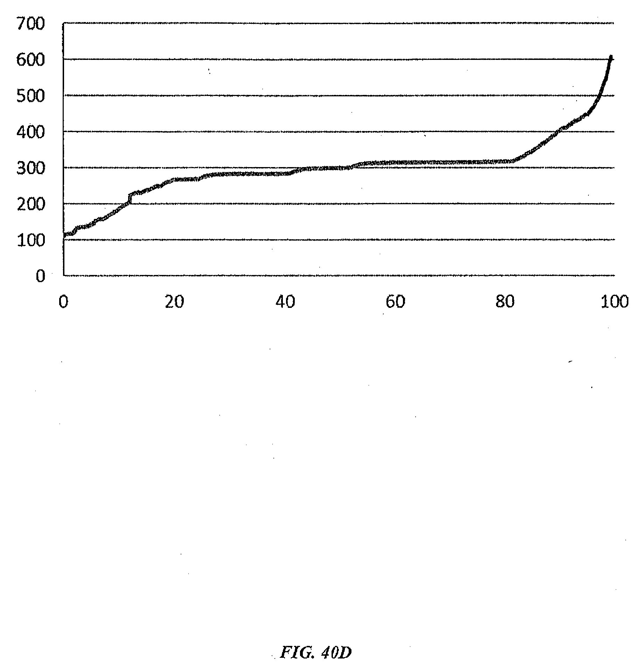

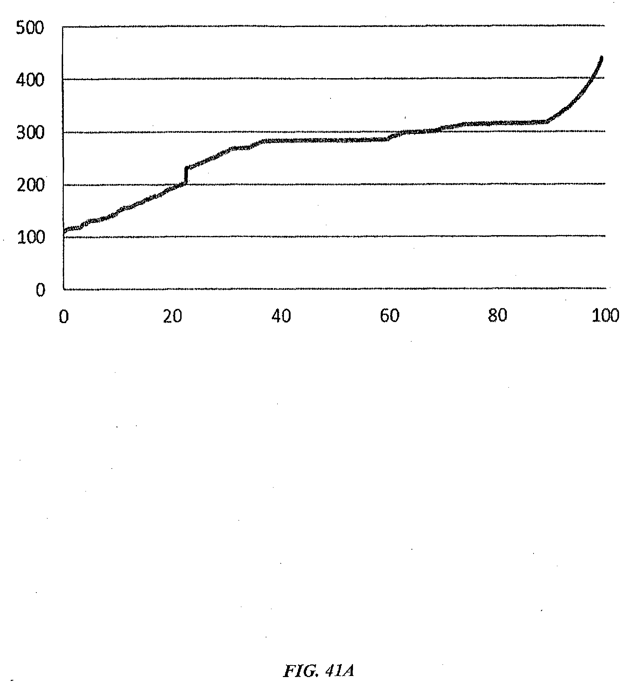

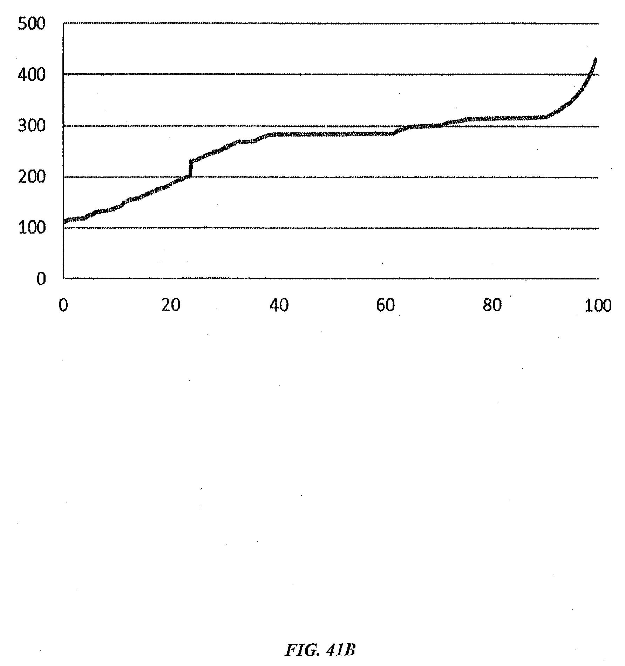

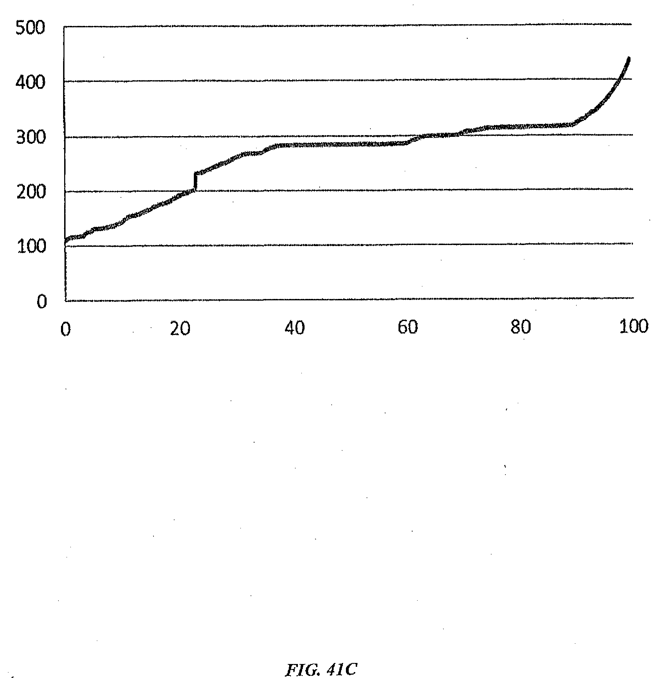

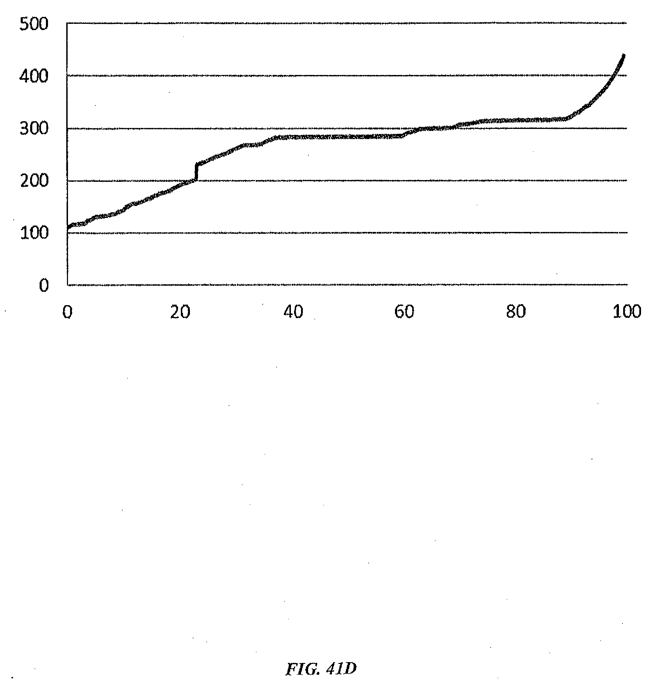

Simulated Distillation (ASTM D7169) was used to characterize both *Desmodesmus* and *Spirulina* upgraded oil products, as detailed in the Analytical Methods section above. Boiling point distribution plots for the oil products of the upgrading processes are shown in **Figures 39A****, B, C** and **D,** **Figures 40A****, B, C** and **D,** and **Figures 41A****, B, C** and **D.** **Figures 39A****, B, C** and **D,** represent boiling point distribution plots for upgraded oil products from *Spirulina,* hexane extracted, with samples taken at 3, 6, 9 and 12 hours respectively (Run 1). **Figures 40A****, B, C** and **D,** represent boiling point distribution plots for upgraded oil products from *Desmodesmus,* hexane extracted, with samples taken at 3, 6, 9 and 12 hours respectively (Run 2). **Figures 41A****, B, C** and **D,** represent boiling point distribution plots for upgraded oil products from *Spirulina,* MIBK extracted; with samples taken at 3, 6, 9 and 12 hours respectively (Run 3). The four distribution plots for each of the upgraded oils were approximately the same over the twelve hour time period. It is notable that all of the algal oil obtained after hydrotreating, as shown in **Figure 39A** to **Figure 41D**, has 90% of its components boiling *below* about 320 degrees C (608 degrees F)(this can be observed by looking at the temperature corresponding to the 90% points). In contrast, the algal oil feed as exemplified in **Figure 2** for *Nannochloropsis salina* has about 90% of its components with a boiling point *above* 320 degrees C (608 degrees F). Therefore, hydrotreating of the algal oils (as described in **Table 15** and directly above), as with EXAMPLE I, produces a significant reduction in boiling point.

### Compound Groups by HT GC-MS and Elemental Analysis

High Temperature GC-MS (HT GC-MS) was used to identify compounds in the upgraded oil products and to measure the effects of the upgrading methods and process conditions on oil quality and composition. HT GC-MS also provided information at the molecular level. EA (as described in the Analytical Methods section above) was used to determine nitrogen levels, as is shown in **Table 17** discussed below.

The modified HT GC-MS equipment and methods used for the Examples are detailed earlier in this document and in Provisional Patent Application 61/547,391 (incorporated herein), with a summary being: Column - Zebron ZB-1HT Inferno™, Dimensions - 15 meter x 0.25 mm x 0.1 µm; Injection - PTV, GERSTEL CIS4 @ 10 °C, 12°/sec to 380 °C, 0.1 µL, Split 1:10; Carrier Gas - Helium @ 1.5 mL/min (constant flow); Oven Program: 40 °C for 1 min to 380 °C @ 20 °C/min, 10 min hold; Detector - MSD, Interface @ 300 °C, Source @ 230 °C**,** Quad @ 150 °C; Sample: 2% in CS2. Approximately 200 peaks per sample were detected. Roughly 50% of the peaks accounting for 75% to 90% of the total peak area were identified, with a minimum match quality requirement: ≥80%. The HT GC-MS chromatograms were integrated and peak spectra (TIC) compared against the NIST08 and Wiley 9 library. Identified peaks were sorted according to the number of carbons.

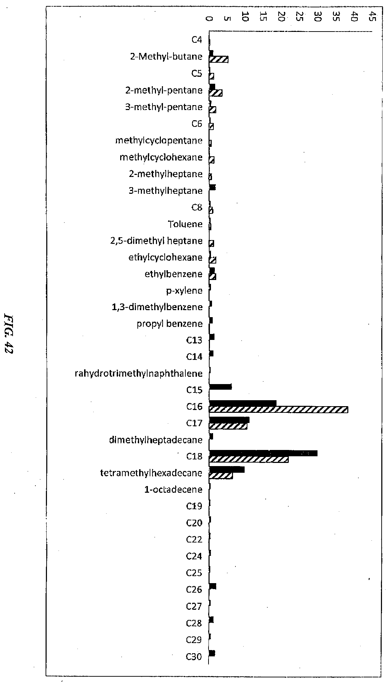

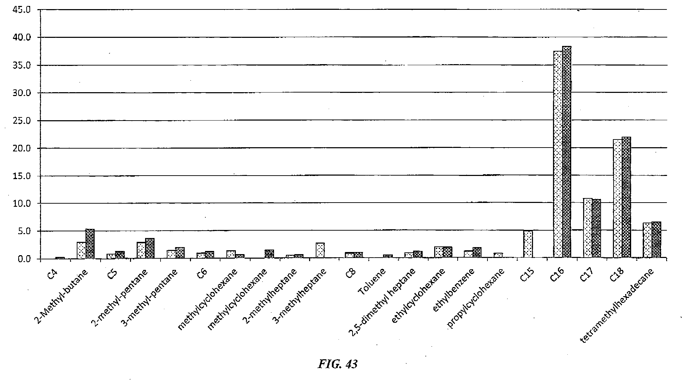

**Figure 42** is a graphical representation of data from a HT GG-MS analysis of Run 1 and Run 2. The first bar of each set of two bars is from Run 2, the second bar of each set of two bars is from Run 1. The y-axis is normalized area percent and the x-axis lists various compound types. HT GC-MS results show significant differences between *Desmodesmus* and *Spirulina* hydrotreated oil, both extracted with hexane. As is shown in **Figure 42****,** upgraded *Spirulina* oil comprises a higher percentage of low carbon number containing compounds than upgraded *Desmodesmus* oil. Additionally, upgraded *Spirulina* oil comprises a higher percentage of C16 containing compounds, and upgraded *Desmodesmus* oil comprises a higher percentage of C18 containing compounds. These are just a few examples of some of the differences between *Desmodesmus* and *Spirulina* hydrotreated oil, both extracted with hexane. As is apparent in **Figure 42****,** the two hydrotreated oil products are very different in composition from each other. These compositional differences are significant since the two algae strains were processed in the same manner, specifically, the same hydrothermal treatment, the same solvent extraction, and the same hydrotreating conditions. **Figure 43** is a graphical representation of data from a HT GC-MS analysis of Run 1 and Run 3. The first bar of each set of two bars is from Run 1, the second bar of each set of two bars is from Run 3. The y-axis is normalized area percent and the x-axis lists various compound types. HT GC-MS results show no significant differences between *Spirulina* hydrotreated oil extracted with hexane and with MIBK. However, it should be noted that a significant amount of the algal oil from the MIBK extraction was not dissolved in dodecane. **Table 16** below contains the data of **Figure 42** and **Figure 43****.** Notable are the percent C16 (*see* *) and the percent C18 *(see* **) values. Also notable is the presence of longer chain compounds (C19-C30) only in the *Desmodesmus* strain.

**Table 16**

| **compound type** | **normalized area % *Spirulina* hexane extracted -Run 1** | **normalized area %** *Desmodesmus* **hexane extracted -Run 2** | **normalized area % *Spirulina* MIBK extracted - Run 3** |
|---|---|---|---|
| **C4** | 0.2 | | |
| **2-methyl-butane** | 5.3 | 1.1 | 3.0 |
| **C5** | 1.3 | 0.3 | 0.8 |
| **2-methyl-pentane** | 3.6 | 1.7 | 2.9 |
| **3-methyl-pentane** | 1.9 | 0.6 | 1.4 |
| **C6** | 1.2 | 0.4 | 0.9 |
| **Methylcyclopentane** | 0.6 | | |
| **Methylcyclohexane** | 1.4 | | 1.3 |
| **2-methyl-heptane** | 0.6 | | 0.5 |
| **3-methyl-heptane** | | 1.7 | 2.7 |
| **C8** | 1 | 0.4 | 0.9 |
| **Toluene** | 0.5 | 0.4 | |
| **2,5-dimethylheptane** | 1.2 | | 0.9 |
| **Ethylcyclohexane** | 1.9 | 0.4 | 2.0 |
| **Ethylbenzene** | 1.8 | 1.6 | 1.2 |
| **p-xylene** | | 0.4 | |
| **1,3-dimethylbenzene** | | 0.7 | |
| **Propylbenzene** | | 0.9 | |
| **Propylcyclohexane** | | | 0.8 |
| **C13** | | 1.4 | |
| **C14** | | 1.2 | |
| **tetrahydrotrimethylnapthalene** | | 0.4 | |
| **C15** | | 6.3 | 4.9 |
| **C16** | 38.3* | 18.7* | 37.4* |
| **C17** | 10.6 | 11.2 | 10.7 |
| **Dimethylheptadecane** | | 1.1 | |
| **C18** | 22** | 30.1** | 21.5** |
| **tetramethylhexadecane** | 6.5 | 9.9 | 6.3 |
| **1-octadecane** | | 0.4 | |
| **C19** | | 0.3 | |
| **C20** | | 0.6 | |
| **C22** | | 0.4 | |
| **C24** | | 0.5 | |
| **C25** | | 0.4 | |
| **C26** | | 2.1 | |
| **C27** | | 0.6 | |
| **C28** | | 1.4 | |
| **C29** | | 0.6 | |
| **C30** | | 1.7 | |

Nitrogen levels prior to solvent dilution and hydrotreatment can be, for example, in the range of 10,000 to 100,000 ppm, 19,000 to 64,000 ppm, or 20,000 to 80,000 ppm. Run 1 had 6.4 weight percent (standard deviation of 0.15) nitrogen; Run 2 had 1.9 weight percent (standard deviation of 0.1) nitrogen; and Run 3 had 3.7 weight percent (standard deviation of 1.3) nitrogen. Prior to hydrotreating, the algal oil can have, for example, up to 8 weight % nitrogen, and after hydrotreating the algal oil can have, for example, less than 1 weight % nitrogen. Alternatively, prior to hydrotreating, the algal oil can have, for example, up to 7 weight %, up to 6 weight %, up to 5 weight %, up to 4 weight %, up to 3 weight %, up to 2 weight %, or up to 1 weight % nitrogen, and after hydrotreating the algal oil can have, for example, less than 0.9 weight %, less than 0.8 weight %, less than 0.7 weight %, less than 0.6 weight %, less than 0.5 weight %, less than 0.4 weight %, less than 0.3 weight %, less than 0.2 weight %, or less than 0.1 weight % nitrogen.

Nitrogen was reduced after hydrotreating for all three runs as is shown below in **Table 17.** Nitrogen levels were decreased at the 3 hour time point and remained low for each of the samples taken at 6,9 and 12 hours. Notable here is that the denitrogenation of Runs 1-3 was in excess of 99%. Considering the elevated nitrogen levels in the algal oil, prior to hydrotreating, this indicates that the nitrogen containing species are readily converted under the hydrotreating conditions described in **Table 15,** and alternatively the conditions provided throughout the disclosure. Both the hydrotreating conditions disclosed in **Table 15** and throughout the disclosure can be found in conventional petroleum refineries.

**Table 17**

| | ppm of nitrogen after HTT and solvent extraction | ppm of nitrogen before hydrotreating but after solvent dilution | ppm of nitrogen after hydrotreating |
|---|---|---|---|
| **Run 1** | 64,000 | 8800 | 15 |
| **Run 2** | 19,000 | 4000 | 29 |
| **Run 3** | 37,000 | 3500 | 11 |

### EXAMPLE IX

Algae oil feed was obtained from a *Spirulina* species produced by hydrothermal treatment and hexane extraction, according to the methods listed above in the section entitled "Hydrothermal-Treatment of Biomass, with Acidification, for Production of Crude Algae Oil". The hydrothermal treatment step (step b in the method listed above) was conducted at 260 degrees C for 1.0 hour. The acidification step (step d in the method listed above) was conducted at a pH of 4.5 to 5.0. The heating step (step e in the method listed above) was not done. In addition, multiple solvent extractions were conducted (steps f to j were repeated twice).

A fraction of the resulting oil feed was then subjected to an additional thermal treatment. Thermal treatment was at 400 degrees C for 30 minutes (as is described in United States Provisional Application Number 61/504,134, filed July 1,2011, entitled THERMAL TREATMENT OF CRUDE ALGAE OIL AND OTHER RENEWABLE OILS FOR IMPROVED OIL QUALITY, and United States Provisional Application Number 61/552,628, filed October 28,2011, entitled THERMAL TREATMENT OF ALGAE OIL).

As indicated in United States Provisional Application Number 61/504,134 and United States Provisional Application Number 61/552,628, thermal treatment of algal oil has a number of benefits including lowering the viscosity of the oil, reducing its sulfur, oxygen and metals content and shifting the boiling point distribution of its components to lighter (lower boiling materials). This thermal treatment can be done in a standalone processing unit dedicated to algal oil or it can be done in a conventional refinery where algal oil can be co-processed in coking units with conventional feedstocks derived from crude in coking units or in cokers dedicated to algal oil. The thermal treatment can also be conducted in the heat exchanger trains that precede many processing units where algal oil could be processed unblended or in mixtures with conventional oils derived from crude.

The resulting algae oil both thermally treated oil and oil that which was not thermally treated, was further subjected to catalytic-hydrotreatment. The conditions of these experiments are summarized in **Table 18** below. Runs 4 and 5 were conducted in semi-batch reactor mode with hydrogen continuously fed to the reactor that had been previously charged with catalyst and oil as described in EXAMPLE 1. The hydrotreatment catalyst was the same catalyst as described in EXAMPLES I. Specifically, it was a commercially-available NiMo/Al₂O₃ that had been pre-sulfided and handled prior to the batch-feed reaction such that re-oxidation did not occur. The NiMo/Al₂O₃ catalyst was a sample of catalyst used for processing Canadian oil sands, believed to have a pore structure with a BET surface area in the range of 150 - 250 m²/g, micropores in the average diameter range of 50 - 200 Angstroms, and macropores in the range of 1000 - 3000 Angstroms.

**Table 18**

| | **Run No. 4** | **Run No. 5** |
|---|---|---|
| **algae type** | *Spirulina* | *Spirulina* |
| **thermally treated** | No | Yes |
| **extraction solvent** | Heptane | Heptane |
| **Pressure** | 1800 psi H₂ | 1800 psi H₂ |
| **Temperature** | 370 degrees C | 370 degrees C |
| **Catalyst** | NiMo/Al₂O₃ pre-sulfided | NiMo/Al₂O₃ pre-sulfided |

Specific details of the catalytic-hydrotreatment protocol are as follows: 15 g algae oil were weighed directly into the reactor; the reactor was purged with Ar; 7.5 g NiMo/Al₂O₃ pre-sulfided catalyst was added to the reactor (the catalyst was pre-weighed in a glove bag under inert atmosphere and stored in a desiccator to limit the exposure to air) and the reactor was sealed; stirring began at about 100 rpm; the reactor was purged three times with Ar and pressurized to 1000 psi with H₂; the stirring speed was increased to about 600 rpm and the temperature was ramped to 370 degrees C; the pressure was then adjusted to 1800 psi with H₂ and the reactor held at 370 degrees C for one hour; the reactor was then cooled to 35 degrees C; the gas sample was collected; and the solid and liquid products were recovered. The recovered oil and residual solids left on the catalyst was in excess of 90% with the remainder of the mass lost to hydrogen that was flowing through the reactor. The oil constituted at least 85% of the recovered oil and residual solids with the latter being material that adhered to the catalyst particles or reactor walls.

The recovered solid and liquid products were then analyzed as follows: the total product which consisted of the oil in the reactor along with the catalyst, was weighed; the total product was then gravity filtered in order to separate out the *light fraction* from the solids (oil that could not be poured out and that still adhered to the catalyst at room temperature); the solids were then extracted with chloroform; chloroform was removed from the liquid by rotary evaporation at 60 degrees C under vacuum; and the *heavy fraction* was obtained. The *light fraction* is what can be poured out of the reactor and filtered. The *heavy fraction* is extracted with chloroform from the solids. The solids comprise oil adhered to the catalyst and the catalyst itself. The heavy and light fractions were then analyzed by simulated distillation and HT GC-MS.

### Boiling Range Distribution

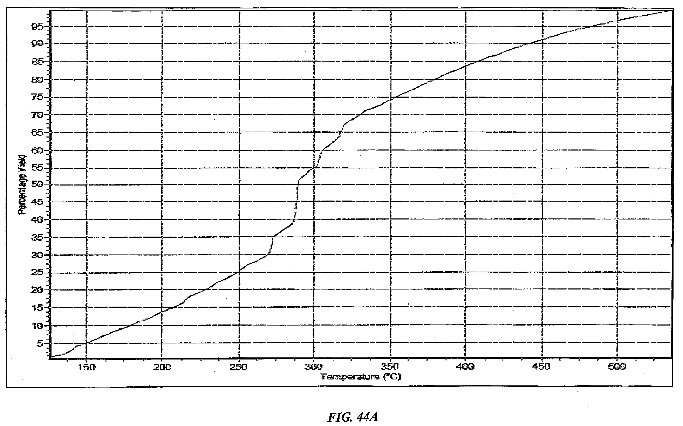

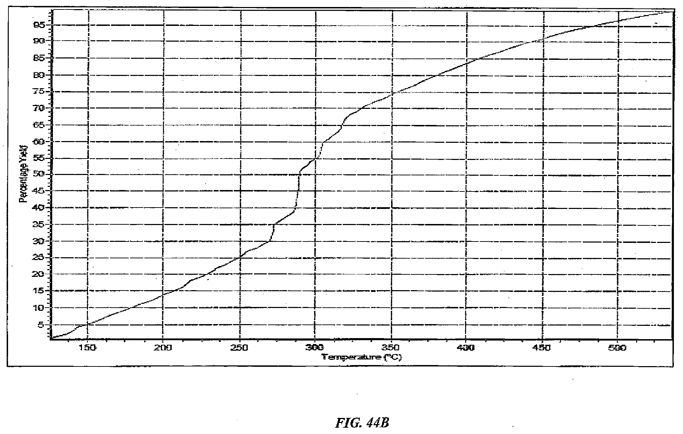

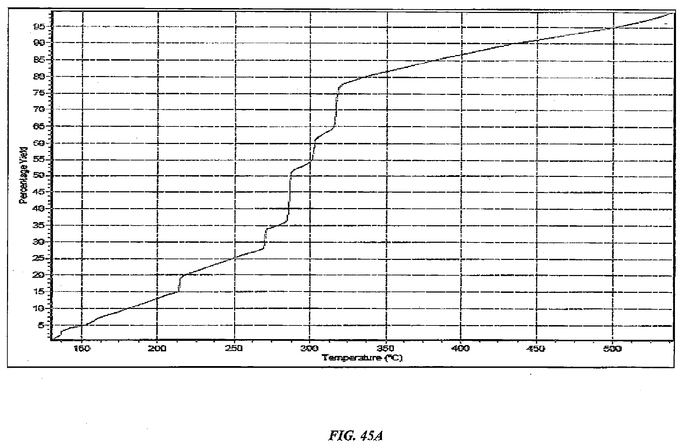

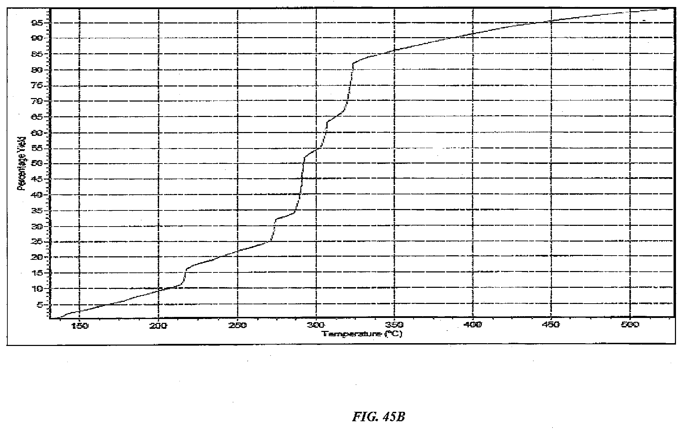

Simulated Distillation (ASTM D7169) was used to characterize both the *light* and *heavy fractions* of the *Spirulina* upgraded oil products from Run 4 and Run 5, as detailed in the Analytical Methods section above. Boiling point distribution plots for the oil products of the upgrading processes are shown in **Figures 44A** and **Figure 44B** (Run 4; light and heavy fractions respectively) and **Figures 45A** and **Figure 45B** (Run 5; light and heavy fractions respectively)).

Notable is that there was little difference in the boiling point distributions when comparing the light fraction to the heavy fraction for either the hydrotreated products that were first thermally treated or for the hydrotreated products that were not first thermally treated. These results are not unexpected since, as described above, the light and heavy designations came from how the products were recovered from the reactor (by chloroform extraction) and the method of chloroform extraction does not necessarily distinguish materials by their boiling points.

It is also notable that the hydrotreated products (both the light and heavy fractions) that were first thermally treated have a lower average boiling point than those which were not first thermally treated. The temperature at which 90% of the material has boiled overhead for the hydrotreated products that were first thermally treated was about 400 degrees C (*see* **Figure 45A** and **Figure 45B**). The temperature at which 90% of the material has boiled overhead for the hydrotreated products that were not first thermally treated was about 450 degrees C (*see* **Figure 44A** and **Figure 44B**).

### Compound Groups by HT GC-MS

High Temperature GC-MS (HT GC-MS) was used to identify compounds in the upgraded oil products. The modified HT GC-MS equipment and methods used for the Examples are detailed earlier in this document and in Provisional Patent Application 61/547,391 (incorporated herein), with a summary being: Column - Zebron ZB-1HT Inferno™, Dimensions - 15 meter x 0.25 mm x 0.1 µm; Injection - PTV, GERSTEL CIS4 @ 10 °C, 12°/sec to 380 °C, 0.1 µL, Split 1:10; Carrier Gas - Helium @ 1.5 mL/min (constant flow); Oven Program: 40 °C for 1 min to 380 °C @ 20 °C/min, 10 min hold; Detector - MSD, Interface @ 300 °C, Source @ 230 °C, Quad @ 150 °C; Sample: 2% in CS2. Approximately 200 peaks per sample were detected. Roughly 50% of the peaks accounting for 75% to 90% of the total peak area were identified, with a minimum match quality requirement: ≥80%. The HT GC-MS chromatograms were integrated and peak spectra (TIC) compared against the NIST08 and Wiley 9 library. Identified peaks were sorted according to the number of carbons and by compound class.

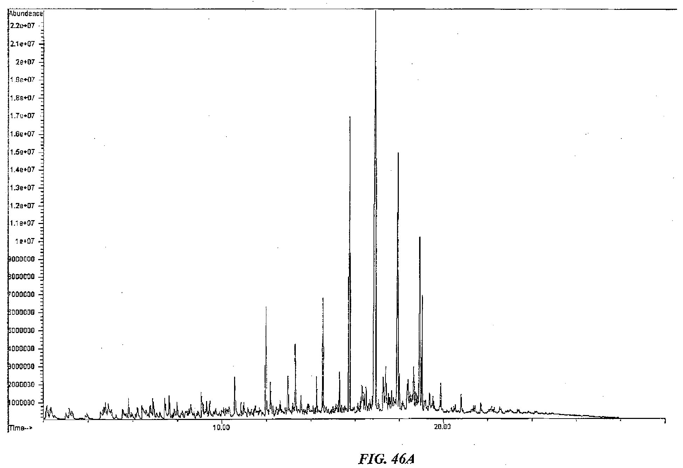

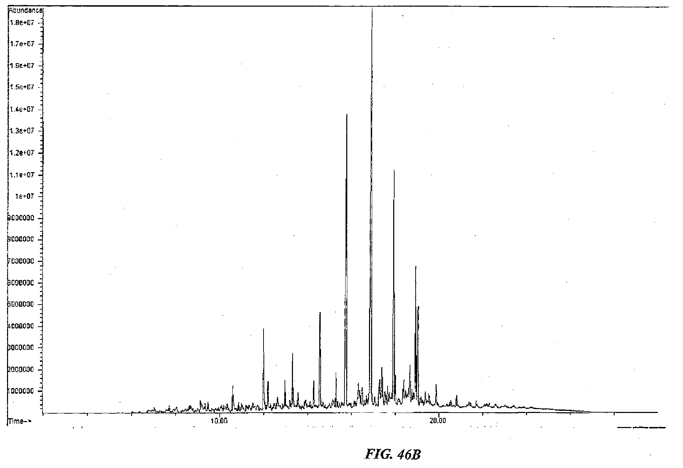

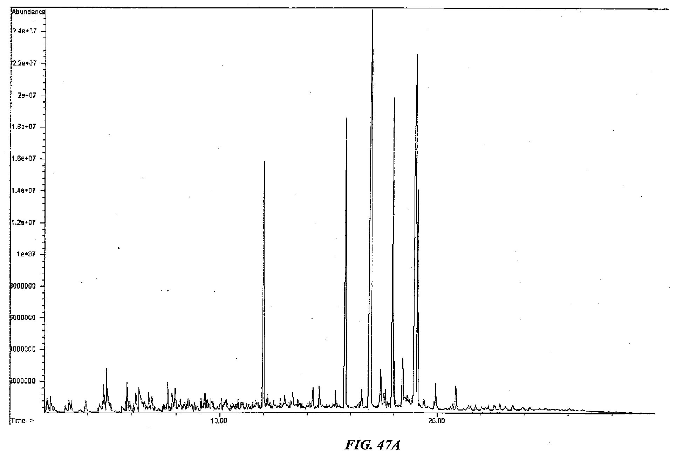

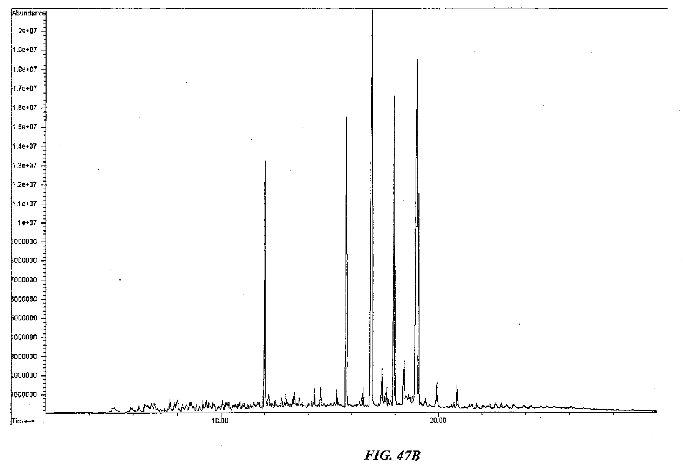

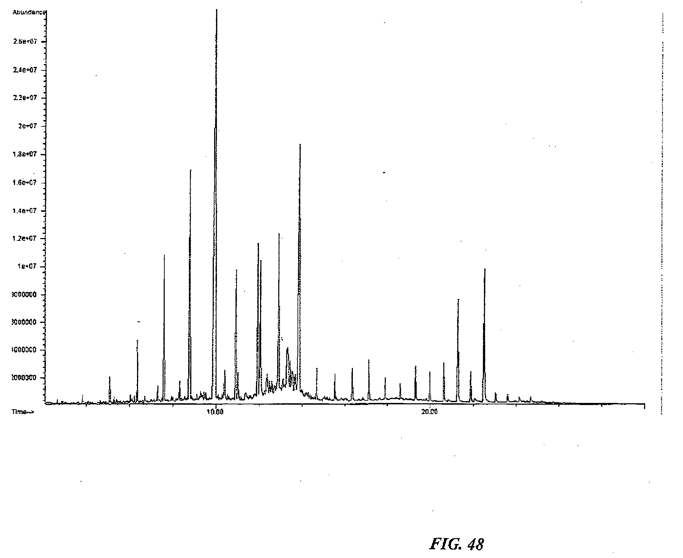

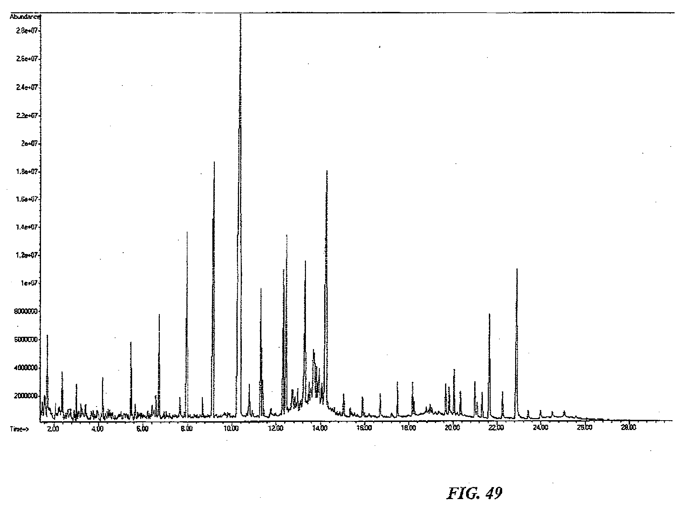

**Figure 46A** and **Figure 46B** show a chromatogram of the light and heavy fractions of Run 4 (no thermal treatment prior to hydrotreatment). **Figure 47A** and **Figure 47B** shows a chromatogram of the light and heavy fractions of Run 5 (thermal treatment prior to hydrotreatment). **Figure 48** shows a chromatogram of the *Nannochloropsis* upgraded oil 7SEBR described above (in EXAMPLE I) for comparison with the light and heavy fractions of Run 4 and Run 5. **Figure 49** shows a chromatogram of the *Nannochloropsis* upgraded oil 10SEBR described above (in EXAMPLE II) for comparison with the light and heavy fractions of Run 4 and Run 5. It should be noted that the oil products shown in **Figure 48****,** **Figure 49****,** and **Figures 46A** and **B****,** were not thermally treated prior to hydrotreating, and the oil products shown in **Figures 47A** and **B** were thermally treated prior to hydrotreatment.

As is shown in **Figures 46A** and **B****,** and **Figures 47A** and **B****,** the distribution of hydrocarbons in oil derived from *Spirulina* (from both Run 4 and Run 5, and from both heavy and light fractions) has fewer compounds, and the compounds are mainly concentrated in the C12-C18 range. This means that fuels derived from *Spirulina* will have yields that are higher in jet materials than from a *Nannochloropsis* species. By contrast, *Nannochloropsis* derived oils will have a wider range of hydrocarbons, for example, from C9-C32 (as is shown in **Figure 48**). This means that fuel derived from *Nannochloropsis* will have yields that are different, ranging from naphtha to gas oil range materials (terms commonly used in refining technology). Similarly the fuel quality of oil derived from *Spirulina* will be different from that of *Nannochloropsis.* These qualities include, but are not limited to, cold flow properties (pour point, cloud point, and freeze point). Cold flow properties are relevant in cold weather operations. An example of the differing qualities is that *Nannochloropsis* derived oil was a solid at room temperature whereas *Spirulina* derived oil was a liquid at room temperature.

**Table 19A** below provides the weight percent of each of C12-C18 wherein the weight percent is of the total amount of compounds detectable by mass spectrometry analysis. This data is shown in **Figure 46A****,** **Figure 46B****,** **Figure 47A****,** and **Figure 47B****.**

**Table 19A**

| | **13H** | | **13L** | | **14H** | | **14L** | |
|---|---|---|---|---|---|---|---|---|
| **Identity** | **Retention Time** | **%** | **Retention Time** | **%** | **Retention Time** | **%** | **Retention Time** | **%** |
| **C12** | 11.9746 | 2.2 | 11.9746 | 2.3 | 12.0009 | 6.6 | 12.0007 | 5.9 |
| **C14** | 14.5437 | 2.5 | 14.5437 | 2.4 | 14.5525 | 0.5 | 14.5523 | 0.5 |
| **C15** | 15.7541 | 9.2 | 15.7629 | 8.7 | 15.763 | 9.7 | 15.7715 | 8.8 |
| **C16** | 16.9124 | 19.5 | 16.9211 | 18.2 | 16.9387 | 20.9 | 16.9472 | 19.4 |
| **C17** | 17.9226 | 7.6 | 17.9313 | 7.1 | 17.9576 | 11.4 | 17.9661 | 10.5 |
| **C18** | 18.9241 | 4 | 18.9241 | 3.9 | 19.0026 | 16.6 | 19.0111 | 15.2 |
| **Total** | | 45 | | 42.7 | | 65.7 | | 60.2 |

**Table 19B** below provides the weight percent of each of C9-C32 wherein the weight percent is of the total amount of compounds detectable by mass spectrometry analysis. This data is shown in **Figure 48** and **Figure 49****.**

**Table 19B**

| | **7SEBR** | | **10SEBR** | |
|---|---|---|---|---|
| | **Retention Time** | **Percent** | **Retention Time** | **Percent** |
| **C9** | | | 2.0747 | 0.2298 |
| **C10** | 2.6268 | 0.5356 | 3.0152 | 0.3873 |
| **C11** | 3.8112 | 0.6199 | 4.1909 | 0.4723 |
| **C12** | 5.0913 | 0.8708 | 5.4623 | 0.9078 |
| **C13** | 6.3628 | 1.2372 | 6.7338 | 1.3064 |
| **C14** | 7.5994 | 2.2141 | 7.9791 | 3.3125 |
| **C15** | 8.7925 | 4.3198 | 7.9791 | 3.3125 |
| **C16** | 9.9159 | 6.4955 | 10.374 | 17.8651 |
| **C17** | 10.9261 | 2.2844 | 11.3059 | 2.004 |
| **C18** | | | 12.3248 | 2.6294 |
| **C19** | | | 13.2914 | 3.8137 |
| **C20** | | | 14.2581 | 7.0972 |
| **C27** | 19.478 | 1.0101 | | |
| **C29** | 21.2981 | 1.6185 | | |
| **C30** | | | 21.6604 | 1.9208 |
| **C31** | 22.5347 | 2.697 | | |
| **C32** | | | 22.8883 | 3.273 |
| | | 23.9029 | | 45.2588 |

One observations when comparing hydrotreated algal oil that was thermally pretreated with hydrotreated algal oil that was not thermally pretreated is that both oils are upgraded in a similar manner upon hydrotreatment. This means that the decision to thermally pre-treat or not will be driven by cost economics, refinery configurations, etc., since both are technically feasible. Shown below in **Table 19C** are the compound classes as determined by HT GC-MS of hydro-treated products from thermally versus non-thermally treated algae crude feed.

**Table 19C**

| **hydro-treated** | **hydro-treated products of thermally treated oil -Run 5** | | **hydro-treated products of non-thermally treated oil -Run 4** | |
|---|---|---|---|---|
| **Fraction** | **Heavy** | **light** | **heavy** | **light** |
| **Aromatics** | 1.8 | 2.3 | 0.7 | 1.5 |
| **Amides** | 0.0 | 0.0 | 0.0 | 0.0 |
| **Nitrogen compounds** | 3.0 | 0.4 | 5.4 | 0.1 |
| **Fatty Acids** | 0.0 | 0.0 | 0.0 | 0.0 |
| **Hydrocarbon-saturated** | 49.6 | 61.7 | 43.0 | 74.0 |
| **Hydrocarbon-unsaturated** | 0.0 | 0.6 | 0.7 | 0.5 |
| **Nitriles** | 0.1 | 0.0 | 0.6 | 0.0 |
| **Oxygen compounds** | 1.4 | 2.1 | 2.3 | 2.7 |
| **Phosphorus compounds** | 0.0 | 0.0 | 0.0 | 0.0 |
| **Sterols** | 0.0 | 0.0 | 0.0 | 0.0 |
| **Sulfur compounds** | 0.0 | 0.0 | 0.0 | 0.0 |

In this disclosure, ranges of temperature, holding time/residence time/LHSV, gas to oil ratios, BET surface in m2/g, pore sizes in Angstroms, pressure in psig, and/or other ranges of variables, are given for many embodiments of the invention. It should be understood that the ranges are intended to include all sub-ranges, and to include each incremental amount of temperature, holding time/residence time/LHSV, gas to oil ratios, BET surface in m2/g, pore sizes in Angstroms, pressure in psig, and other variable, within each broad range given. For example, while a broad range of pressure of 1000 - 2000 psig is mentioned, certain embodiments may include any of the following sub-ranges or any pressure within any of the following sub-ranges: 1000 - 1050, 1050 - 1100, 1100 - 1150, 1150 - 1200, 1200 - 1250, 1250 - 1300, 1300 - 1350, 1350 - 1400, 1400 - 1450, 1450 - 1500, 1500 - 1550, 1550 - 1600, 1600 - 1650, 1650 - 1700, 1700 - 1750, 1750 - 1800, 1800 - 1850, 1850 - 1900, 1900 - 1950, and 1950 - 2000 psig. For example, while a broad range of 300 -- 500 degrees C is mentioned, certain embodiments may include any of the following sub-ranges or any temperature within any of the following sub-ranges: 300 - 310, 310 - 320, 320 - 330, 330 - 340, 340 - 350, 350 - 360, 360- 370, 370 - 380, 380 - 390, 390 - 400, 400 - 410,410 - 420, 420 - 430, 430 - 440, 440 - 450, 450 - 460, 460 - 470, 470 - 480, 480- 490, and 490 - 500 degrees C. For example, while a broad range of 300 - 600 degrees C maximum temperature is mentioned (for thermal treating, for example), certain embodiments may include any of the following sub-ranges or any temperature within any of the following sub-ranges: 300 - 310, 310- 320, 320- 330, 330-340, 340 - 350, 350 - 360, 360 - 370, 370 - 380, 380 - 390,390 - 400, 400 - 410, 410 - 420, 420 - 430, 430 - 440, 440 - 450, 450 - 460, 460 - 470, 470 - 480, 480-90, 490 - 500, 500 - 510, 510 - 520, 520 - 530, 530 - 540, 540 - 550, 550 - 560, 560 - 570, 570 - 580, 580 - 590, and/or 590 - 600 degrees C.

Also included this disclosure, wherein values, for example, such as area percent, mass percent, or weight percent are written or shown in Tables or Figures, are those values but with "about" inserted before each value, as one of average skill in the art will understand that "about" these values may be appropriate in certain embodiments of this disclosure.

While certain embodiments have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the disclosure.

## Claims

1. A method of upgrading algae oil obtained from biomass comprising algae, the method comprising:
a) providing the algae oil;
b) dissolving at least a portion of the algae oil in a solvent;
c) upgrading the algae oil in the solvent by a method comprising: hydrotreating the algae oil in the solvent in the presence of a catalyst, at a temperature of from about 300 degrees C to about 500 degrees C; a total pressure and/or hydrogen partial pressure of from about 800 psi to about 3000 psi; a space velocity from about 0.1 volume of oil per volume of catalyst per hour to about 10 volume of oil per volume of catalyst per hour; and a hydrogen feed rate of from about 10 m³ H₂/m³ dissolved oil to about 1700 m³ H₂/m³ dissolved oil; wherein said catalyst is a large-pore catalyst selected from the group consisting of petroleum residuum and petroleum bitumen hydrotreating catalysts, to obtain a hydrotreating effluent.

2. The method of claim 1, wherein prior to step a), step b), and step c) the algae oil was not refined-bleached-deodorized (RBD).

3. The method of claim 1, wherein the method further comprises, separating a hydrotreated oil from the hydrotreating effluent; and further upgrading the hydrotreated oil by sending the hydrotreated oil or a fraction thereof to one or more of an FCC unit, a hydrocracking unit, a hydroisomerization unit, a dewaxing unit, a naphtha reformer, or a unit utilizing Ni/MO, Co/MO, Ni/W, a precious metal, a noble metal, a group VIII catalyst, or a zeolitic catalyst.

4. The method of claim 1, wherein the solvent is naphtha, diesel, kerosene, light gasoil, heavy gasoil, resid, heavy crude, dodecane, a cyclic solvent, an aromatic solvent, a hydrocarbon solvent, crude oil, any product obtained after distillation of crude oil and/or the further refining of crude oil fractions, or any combination thereof.

5. The method of Claim 4, wherein the solvent is heavy gasoil, resid, heavy crude, crude oil or any combination thereof.

6. The method of claim 1, wherein the total pressure and/or hydrogen partial pressure is from about 1000 psi to about 2000 psi, about 1500 psi to about 2000 psi; or selected from the group consisting of: 1000 psi to 1100 psi, 1100 psi to 1200 psi, 1200 psi to 1300 psi, 1300 psi to 1400 psi, 1400 psi to 1500 psi, 1500 psi to 1600 psi, 1600 psi to 1700 psi, 1700 psi to 1800 psi, 1800 psi to 1900 psi, 1900 psi to 2000 psi, 2000 psi to 2100 psi, 2100 psi to 2200 psi, 2200 psi to 2300 psi, 2300 psi to 2400 psi, 2400 psi to 2500 psi, 2500 psi to 2600 psi, 2600 psi to 2700 psi, 2700 psi to 2800 psi, 2800 psi to 2900 psi, and 2900 psi to 3000 psi.

7. The method of claim 6, wherein said total pressure and/or hydrogen partial pressure is in the range of about 1500 psi to about 2000 psi.

8. The method of any of claims 1 - 6, wherein the catalyst comprises Ni/MO and/or Co/Mo on an alumina or a silica-alumina support.

9. The method of Claim 1, wherein the catalyst is **characterized by** having a pore structure comprising macropores and **characterized by** BET surface area in the range of about 10 m²/g to about 350 m²/g or about 150 m²/g to about 250 m²/g; micropores in the average diameter range of about 50 Angstroms to about 200 Angstroms; or macropores in the range of about 1000 Angstroms to about 3000 Angstroms.

10. The method claim 1, wherein the method further comprises, either prior to step b) or after step b), thermally treating the algae oil prior to hydrotreating, by raising the algae oil to a temperature in the range of about 300 to about 600 degrees C, and holding at about that temperature for a hold time in the range of 0 minutes to about 8 hours, about 0.25 to about 8 hours, or about 0.5 to about 2 hours, wherein no hydrogen is added to the thermal treatment process.

11. The method of claim 10, wherein the thermal treatment is conducted in the range of about atmospheric to about 300 psi.

12. The method of claim 10, wherein the method further comprising separating a hydrotreated oil from the hydrotreating effluent and fluid-catalytic-cracking (FCC) the hydrotreated oil.

13. The method of any of claims 1 - 6 and 8 - 12, wherein the biomass comprises microalga and/or macroalga.

14. The method of claim 13, wherein the microalga is a cyanobacterium, a *Desmodesmus* species, *Spirulina* species, *Nannochloropsis* species, or *Nannochloropsis salina.*

15. The hydrotreating effluent made by the method of any one of claims 1, 2, 4 - 6, 8 - 11, and 13 - 14.

## Patentansprüche

1. Verfahren zur Veredelung von Algenöl, erhalten aus Biomasse, umfassend Algen, wobei das Verfahren umfasst:
a) Bereitstellung des Algenöls;
b) Lösung zumindest eines Teils des Algenöls in einem Lösungsmittel;
c) Veredelung des Algenöls in dem Lösungsmittel durch ein Verfahren, umfassend: Hydrodesulfurierung des Algenöls in dem Lösungsmittel in der Gegenwart eines Katalysators, bei einer Temperatur von etwa 300°C bis etwa 500°C; einem Gesamtdruck und/oder Wasserstoffpartialdruck von etwa 800 psi bis etwa 3000 psi; einer Raumgeschwindigkeit von etwa 0,1 Ölvolumen pro Katalysatorvolumen pro Stunde bis etwa 10 Ölvolumen pro Katalysatorvolumen pro Stunde; und einer Wasserstoffförderrate von etwa 10 m³ H₂/m³ gelöstes Öl bis etwa 1700 m³ H₂/m³ gelöstes Öl; wobei dieser Katalysator ein großporiger Katalysator, ausgewählt aus der Gruppe, bestehend aus Erdölrückstand- und Erdölbitumen-Hydrodesulfurierungskatalysatoren, ist, um ein Hydrodesulfurierungsabwasser zu erhalten.

2. Verfahren nach Anspruch 1, wobei das Algenöl vor Schritt a), Schritt b) und Schritt c) nicht raffiniertgebleicht-desodoriert (RBD) wurde.

3. Verfahren nach Anspruch 1, wobei das Verfahren ferner Abtrennen eines hydrodesulfurierten Öls aus dem Hydrodesulfurierungsabwasser und weitere Veredelung des hydrodesulfurierten Öls durch Befördern des hydrodesulfurierten Öls oder einer Fraktion davon an einen oder mehrere Vertreter von einer FCC-Einheit, einer Hydrocracking-Einheit, einer Hydroisomerisierungseinheit, einer Entparaffinisierungseinheit, eines Naphtha-Reformers oder einer Einheit, die Ni/MO, Co/MO, Ni/W, ein Edelmetall, ein edles Metall, einen Gruppe VIII-Katalysator oder einen zeolithischen Katalysator verwendet, umfasst.

4. Verfahren nach Anspruch 1, wobei das Lösungsmittel Naphtha, Diesel, Kerosin, leichtes Gasöl, schweres Gasöl, Rückstand, Schweröl, Dodecan, ein zyklisches Lösungsmittel, ein aromatisches Lösungsmittel, ein Kohlenwasserstofflösungsmittel, Rohöl, jedes nach Rohöldestillation und/oder weiterer Raffination von Rohölfraktionen erhaltene Produkt, oder jede Kombination hiervon, ist.

5. Verfahren nach Anspruch 4, wobei das Lösungsmittel schweres Gasöl, Rückstand, Schweröl, Rohöl oder jede Kombination hiervon ist.

6. Verfahren nach Anspruch 1, wobei der Gesamtdruck und/oder Wasserstoffpartialdruck von etwa 1000 psi bis etwa 2000 psi, etwa 1500 psi bis etwa 2000 psi ist; oder ausgewählt ist aus der Gruppe, bestehend aus: 1000 psi bis 1100 psi, 1100 psi bis 1200 psi, 1200 psi bis 1300 psi, 1300 psi bis 1400 psi, 1400 psi bis 1500 psi, 1500 psi bis 1600 psi, 1600 psi bis 1700 psi, 1700 psi bis 1800 psi, 1800 psi bis 1900 psi, 1900 psi bis 2000 psi, 2000 psi bis 2100 psi, 2100 psi bis 2200 psi, 2200 psi bis 2300 psi, 2300 psi bis 2400 psi, 2400 psi bis 2500 psi, 2500 psi bis 2600 psi, 2600 psi bis 2700 psi, 2700 psi bis 2800 psi, 2800 psi bis 2900 psi und 2900 psi bis 3000 psi.

7. Verfahren nach Anspruch 6, wobei der Gesamtdruck und/oder Wasserstoffpartialdruck im Bereich von etwa 1500 psi bis etwa 2000 psi ist.

8. Verfahren nach einem der Ansprüche 1 - 6, wobei der Katalysator Ni/MO und/oder Co/Mo auf einem Aluminiumoxid- oder einem Silica-Aluminiumoxidträger umfasst.

9. Verfahren nach Anspruch 1, wobei der Katalysator **dadurch gekennzeichnet ist, dass** er eine Porenstruktur, umfassend Makroporen, und **gekennzeichnet durch** eine BET-Oberfläche in dem Bereich von etwa 10 m²/g bis etwa 350 m²/g oder etwa 150 m²/g bis etwa 250 m²/g; Mikroporen in dem mittleren Durchmesserbereich von etwa 50 Angström bis etwa 200 Angström; oder Makroporen in dem Bereich von etwa 1000 Angström bis etwa 3000 Angström aufweist.

10. Verfahren nach Anspruch 1, wobei das Verfahren ferner, entweder vor Schritt b) oder nach Schritt b), thermische Behandlung des Algenöls vor der Hydrodesulfurierung, durch Hochheizen des Algenöls auf eine Temperatur in dem Bereich von etwa 300 bis etwa 600°C und Halten bei etwa dieser Temperatur für eine Haltezeit in dem Bereich von 0 Minuten bis etwa 8 Stunden, etwa 0,25 bis etwa 8 Stunden oder etwa 0,5 bis etwa 2 Stunden umfasst, wobei dem thermischen Behandlungsprozess kein Wasserstoff hinzugefügt wird.

11. Verfahren nach Anspruch 10, wobei die thermische Behandlung im Bereich von etwa Atmosphärendruck bis etwa 300 psi durchgeführt wird.

12. Verfahren nach Anspruch 10, wobei das Verfahren ferner Abtrennen eines hydrodesulfurierten Öls aus dem Hydrodesulfurierungsabwasser und Fluid-katalytisches Cracken (FCC) des hydrodesulfurierten Öls umfasst.

13. Verfahren nach einem der Ansprüche 1 - 6 und 8 - 12, wobei die Biomasse Mikroalgen und/oder Makroalgen umfasst.

14. Verfahren nach Anspruch 13, wobei die Mikroalge ein Cyanobacterium, eine *Desmodesmus*-Spezies, *Spirulina-*Spezies, *Nannochloropsis*-Spezies oder *Nannochloropsis salina* ist.

15. Hydrodesulfurierungsabwasser, hergestellt durch ein Verfahren nach einem der Ansprüche 1, 2, 4 - 6, 8 - 11 und 13 - 14.

## Revendications

1. Procédé de valorisation d'huile algale obtenue à partir de biomasse comprenant des algues, le procédé comprenant :
a) la fourniture de l'huile algale ;
b) la dissolution d'au moins une portion de l'huile algale dans un solvant ;
c) la valorisation de l'huile algale dans le solvant par un procédé comprenant : l'hydrotraitement de l'huile algale dans le solvant en présence d'un catalyseur, à une température d'environ 300 degrés C à environ 500 degrés C ; une pression totale et/ou une pression partielle d'hydrogène d'environ 800 psi à environ 3000 psi ; une vitesse spatiale d'environ 0,1 volume d'huile par volume de catalyseur par heure à environ 10 volumes d'huile par volume de catalyseur par heure ; et un débit d'alimentation en hydrogène d'environ 10 m³ de H₂/m³ d'huile dissoute à environ 1700 m³ de H₂/m³ d'huile dissoute ; dans lequel ledit catalyseur est un catalyseur à gros pores choisi dans le groupe constitué des catalyseurs d'hydrotraitement de résidu de pétrole et de bitume de pétrole, pour obtenir un effluent d'hydrotraitement.

2. Procédé selon la revendication 1, dans lequel, avant l'étape a), l'étape b) et l'étape c), l'huile algale n'a pas été raffinée-blanchie-désodorisée (RBD).

3. Procédé selon la revendication 1, dans lequel le procédé comprend en outre la séparation d'une huile hydrotraitée de l'effluent d'hydrotraitement ; et en outre la valorisation de l'huile hydrotraitée par envoi de l'huile hydrotraitée ou d'une fraction de celle-ci à un(e) ou plus d'une unité FCC, d'une unité d'hydrocraquage, d'une unité d'hydroisomérisation, d'une unité de déparaffinage, d'un reformeur de naphta, ou d'une unité utilisant Ni/MO, Co/MO, Ni/W, un métal précieux, un métal noble, un catalyseur du groupe VIII ou un catalyseur zéolitique.

4. Procédé selon la revendication 1, dans lequel le solvant est un naphta, du diesel, du kérosène, du gasoil léger, du gasoil lourd, un résidu de distillation, du brut lourd, un dodécane, un solvant cyclique, un solvant aromatique, un solvant hydrocarboné , du pétrole brut, tout produit obtenu après distillation de pétrole brut et/ou le raffinage poussé de fractions de pétrole brut, ou toute combinaison de ceux-ci.

5. Procédé selon la revendication 4, dans lequel le solvant est du gasoil lourd, un résidu de distillation, du brut lourd, du pétrole brut ou toute combinaison de ceux-ci.

6. Procédé selon la revendication 1, dans lequel la pression totale et/ou la pression partielle d'hydrogène sont d'environ 1000 psi à environ 2000 psi, d'environ 1500 psi à environ 2000 psi ; ou choisies dans le groupe constitué des suivantes : 1000 psi à 1100 psi, 1100 psi à 1200 psi, 1200 psi à 1300 psi, 1300 psi à 1400 psi, 1400 psi à 1500 psi, 1500 psi à 1600 psi, 1600 psi à 1700 psi, 1700 psi à 1800 psi, 1800 psi à 1900 psi, 1900 psi à 2000 psi, 2000 psi à 2100 psi, 2100 psi à 2200 psi, 2200 psi à 2300 psi, 2300 psi à 2400 psi, 2400 psi à 2500 psi, 2500 psi à 2600 psi, 2600 psi à 2700 psi, 2700 psi à 2800 psi, 2800 psi à 2900 psi et 2900 psi à 3000 psi.

7. Procédé selon la revendication 6, dans lequel ladite pression totale et/ou ladite pression partielle d'hydrogène sont dans la plage d'environ 1500 psi à environ 2000 psi.

8. Procédé selon l'une quelconque des revendications 1 à 6, dans lequel le catalyseur comprend Ni/MO et/ou Co/Mo sur un support d'alumine ou de silice-alumine.

9. Procédé selon la revendication 1, dans lequel le catalyseur est **caractérisé en ce qu'**il possède une structure de pores comprenant des macropores et **caractérisé par** une superficie BET dans la plage d'environ 10 m²/g à environ 350 m²/g ou d'environ 150 m²/g à environ 250 m²/g ; des micropores dans la plage de diamètre moyenne d'environ 50 angstroms à environ 200 angstroms ; ou des macropores dans la plage d'environ 1000 angstroms à environ 3000 angstroms.

10. Procédé selon la revendication 1, dans lequel le procédé comprend en outre, soit avant l'étape b), soit après l'étape b), un traitement thermique de l'huile algale avant hydrotraitement, suivi d'une augmentation de l'huile algale à une température dans la plage d'environ 300 à environ 600 degrés C, et du maintien à peu près à cette température pendant un temps de maintien dans la plage de 0 minute à environ 8 heures, d'environ 0,25 à environ 8 heures, ou d'environ 0,5 à environ 2 heures, où de l'hydrogène n'est pas ajouté au processus de traitement thermique.

11. Procédé selon la revendication 10, dans lequel le traitement thermique est conduit dans la plage d'environ la pression atmosphérique à environ 300 psi.

12. Procédé selon la revendication 10, dans lequel le procédé comprend en outre la séparation d'une huile hydrotraitée de l'effluent d'hydrotraitement et le craquage catalytique fluide (FCC) de l'huile hydrotraitée.

13. Procédé selon l'une quelconque des revendications 1 à 6 et 8 à 12, dans lequel la biomasse comprend une microalgue et/ou une macroalgue.

14. Procédé selon la revendication 13, dans lequel la microalgue est une cyanobactérie, une espèce *Desmodesmus,* une espèce *Spirulina,* une espèce *Nannochloropsis* ou *Nannochloropsis salina.*

15. Effluent d'hydrotraitement préparé par le procédé de l'une quelconque des revendications 1, 2, 4 à 6, 8 à 11 et 13 à 14.
